# EUROPEAN PATENT SPECIFICATION

(11) **EP 1 858 879 B1**
(45) Date of publication and mention of the grant of the patent: **06.06.2012**
(21) Application number: 06718359.0
(22) Date of filing: 13.01.2006
(51) Int. Cl.: C07D 401/06, A61K 31/404, A61P 35/04

(54) **1,3-DIARYL SUBSTITUTED UREAS AS MODULATORS OF KINASE ACTIVITY**
1,3-SUBSTITUIERTE DIARYL-HARNSTOFFE ALS MODULATOREN VON KINASE-AKTIVITÄT
UREES A SUBSTITUTION 1,3-DIARYLE, MODULATEURS DE L'ACTIVITE DE KINASES

(30) Priority: 14.01.2005 US 643886 P; 02.11.2005 US 732999 P
(43) Date of publication of application: 28.11.2007
(60) Divisional application: 11181551.0
(73) Proprietor: Gilead Connecticut, Inc., Wilmington, DE 19801 (US)
(72) Inventor: MITCHELL, Scott A., East Haven, Connecticut 06513 (US); DANCA, Mihaela Diana, Mendham, New Jersey 07945 (US); BLOMGREN, Peter A., North Branford, Connecticut 06471 (US); DESIMONE, Robert W., Durham, Connecticut 06422 (US); PIPPIN, Douglas A. I., Chester Springs, Pennsylvania 19425 (US); BRITTELLI, David R., Branford, Connecticut 06405 (US)
(74) Representative: Marshall, Cameron John
(86) International application number: PCT/US2006/001272
(87) International publication number: WO 2006/076593

(56) References cited:
- WO-A-96/40673
- WO-A-99/32106
- WO-A-99/32436
- WO-A-03/072541
- WO-A2-03/087087

## Description

Provided herein are certain substituted ureas and related compounds, compositions comprising such compounds, and methods of their use.

Protein kinases, the largest family of human enzymes, encompass well over 500 proteins. Kinases play a key role in angiogenesis. Angiogenesis, the formation of new blood vessels from preexisting ones, plays a significant role in many pathological settings, including cancer, chronic inflammation, diabetic retinopathy, psoriasis, rheumatoid arthritis, and macular degeneration. Anti-angiogenic therapy represents a potentially important approach for the treatment of solid tumors and other diseases associated with dysregulated vascularization.

The process of angiogenesis is complex, requiring the concerted actions of multiple angiogenic mediators as well as the participation of different cell types. Key angiogenesis mediators, including, VEGF, FGF, and angiopoietin 1 and 2 (Angl and Ang2) that bind to their cognate receptors (VEGFRs, FGFRs and Tie1 and Tie2, respectively) expressed on endothelial cells, as well as platelet-derived growth factor (PDGF) that binds to its receptor (PDGFRα) expressed on VEGF-producing stromal cells or its receptor (PDGFRβ) expressed on pericytes and smooth muscle cells have been identified. Recent studies indicate that several members of the ephrin family and their receptor Eph family are also regulators of angiogenesis. VEGFRs, FGFRs, Tie1, Tie2, PDGFRs, and Eph receptors all belong to the receptor protein tyrosine kinase (RTK) superfamily. Given the important roles of these RTKs in angiogenesis, their modulation would be pharmacologically desirable for the treatment of cancer and other disease.

Provided is at least one chemical entity chosen from compounds of Formula 1 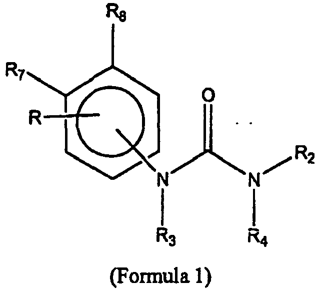 and pharmaceutically acceptable salts, solvates, crystal forms, and mixtures thereof, wherein
R represents 0 to 2 substituents independently chosen from hydroxy, nitro, cyano, optionally substituted amino, aminocarbonyl, halo, carboxy, optionally substituted acyl, optionally substituted alkoxycarbonyl, optionally substituted C₁-C₆ alkyl, optionally substituted C₁-C₆ alkoxy, sulfanyl, sulfinyl, sulfonyl, optionally substituted aryl, optionally substituted heteroaryl, and optionally substituted heterocycloalkyl;
R₇ and R₈, taken together with the carbons to which they are bound, form a fused 5- to 7-membered heteroaryl ring substituted with a group -(Z₁)ₘR₁, wherein the fused 5- to 7-membered heteroaryl ring is optionally furrther substituted and wherein
R₁ is optionally substituted heteroaryl;
Z₁ is -CR₅R₆- wherein each R₅ and R₆ is independently chosen from hydrogen, optionally substituted C₁-C₆ alkyl, and halo; and
m is chosen from 0, 1, and 2;
   R₂ is optionally substituted aryl; and
   R₃ and R₄ are each independently chosen from hydrogen, optionally substituted C₁-C₆ alkyl, optionally substituted aryl, and optionally substituted heteroaryl, ***provided that***
   the compound of Formula 1 is not chosen from 5-(phenylcarbamoylamino)-3-(2-(4-pyridyl)ethyl)indole; 1-(4-chloro-3-(trifluoromethyl)phenyl)-3-(1-(pyridin-4-yl)-1H-indol-5-yl)urea; and 1-(2-methoxy-5-(trifluoromethyl)phenyl)-3-(1-(pyridin-4-yl)-1H-indol-5-yl)urea.

Also provided is a pharmaceutical composition, comprising at least one chemical entity described herein, together with at least one pharmaceutically acceptable vehicle chosen from carriers, adjuvants, and excipients. Also provided is a packaged pharmaceutical composition, comprising the pharmaceutical composition described herein in a container; and instructions for using the composition to treat a patient suffering from a disease or disorder responsive to kinase activity modulation of one or more tyrosine kinase.

Also provided is at least one chemical entity for use in a method of treating a patient having a disease or disorder responsive to kinase activity modulation said method comprising administering to the patient a therapeutically effective amount of at least one chemical entity described herein or a composition described herein.

Also provided is at least one chemical entity for use in a method for treating a female patient having a female reproductive disorder or condition said method comprising administering to the female patient a therapeutically effective amount of at least one chemical entity described herein or a pharmaceutical composition described herein.

Also provided is at least one chemical entity for use in a method of modulating EphB₄ kinase activity, the method comprising contacting cells expressing EphB₄ with at least one chemical entity described herein in an amount sufficient to detectably inhibit EphB₄ kinase activity *in vitro.*

Also provided at least one chemical entity for use in is a method of modulating VEGFR2 kinase activity, the method comprising contacting cells expressing VEGFR2 with at least one chemical entity described herein in an amount sufficient to detectably inhibit VEGFR2 kinase activity *in vitro.*

Also provided is at least one chemical entity for use in a method of modulating PDGFRβ kinase activity, the method comprising contacting cells expressing PDGFRβ with at least one chemical entity described herein in an amount sufficient to detectably inhibit PDGFRβ kinase activity *in vitro.*

Also provided is at least one chemical entity for use in a method of modulating c-Kit kinase activity, the method comprising contacting cells expressing c-Kit with at least one chemical entity described herein in an amount sufficient to detectably inhibit c-Kit kinase activity *in vitro.*

Also provided is at least one chemical entity for use in a method of modulating an activity of at least one kinase chosen from VEGFR2, EphB₄, PDGFRβ, and c-Kit, the method comprising contacting cells expressing at least one kinase chosen from VEGFR2, EphB₄, PDGFRβ, and c-Kit with at least one chemical entity described herein in an amount sufficient to detectably inhibit the activity of at least one kinase chosen from VEGFR2, EphB₄, PDGFRβ, and c-Kit *in vitro.*

Also provided is the use of at least one chemical entity for the manufacture of a medicament for the treatment of a patient having a disease responsive to inhibition of at least one kinase chosen from VEGFR2, EphB₄, PDGFRβ, and c-Kit, wherein the at least one chemical entity is a chemical entity described herein.

Also provided is a method for the manufacture of a medicament for the treatment of a patient having a disease responsive to inhibition of at least one kinase chosen from VEGFR2, EphB₄, PDGFRβ, and c-Kit, comprising including in said medicament at least one chemical entity described herein.

As used in the present specification, the following words and phrases are generally intended to have the meanings as set forth below, except to the extent that the context in which they are used indicates otherwise. The following abbreviations and terms have the indicated meanings throughout:
As used in the present specification, the following words and phrases are generally intended to have the meanings as set forth below, except to the extent that the context in which they are used indicates otherwise.

Formula 1 includes all subformulae thereof. For example Formula 1 includes compounds of Formulae 1 to 5.

As used herein, when any variable occurs more than one time in a chemical formula, its definition on each occurrence is independent of its definition at every other occurrence. In accordance with the usual meaning of "a" and "the" in patents, reference, for example, to "a" kinase or "the" kinase is inclusive of one or more kinases.

A dash ("-") that is not between two letters or symbols is used to indicate a point of attachment for a substituent. For example, -CONH₂ is attached through the carbon atom.

By "optional" or "optionally" is meant that the subsequently described event or circumstance may or may not occur, and that the description includes instances where the event or circumstance occurs and instances in which it does not. For example, "optionally substituted alkyl" encompasses both "alkyl" and "substituted alkyl" as defined below. It will be understood by those skilled in the art, with respect to any group containing one or more substituents, that such groups are not intended to introduce any substitution or substitution patterns that are sterically impractical, synthetically non-feasible and/or inherently unstable.

"Alkyl" encompasses straight chain and branched chain having the indicated number of carbon atoms, usually from 1 to 20 carbon atoms, for example 1 to 8 carbon atoms, such as 1 to 6 carbon atoms. For example C₁-C₆ alkyl encompasses both straight and branched chain alkyl of from 1 to 6 carbon atoms. Examples of alkyl groups include methyl, ethyl, propyl, isopropyl, n-butyl, sec-butyl, tert-butyl, pentyl, 2-pentyl, isopentyl, neopentyl, hexyl, 2-hexyl, 3-hexyl, 3-methylpentyl, and the like. Alkylene is another subset of alkyl, referring to the same residues as alkyl, but having two points of attachment. Alkylene groups will usually have from 2 to 20 carbon atoms, for example 2 to 8 carbon atoms, such as from 2 to 6 carbon atoms. For example, C₀ alkylene indicates a covalent bond and C₁ alkylene is a methylene group. When an alkyl residue having a specific number of carbons is named, all geometric combinations having that number of carbons are intended to be encompassed; thus, for example, "butyl" is meant to include n-butyl, sec-butyl, isobutyl and t-butyl; "propyl" includes n-propyl and isopropyl. "Lower alkyl" refers to alkyl groups having one to four carbons.

"Alkenyl" refers to an unsaturated branched or straight-chain alkyl group having at least one carbon-carbon double bond derived by the removal of one hydrogen atom from a single carbon atom of a parent alkene. The group may be in either the *cis* or *trans* conformation about the double bond(s). Typical alkenyl groups include, but are not limited to, ethenyl; propenyls such as prop-1-en-1-yl, prop-1-en-2-yl, prop-2-en-1-yl (allyl), prop-2-en-2-yl, cycloprop-1-en-1-yl; cycloprop-2-en-1-yl; butenyls such as but-1-en-1-yl, but-1-en-2-yl, 2-methyl-prop-1-en-1-yl, but-2-en-1-yl, but-2-en-1-yl, but-2-en-2-yl, buta-1,3-dien-1-yl, buta-1,3-dien-2-yl, cyclobut-1-en-1-yl, cyclobut-1-en-3-yl, cyclobuta-1,3-dien-1-yl; and the like. In certain embodiments, an alkenyl group has from 2 to 20 carbon atoms and in other embodiments, from 2 to 6 carbon atoms.

"Alkynyl" refers to an unsaturated branched or straight-chain alkyl group having at least one carbon-carbon triple bond derived by the removal of one hydrogen atom from a single carbon atom of a parent alkyne. Typical alkynyl groups include, but are not limited to, ethynyl; propynyls such as prop-1-yn-1-yl, prop-2-yn-1-yl; butynyls such as but-1-yn-1-yl, but-1-yn-3-yl, but-3-yn-1-yl; and the like. In certain embodiments, an alkynyl group has from 2 to 20 carbon atoms and in other embodiments, from 3 to 6 carbon atoms.

"Cycloalkyl" indicates a non-aromatic carbocyclic ring, usually having from 3 to 7 ring carbon atoms. The ring may be saturated or have one or more carbon-carbon double bonds. Examples of cycloalkyl groups include cyclopropyl, cyclobutyl, cyclopentyl, cyclopentenyl, cyclohexyl, and cyclohexenyl, as well as bridged and caged saturated ring groups such as norbomane.

By "alkoxy" is meant an alkyl group of the indicated number of carbon atoms attached through an oxygen bridge such as, for example, methoxy, ethoxy, propoxy, isopropoxy, n-butoxy, sec-butoxy, tert-butoxy, pentyloxy, 2-pentyloxy, isopentyloxy, neopentyloxy, hexyloxy, 2-hexyloxy, 3-hexyloxy, 3-methylpentyloxy, and the like. Alkoxy groups will usually have from 1 to 6 carbon atoms attached through the oxygen bridge. "Lower alkoxy" refers to alkoxy groups having one to four carbons.

"Mono- and di-alkylcarboxamide" encompasses a group of the formula - (C=O)NRₐR_{b} where Rₐ and R_{b} are independently chosen from hydrogen and alkyl groups of the indicated number of carbon atoms, provided that Rₐ and R_{b} are not both hydrogen.

"Acyl" refers to the groups (alkyl)-C(O)-; (cycloalkyl)-C(O)-; (aryl)-C(O)-; (heteroaryl)-C(O)-; and (heterocycloalkyl)-C(O)-, wherein the group is attached to the parent structure through the carbonyl functionality and wherein alkyl, cycloalkyl, aryl, heteroaryl, and heterocycloalkyl are as described herein. Acyl groups have the indicated number of carbon atoms, with the carbon of the keto group being included in the numbered carbon atoms. For example a C₂ acyl group is an acetyl group having the formula CH₃(C=O)-.

By "alkoxycarbonyl" is meant a group of the formula (alkoxy)(C=O)- attached through the carbonyl carbon wherein the alkoxy group has the indicated number of carbon atoms. Thus a C₁-C₆ alkoxycarbonyl group is an alkoxy group having from 1 to 6 carbon atoms attached through its oxygen to a carbonyl linker.

By "amino" is meant the group -NH₂.

"Mono- and di-(alkyl)amino" encompasses secondary and tertiary alkyl amino groups, wherein the alkyl groups are as defined above and have the indicated number of carbon atoms. The point of attachment of the alkylamino group is on the nitrogen. Examples of mono- and di-alkylamino groups include ethylamino, dimethylamino, and methyl-propyl-amino.

By "amino(alkyl)" is meant an amino group linked to an alkyl group having the indicated number of carbons. Similarly "hydroxyalkyl" is a hydroxy group linked to an alkyl group.

The term "aminocarbonyl" refers to the group -CONR^{b}R^{c}, where
R^{b} is chosen from H, optionally substituted C₁-C₆ alkyl, optionally substituted cycloalkyl, optionally substituted heterocycloalkyl, optionally substituted aryl, and optionally substituted heteroaryl; and
R^{c} is independently chosen from hydrogen and optionally substituted C₁-C₄ alkyl; or
R^{b} and R^{c} taken together with the nitrogen to which they are bound, form an optionally substituted 5- to 7-membered nitrogen-containing heterocycloalkyl which optionally includes 1 or 2 additional heteroatoms selected from O, N, and S in the heterocycloalkyl ring;
where each substituted group is independently substituted with one or more substituents independently selected from C₁C₄ alkyl, aryl, heteroaryl, aryl-C₁₋C₄ alkyl,-, heteroaryl-C₁-C₄ alkyl,-, C₁-C₄ haloalkyl-, -OC₁-C₄ alkyl, -OC₁-C₄ alkylphenyl, - C₁-C₄ alkyl-OH, -OC₁-C₄ haloalkyl, halo, -OH, -NH₂, -C₁-C₄ alkyl-NH₂, -N(C₁-C₄ alkyl)(C₁-C₄ alkyl), -NH(C₁-C₄ alkyl), -N(C₁-C₄ alkyl)(C₁-C₄ alkylphenyl), -NH(C₁-C₄ alkylphenyl), cyano, nitro, oxo (as a substitutent for cycloalkyl, heterocycloalkyl, or heteroaryl), -CO₂H, -C(O)OC₁-C₄ alkyl, -CON(C₁-C₄ alkyl)(C₁-C₄ alkyl), -CONH(C₁-C₄ alkyl), -CONH₂, -NHC(O)(C₁-C₄ alkyl), -NHC(O)(phenyl), -N(C₁-C₄ alkyl)C(O)(C₁-C₄ alkyl), -N(C₁-C₄ alkyl)C(O)(phenyl), -C(O)C₁-C₄ alkyl, -C(O)C₁-C₄ alkylphenyl, -C(O)C₁-C₄ haloalkyl, -OC(O)C₁-C₄ alkyl, -SO₂(C₁-C₄ alkyl), - SO₂(phenyl), -SO₂(C₁-C₄ haloalkyl), -SO₂NH₂, -SO₂NH(C₁-C₄ alkyl), -SO₂NH(phenyl), - NHSO₂(C₁-C₄ alkyl), -NHSO₂(phenyl), and -NHSO₂(C₁-C₄ haloalkyl).

"Aryl" encompasses:
6-membered carbocyclic aromatic rings, for example, benzene;
bicyclic ring systems wherein at least one ring is carbocyclic and aromatic, for example, naphthalene, indane, and tetralin; and
tricyclic ring systems wherein at least one ring is carbocyclic and aromatic, for example, fluorene.
Aryl includes 6-membered carbocyclic aromatic rings fused to a 5- to 7-membered heterocycloalkyl ring containing 1 or more heteroatoms chosen from N, O, and S. For example, aryl includes phenyl substituted with -O(C₁-C₂ alkyl)O- (e.g., phenyl substituted with a methylenedioxy or ethylenedioxy group). For such fused, bicyclic ring systems wherein only one of the rings is a carbocyclic aromatic ring, the point of attachment may be at the carbocyclic aromatic ring or the heterocycloalkyl ring. Bivalent radicals formed from substituted benzene derivatives and having the free valences at ring atoms are named as substituted phenylene radicals. Bivalent radicals derived from univalent polycyclic hydrocarbon radicals whose names end in "-yl" by removal of one hydrogen atom from the carbon atom with the free valence are named by adding "-idene" to the name of the corresponding univalent radical, e.g., a naphthyl group with two points of attachment is termed naphthylidene. Aryl, however, does not encompass or overlap in any way with heteroaryl, separately defined below. Hence, if one or more carbocyclic aromatic rings is fused with a heterocycloalkyl aromatic ring, the resulting ring system is heteroaryl, not aryl, as defined herein.

The term "aryloxy" refers to the group -O-aryl.

The term "halo" includes fluoro, chloro, bromo, and iodo, and the term "halogen" includes fluorine, chlorine, bromine, and iodine.

"Haloalkyl" indicates alkyl as defined above having the specified number of carbon atoms, substituted with 1 or more halogen atoms, generally up to the maximum allowable number of halogen atoms. Examples of haloalkyl include, but are not limited to, trifluoromethyl, difluoromethyl, 2-fluoroethyl, and penta-fluoroethyl.

"Heteroaryl" encompasses:
5- to 7-membered aromatic, monocyclic rings containing one or more, for example, from 1 to 4, or in certain embodiments, from 1 to 3, heteroatoms chosen from N, O, and S, with the remaining ring atoms being carbon; and
bicyclic heterocycloalkyl rings containing one or more, for example, from 1 to 4, or in certain embodiments, from 1 to 3, heteroatoms chosen from N, O, and S, with the remaining ring atoms being carbon and wherein at least one heteroatom is present in an aromatic ring.
Heteroaryl includes a 5- to 7-membered heterocycloalkyl, aromatic ring fused to a 5- to 7-membered cycloalkyl or heterocycloalkyl ring. For example, heteroaryl includes pyridinyl substituted with -O(C₁-C₂ alkyl)O- (e.g., pyridinyl substituted with a methylenedioxy or ethylenedioxy group). For such fused, bicyclic heteroaryl ring systems, the point of attachment may be at either ring. When the total number of S and O atoms in the heteroaryl group exceeds 1, those heteroatoms are not adjacent to one another. In certain embodiments, the total number of S and O atoms in the heteroaryl group is not more than 2. In certain embodiments, the total number of S and O atoms in the aromatic heterocycle is not more than 1. Examples of heteroaryl groups include, but are not limited to, (as numbered from the linkage position assigned priority 1), 2-pyridyl, 3-pyridyl, 4-pyridyl, 2,3-pyrazinyl, 3,4-pyrazinyl, 2,4-pyrimidinyl, 3,5-pyrimidinyl, 2,3-pyrazolinyl, 2,4-imidazolinyl, isoxazolinyl, oxazolinyl, thiazolinyl, thiadiazolinyl, tetrazolyl, thienyl, benzothiophenyl, furanyl, benzofuranyl, benzoimidazolinyl, indolinyl, pyridazinyl, triazolyl, quinolinyl, pyrazolyl, and 5,6,7,8-tetrahydroisoquinoline. Bivalent radicals derived from univalent heteroaryl radicals whose names end in "-yl" by removal of one hydrogen atom from the atom with the free valence are named by adding "-idene" to the name of the corresponding univalent radical, e.g., a pyridyl group with two points of attachment is a pyridylidene. Heteroaryl does not encompass or overlap with aryl, cycloalkyl, or heterocycloalkyl, as defined herein

Substituted heteroaryl also includes ring systems substituted with one or more oxide (-O⁻) substituents, such as pyridinyl N-oxides.

In the term "heteroarylalkyl," heteroaryl and alkyl are as defined herein, and the point of attachment is on the alkyl group. This term encompasses, but is not limited to, pyridylmethyl, thiophenylmethyl, and (pyrrolyl)1-ethyl.

By "heterocycloalkyl" is meant a single, non-aromatic ring, usually with 3 to 7 ring atoms, containing at least 2 carbon atoms in addition to 1-3 heteroatoms independently selected from oxygen, sulfur, and nitrogen, as well as combinations comprising at least one of the foregoing heteroatoms. The ring may be saturated or have one or more carbon-carbon double bonds. Suitable heterocycloalkyl groups include, for example (as numbered from the linkage position assigned priority 1), 2-pyrrolinyl, 2,4-imidazolidinyl, 2,3-pyrazolidinyl, 2-piperidyl, 3-piperidyl, 4-piperdyl, and 2,5-piperzinyl. Morpholinyl groups are also contemplated, including 2-morpholinyl and 3-morpholinyl (numbered wherein the oxygen is assigned priority 1). Substituted heterocycloalkyl also includes ring systems substituted with one or more oxo (=0) or oxide (-O⁻) substituents, such as piperidinyl N-oxide, morpholinyl-N-oxide, 1-oxo-1-thiomorpholinyl and 1,1-dioxo-1-thiomorpholinyl.

"Heterocycloalkyl" also includes bicyclic ring systems wherein one non-aromatic ring, usually with 3 to 7 ring atoms, contains at least 2 carbon atoms in addition to 1-3 heteroatoms independently selected from oxygen, sulfur, and nitrogen, as well as combinations comprising at least one of the foregoing heteroatoms; and the other ring, usually with 3 to 7 ring atoms, optionally contains 1-3 heteratoms independently selected from oxygen, sulfur, and nitrogen and is not-aromatic.

As used herein, "modulation" refers to a change in kinase activity as a direct or indirect response to the presence of compounds of Formula 1, relative to the activity of the kinase in the absence of the compound. The change may be an increase in activity or a decrease in activity, and may be due to the direct interaction of the compound with the kinase, or due to the interaction of the compound with one or more other factors that in turn affect kinase activity. For example, the presence of the compound may, for example, increase or decrease kinase activity by directly binding to the kinase, by causing (directly or indirectly) another factor to increase or decrease the kinase activity, or by (directly or indirectly) increasing or decreasing the amount of kinase present in the cell or organism.

The term "sulfanyl" includes the groups: -S-(optionally substituted (C₁-C₆)alkyl), -S-(optionally substituted aryl), -S-(optionally substituted heteroaryl), and -S-(optionally substituted heterocycloalkyl). Hence, sulfanyl includes the group C₁-C₆ alkylsulfanyl.

The term "sulfinyl" includes the groups: -S(O)-(optionally substituted (C₁-C₆)alkyl), -S(O)-optionally substituted aryl), -S(O)-optionally substituted heteroaryl), -S(O)-(optionally substituted heterocycloalkyl); and -S(O)-(optionally substituted amino).

The term "sulfonyl" includes the groups: -S(O₂)-(optionally substituted (C₁-C₆)alkyl), -S(O₂)-optionally substituted aryl), -S(O₂)-optionally substituted heteroaryl), - S(O₂)-(optionally substituted heterocycloalkyl) ,-S(O₂)-(optionally substituted alkoxy), -S(O₂)-optionally substituted aryloxy), -S(O₂)-optionally substituted heteroaryloxy), -S(O₂)-(optionally substituted heterocyclyloxy); and -S(O₂)-(optionally substituted amino).

The term "substituted", as used herein, means that any one or more hydrogens on the designated atom or group is replaced with a selection from the indicated group, provided that the designated atom's normal valence is not exceeded. When a substituent is oxo (i.e., =O) then 2 hydrogens on the atom are replaced. Combinations of substituents and/or variables are permissible only if such combinations result in stable compounds or useful synthetic intermediates. A stable compound or stable structure is meant to imply a compound that is sufficiently robust to survive isolation from a reaction mixture, and subsequent formulation as an agent having at least practical utility. Unless otherwise specified, substituents are named into the core structure. For example, it is to be understood that when (cycloalkyl)alkyl is listed as a possible substituent, the point of attachment of this substituent to the core structure is in the alkyl portion.

The terms "substituted" alkyl, cycloalkyl, aryl, heterocycloalkyl, and heteroaryl, unless otherwise expressly defined, refer respectively to alkyl, cycloalkyl, aryl, heterocycloalkyl, and heteroaryl wherein one or more (such as up to 5, for example, up to 3) hydrogen atoms are replaced by a substituent independently chosen from:
R^{a}, -OR^{b}, -SR^{b}, guanidine, guanidine wherein one or more of the guanidine hydrogens are replaced with a lower-alkyl group, -NR^{b}R^{c}, halo, cyano, nitro, oxo (as a substitutent for cycloalkyl, heterocycloalkyl, and heteroaryl), -COR^{b}, -CO₂R^{b}, -CONR^{b}R^{c}, -OCOR^{b}, -OCO₂R^{a}, -OCONR^{b}R^{c}, -NR^{c}COR^{b}, -NR^{c}CO₂R^{a}, -NR^{c}CONR^{b}R^{c}, -CO₂R^{b}, -CONR^{b}R^{c}, -NR^{c}COR^{b}, -SOR^{a}, -SO₂R^{a}, -SO₂NR^{b}R^{c}, and -NR^{c}SO₂R^{a},
   where R^{a} is chosen from optionally substituted C₁-C₆ alkyl, optionally substituted alkenyl, optionally substituted alkynyl, optionally substituted aryl, and optionally substituted heteroaryl;
R^{b} is chosen from H, optionally substituted C₁-C₆ alkyl, optionally substituted cycloalkyl, optionally substituted heterocycloalkyl, optionally substituted aryl, and optionally substituted heteroaryl; and
R^{c} is independently chosen from hydrogen and optionally substituted C₁-C₄ alkyl; or
R^{b} and R^{c}, and the nitrogen to which they are attached, form an optionally substituted heterocycloalkyl group; and
where each optionally substituted group is unsubstituted or independently substituted with one or more, such as one, two, or three, substituents independently selected from C₁-C₄ alkyl, aryl, heteroaryl, aryl-C₁-C₄ alkyl-, heteroaryl-C₁-C₄ alkyl-, C₁-C₄ haloalkyl-, -OC₁-C₄ alkyl, -OC₁-C₄ alkylphenyl, -C₁-C₄ alkyl-OH, -OC₁-C₄ haloalkyl, halo, -OH, -NH₂, -C₁-C₄ alkyl-NH₂, -N(C₁-C₄ alkyl)(C₁-C₄ alkyl), -NH(C₁-C₄ alkyl), -N(C₁-C₄ alkyl)(C₁-C₄ alkylphenyl), -NH(C₁-C₄ alkylphenyl), cyano, nitro, oxo (as a substitutent for cycloalkyl, heterocycloalkyl, or heteroaryl), -CO₂H, -C(O)OC₁-C₄ alkyl, -CON(C₁-C₄ alkyl)(C₁-C₄ alkyl), -CONH(C₁-C₄ alkyl), -CONH₂, -NHC(O)(C₁-C₄ alkyl), -NHC(O)(phenyl), -N(C₁-C₄ alkyl)C(O)(C₁-C₄ alkyl), -N(C₁-C₄ alkyl)C(O)(phenyl), -C(O)C₁-C₄ alkyl, -C(O)C₁-C₄ alkylphenyl, -C(O)C₁-C₄ haloalkyl, -OC(O)C₁-C₄ alkyl, - SO₂(C₁-C₄ alkyl), -SO₂(phenyl), -SO₂(C₁-C₄ haloalkyl), -SO₂NH₂, -SO₂NH(C₁-C₄ alkyl), -SO₂NH(phenyl), -NHSO₂(C₁-C₄ alkyl), -NHSO₂(phenyl), and -NHSO₂(C₁-C₄ haloalkyl).

The term "substituted acyl" refers to the groups (substituted alkyl)-C(O)-; (substituted cycloalkyl)-C(O)-; (substituted aryl)-C(O)-; (substituted heteroaryl)-C(O)-; and (substituted heterocycloalkyl)-C(O)-, wherein the group is attached to the parent structure through the carbonyl functionality and wherein substituted alkyl, cycloalkyl, aryl, heteroaryl, and heterocycloalkyl, refer respectively to alkyl, cycloalkyl, aryl, heteroaryl, and heterocycloalkyl wherein one or more (such as up to 5, for example, up to 3) hydrogen atoms are replaced by a substituent independently chosen from:
-R^{a}, -OR^{b}, -SR^{b}, guanidine, guanidine wherein one or more of the guanidine hydrogens are replaced with a lower-alkyl group, -NR^{b}R^{c}, halo, cyano, nitro, -COR^{b}, -CO₂R^{b}, -CONR^{b}R^{c}, -OCOR^{b}, -OCO₂R^{a}, -OCONR^{b}R^{c}, -NR^{c}COR^{b}, -NR^{c}CO₂R^{a}, -NR^{c}CONR^{b}R^{c}, -CO₂R^{b}, -CONR^{b}R^{c}, -NR^{c}COR^{b}, -SOR^{a}, -SO₂R^{a}, -SO₂NR^{b}R^{c}, and -NR^{c}SO₂R^{a},
   where R^{a} is chosen from optionally substituted C₁-C₆ alkyl, optionally substituted alkenyl, optionally substituted alkynyl, optionally substituted aryl, and optionally substituted heteroaryl;
R^{b} is chosen from H, optionally substituted C₁-C₆ alkyl, optionally substituted cycloalkyl, optionally substituted heterocycloalkyl, optionally substituted aryl, and optionally substituted heteroaryl; and
R^{c} is independently chosen from hydrogen and optionally substituted C₁-C₄ alkyl; or
R^{b} and R^{c}, and the nitrogen to which they are attached, form an optionally substituted heterocycloalkyl group; and
where each optionally substituted group is unsubstituted or independently substituted with one or more, such as one, two, or three, substituents independently selected from C₁-C₄ alkyl, aryl, heteroaryl, aryl-C₁-C₄ alkyl-, heteroaryl-C₁-C₄ alkyl-, C₁-C₄ haloalkyl-, -OC₁-C₄ alkyl, -OC₁-C₄ alkylphenyl, -C₁-C₄ alkyl-OH, -OC₁-C₄ haloalkyl, halo, -OH, -NH₂, -C₁-C₄ alkyl-NH₂, -N(C₁-C₄ alkyl)(C₁-C₄ alkyl), -NH(C₁-C₄ alkyl), -N(C₁-C₄ alkyl)(C₁-C₄ alkylphenyl), -NH(C₁-C₄ alkylphenyl), cyano, nitro, oxo (as a substitutent for cycloalkyl, heterocycloalkyl, or heteroaryl), -CO₂H, -C(O)OC₁-C₄ alkyl, -CON(C₁-C₄ alkyl)(C₁-C₄ alkyl), -CONH(C₁-C₄ alkyl), -CONH₂, -NHC(O)(C₁-C₄ alkyl), -NHC(O)(phenyl), -N(C₁-C₄ alkyl)C(O)(C₁-C₄ alkyl), -N(C₁-C₄ alkyl)C(O)(phenyl), -C(O)C₁-C₄ alkyl, -C(O)C₁-C₄ alkylphenyl, -C(O)C₁-C₄ haloalkyl, -OC(O)C₁-C₄ alkyl, - SO₂(C₁-C₄ alkyl), -SO₂(phenyl), -SO₂(C₁-C₄ haloalkyl), -SO₂NH₂, -SO₂NH(C₁-C₄ alkyl), -SO₂NH(phenyl), -NHSO₂(C₁-C₄ alkyl), -NHSO₂(phenyl), and -NHSO₂(C₁-C₄ haloalkyl).

The term "substituted alkoxy" refers to alkoxy wherein the alkyl constituent is substituted (i.e., -O-(substituted alkyl)) wherein "substituted alkyl" refers to alkyl wherein one or more (such as up to 5, for example, up to 3) hydrogen atoms are replaced by a substituent independently chosen from:
-R^{a}, -OR^{b}, -SR^{b}, guanidine, guanidine wherein one or more of the guanidine hydrogens are replaced with a lower-alkyl group, -NR^{b}R^{c}, halo, cyano, nitro, -COR^{b}, -CO₂R^{b}, -CONR^{b}R^{c}, -OCOR^{b}, -OCO₂R^{a}, -OCONR^{b}R^{c}, -NR^{c}COR^{b}, -NR^{c}CO₂R^{a}, -NR^{c}CONR^{b}R^{c}, -CO₂R^{b}, -CONR^{b}R^{c}, -NR^{c}COR^{b}, -SOR^{a}, -SO₂R^{a}, -SO₂NR^{b}R^{c}, and -NR^{c}SO₂R^{a},
   where R^{a} is chosen from optionally substituted C₁-C₆ alkyl, optionally substituted alkenyl, optionally substituted alkynyl, optionally substituted aryl, and optionally substituted heteroaryl;
R^{b} is chosen from H, optionally substituted C₁-C₆ alkyl, optionally substituted cycloalkyl, optionally substituted heterocycloalkyl, optionally substituted aryl, and optionally substituted heteroaryl; and
R^{c} is independently chosen from hydrogen and optionally substituted C₁-C₄ alkyl; or
R^{b} and R^{c}, and the nitrogen to which they are attached, form an optionally substituted heterocycloalkyl group; and
where each optionally substituted group is unsubstituted or independently substituted with one or more, such as one, two, or three, substituents independently selected from C₁-C₄ alkyl, aryl, heteroaryl, aryl-C₁-C₄ alkyl-, heteroaryl-C₁-C₄ alkyl-, C₁-C₄ haloalkyl-, -OC₁-C₄ alkyl, -OC₁-C₄ alkylphenyl, -C₁-C₄ alkyl-OH, -OC₁-C₄ haloalkyl, halo, -OH, -NH₂, -C₁-C₄ alkyl-NH₂, -N(C₁-C₄ alkyl)(C₁-C₄ alkyl), -NH(C₁-C₄ alkyl), -N(C₁-C₄ alkyl)(C₁-C₄ alkylphenyl), -NH(C₁-C₄ alkylphenyl), cyano, nitro, oxo (as a substitutent for cycloalkyl, heterocycloalkyl, or heteroaryl), -CO₂H, -C(O)OC₁-C₄ alkyl, -CON(C₁-C₄ alkyl)(C₁-C₄ alkyl), -CONH(C₁-C₄ alkyl), -CONH₂, -NHC(O)(C₁-C₄ alkyl), -NHC(O)(phenyl), -N(C₁-C₄ alkyl)C(O)(C₁-C₄ alkyl), -N(C₁-C₄ alkyl)C(O)(phenyl), -C(O)C₁-C₄ alkyl, -C(O)C₁-C₄ alkylphenyl, -C(O)C₁-C₄ haloalkyl, -OC(O)C₁-C₄ alkyl, - SO₂(C₁-C₄ alkyl), -SO₂(phenyl), -SO₂(C₁-C₄ haloalkyl), -SO₂NH₂, -SO₂NH(C₁-C₄ alkyl), -SO₂NH(phenyl), -NHSO₂(C₁-C₄ alkyl), -NHSO₂(phenyl), and -NHSO₂(C₁-C₄ haloalkyl). In some embodiments, a substituted alkoxy group is "polyalkoxy" or -0-(optionally substituted alkylene)-(optionally substituted alkoxy), and includes groups such as -OCH₂CH₂OCH₃, and residues of glycol ethers such as polyethyleneglycol, and -O(CH₂CH₂O)ₓCH₃, where x is an integer of 2-20, such as 2-10, and for example, 2-5. Another substituted alkoxy group is hydroxyalkoxy or -OCH₂(CH₂)_{y}OH, where y is an integer of 1-10, such as 1-4.

The term "substituted alkoxycarbonyl" refers to the group (substituted alkyl)-O-C(O)- wherein the group is attached to the parent structure through the carbonyl functionality and wherein substituted refers to alkyl wherein one or more (such as up to 5, for example, up to 3) hydrogen atoms are replaced by a substituent independently chosen from:
-R^{a}, -OR^{b}, -SR^{b}, guanidine, guanidine wherein one or more of the guanidine hydrogens are replaced with a lower-alkyl group, -NR^{b}R^{c}, halo, cyano, nitro, -COR^{b}, -CO₂R⁶, -CONR^{b}R^{c}, -OCOR^{b}, -OCO₂R^{a}, -OCONR^{b}R^{c}, -NR^{c}COR^{b}, -NR^{c}CO₂R^{a}, -NR^{c}CONR^{b}R^{c}, -CO₂R^{b}, -CONR^{b}R^{c}, -NR^{c}COR^{b}, -SOR^{a}, -SO₂R^{a}, -SO₂NR^{b}R^{c}, and -NR^{C}SO₂R^{a},
   where R^{a} is chosen from optionally substituted C₁-C₆ alkyl, optionally substituted alkenyl, optionally substituted alkynyl, optionally substituted aryl, and optionally substituted heteroaryl;
R^{b} is chosen from H, optionally substituted C₁-C₆ alkyl, optionally substituted cycloalkyl, optionally substituted heterocycloalkyl, optionally substituted aryl, and optionally substituted heteroaryl; and
R^{c} is independently chosen from hydrogen and optionally substituted C₁-C₄ alkyl; or
R^{b} and R^{c}, and the nitrogen to which they are attached, form an optionally substituted heterocycloalkyl group; and
where each optionally substituted group is unsubstituted or independently substituted with one or more, such as one, two, or three, substituents independently selected from C₁-C₄ alkyl, aryl, heteroaryl, aryl-C₁-C₄ alkyl,-, heteroaryl-C₁-C₄ alkyl-, C₁-C₄ haloalkyl-, -OC₁-C₄ alkyl, -OC₁-C₄ alkylphenyl, -C₁-C₄ alkyl-OH, -OC₁-C₄ haloalkyl, halo, -OH, -NH₂, -C₁-C₄ alkyl-NH₂, -N(C₁-C₄ alkyl)(C₁-C₄ alkyl), -NH(C₁-C₄ alkyl), -N(C₁-C₄ alkyl)(C₁-C₄ alkylphenyl), -NH(C₁-C₄ alkylphenyl), cyano, nitro, oxo (as a substitutent for cycloalkyl, heterocycloalkyl, or heteroaryl), -CO₂H, -C(O)OC₁-C₄ alkyl, -CON(C₁-C₄ alkyl)(C₁-C₄ alkyl), -CONH(C₁-C₄ alkyl), -CONH₂, -NHC(O)(C₁-C₄ alkyl), -NHC(O)(phenyl), -N(C₁-C₄ alkyl)C(O)(C₁-C₄ alkyl), -N(C₁-C₄ alkyl)C(O)(phenyl), -C(O)C₁-C₄ alkyl, -C(O)C₁-C₄ alkylphenyl, -C(O)C₁-C₄ haloalkyl, -OC(O)C₁-C₄ alkyl, - SO₂(C₁-C₄ alkyl), -SO₂(phenyl), -SO₂(C₁-C₄ haloalkyl), -SO₂NH₂, -SO₂NH(C₁-C₄ alkyl), -SO₂NH(phenyl), -NHSO₂(C₁-C₄ alkyl), -NHSO₂(phenyl), and -NHSO₂(C₁-C₄ haloalkyl).

The term "substituted amino" refers to the group -NHR^{d} or NR^{d}R^{e} wherein R^{d} is chosen from: hydroxy, optionally substitued alkoxy, optionally substituted alkyl, optionally substituted cycloalkyl, optionally substituted acyl, aminocarbonyl, optionally substituted aryl, optionally substituted heteroaryl, optionally substituted heterocycloalkyl, optionally substituted alkoxycarbonyl, sulfinyl and sulfonyl, and wherein R^{e} is chosen from: optionally substituted alkyl, optionally substituted cycloalkyl, optionally substituted aryl, optionally substituted heteroaryl, and optionally substituted heterocycloalkyl, and wherein substituted alkyl, cycloalkyl, aryl, heterocycloalkyl, and heteroaryl refer respectively to alkyl, cycloalkyl, aryl, heterocycloalkyl, and heteroaryl wherein one or more (such as up to 5, for example, up to 3) hydrogen atoms are replaced by a substituent independently chosen from:
-R^{a}, -OR^{b}, -SR^{b}, guanidine, guanidine wherein one or more of the guanidine hydrogens are replaced with a lower-alkyl group, -NR^{b}R^{c}, halo, cyano, nitro, -COR^{b}, -CO₂R^{b}, -CONR^{b}R^{c}, -OCOR^{b}, -OCO₂R^{a} -OCONR^{b}R^{c}, -NR^{c}COR^{b}, -NR^{c}CO₂R^{a}, -NR^{c}CONR^{b}R^{c} -CO₂R^{b}, -CONR^{b}R^{c}, -NR^{c}COR^{b}, -SOR^{a}, -SO₂R^{a}, -SO₂NR^{b}R^{c}, and -NR^{c}SO₂R^{a},
   where R^{a} is chosen from optionally substituted C₁-C₆ alkyl, optionally substituted alkenyl, optionally substituted alkynyl, optionally substituted aryl, and optionally substituted heteroaryl;
R^{b} is chosen from H, optionally substituted C₁-C₆ alkyl, optionally substituted cycloalkyl, optionally substituted heterocycloalkyl, optionally substituted aryl, and optionally substituted heteroaryl; and
R^{c} is independently chosen from hydrogen and optionally substituted C₁-C₄ alkyl; or
R^{b} and R^{c}, and the nitrogen to which they are attached, form an optionally substituted heterocycloalkyl group; and
where each optionally substituted group is unsubstituted or independently substituted with one or more, such as one, two, or three, substituents independently selected from C₁-C₄ alkyl, aryl, heteroaryl, aryl-C₁-C₄ alkyl-, heteroaryl-C₁-C₄ alkyl-, C₁-C₄ haloalkyl-, -OC₁-C₄ alkyl, -OC₁-C₄ alkylphenyl, -C₁-C₄ alkyl-OH, -OC₁-C₄ haloalkyl, halo, -OH, -NH₂, -C₁-C₄ alkyl-NH₂, -N(C₁-C₄ alkyl)(C₁-C₄ alkyl), -NH(C₁-C₄ alkyl), -N(C₁-C₄ alkyl)(C₁-C₄ alkylphenyl), -NH(C₁-C₄ alkylphenyl), cyano, nitro, oxo (as a substitutent for cycloalkyl, heterocycloalkyl, or heteroaryl), -CO₂H, -C(O)OC₁-C₄ alkyl, -CON(C₁-C₄ alkyl)(C₁-C₄ alkyl), -CONH(C₁-C₄ alkyl), -CONH₂, -NHC(O)(C₁-C₄ alkyl), -NHC(O)(phenyl), -N(C₁-C₄ alkyl)C(O)(C₁-C₄ alkyl), -N(C₁-C₄ alkyl)C(O)(phenyl), -C(O)C₁-C₄ alkyl, -C(O)C₁-C₄ alkylphenyl, -C(O)C₁-C₄ haloalkyl, -OC(O)C₁-C₄ alkyl, - SO₂(C₁-C₄ alkyl), -SO₂(phenyl), -SO₂(C₁-C₄ haloalkyl), -SO₂NH₂, -SO₂NH(C₁-C₄ alkyl), -SO₂NH(phenyl), -NHSO₂(C₁-C₄ alkyl), -NHSO₂(phenyl), and -NHSO₂(C₁-C₄ haloalkyl); and
wherein optionally substituted acyl, optionally substituted alkoxycarbonyl, sulfinyl and sulfonyl are as defined herein.

The term "substituted amino" also refers to N-oxides of the groups -NHR^{d}, and NR^{d}R^{d} each as described above. N-oxides can be prepared by treatment of the corresponding amino group with, for example, hydrogen peroxide or m-chloroperoxybenzoic acid. The person skilled in the art is familiar with reaction conditions for carrying out the N-oxidation.

Compounds of Formula 1 include, but are not limited to, optical isomers of compounds of Formula 1, racemates, and other mixtures thereof. In addition, compounds of Formula I include Z- and E- forms (or *cis*- and *trans-* forms) of compounds with carbon-carbon double bonds. In those situations, the single enantiomers or diastereomers, i.e., optically active forms, can be obtained by asymmetric synthesis or by resolution of the racemates. Resolution of the racemates can be accomplished, for example, by conventional methods such as crystallization in the presence of a resolving agent, or chromatography, using, for example a chiral high-pressure liquid chromatography (HPLC) column. Where compounds of Formula 1 exists in various tautomeric forms, chemical entities of the present invention include all tautomeric forms of the compound.

Chemical entities of the present invention include compounds of Formula 1 and pharmaceutically acceptable salts solvates, crystal forms, and mixtures thereof. Pharmaceutically acceptable forms of the compounds recited herein include pharmaceutically acceptable salts, solvates, crystal forms (including polymorphs and clathrates), chelates, non-covalent complexes, prodrugs, and mixtures thereof. In certain embodiments, the compounds described herein are in the form of pharmaceutically acceptable salts. Hence, the terms "chemical entity" and "chemical entities" also encompass pharmaceutically acceptable salts, solvates, crystal forms, chelates, non-covalent complexes, prodrugs, and mixtures unless otherwise stated.

"Pharmaceutically acceptable salts" include, but are not limited to salts with inorganic acids, such as hydrochlorate, phosphate, diphosphate, hydrobromate, sulfate, sulfinate, nitrate, and like salts; as well as salts with an organic acid, such as malate, maleate, fumarate, tartrate, succinate, citrate, acetate, lactate, methanesulfonate, p-toluenesulfonate, 2-hydroxyethylsulfonate, benzoate, salicylate, stearate, and alkanoate such as acetate, HOOC-(CH₂)ₙ-COOH where n is 0-4, and like salts. Similarly, pharmaceutically acceptable cations include, but are not limited to sodium, potassium, calcium, aluminum, lithium, and ammonium.

In addition, if the compound of Formula 1 is obtained as an acid addition salt, the free base can be obtained by basifying a solution of the acid salt Conversely, if the product is a free base, an addition salt, particularly a pharmaceutically acceptable addition salt, may be produced by dissolving the free base in a suitable organic solvent and treating the solution with an acid, in accordance with conventional procedures for preparing acid addition salts from base compounds. Those skilled in the art will recognize various synthetic methodologies that may be used to prepare non-toxic pharmaceutically acceptable addition salts.

Prodrugs of chemical entities provided herein are, for example ester or amide derivatives of the compounds of Formula 1. The term "prodrugs" includes any compounds that become compounds of Formula 1 when administered to a patient, e.g., upon metabolic processing of the prodrug. Examples of prodrugs include, but are not limited to, acetate, formate, and benzoate and like derivatives of functional groups (such as alcohol or amine groups) in the compounds of Formula 1.

The term "solvate" refers to the chemical entity formed by the interaction of a solvent and a compound. Suitable solvates are pharmaceutically acceptable solvates, such as hydrates, including monohydrates and hemi-hydrates.

The term "chelate" refers to the chemical entity formed by the coordination of a compound to a metal ion at two (or more) points.

The term "non-covalent complex" refers to the chemical entity formed by the interaction of a compound and another molecule wherein a covalent bond is not formed between the compound and the molecule. For example, complexation can occur through van der Waals interactions, hydrogen bonding, and electrostatic interactions (also called ionic bonding).

The term "active agent" is used to indicate a chemical entity which has biological activity. In certain embodiments, an "active agent" is a compound having pharmaceutical utility. For example an active agent may be an anti-cancer therapeutic.

The term "therapeutically effective amount" of a chemical entity of this invention means an amount effective, when administered to a human or non-human patient, to treat a disease, e.g., a therapeutically effective amount may be an amount sufficient to treat a disease or disorder responsive to kinase inhibition. The therapeutically effective amount may be ascertained experimentally, for example by assaying blood concentration of the chemical entity, or theoretically, by calculating bioavailability.

By "significant" is meant any detectable change that is statistically significant in a standard parametric test of statistical significance such as Student's T-test, where p < 0.05.

"Patient" refers to an animal, such as a mammal, for example a human, that has been or will be the object of treatment, observation or experiment. The methods of the invention can be useful in both human therapy and veterinary applications. In some embodiments, the patient is a mammal, and in some embodiments the patient is human.

By "angiogenic kinase" is meant a kinase involved in angiogenesis and includes but is not limited to a kinase chosen from EphB₄ VEGFR2and PDGFRβ.

By "oncogenic kinase" is meant a kinase having a direct role in a cell signaling pathway that leads to cellular transformation. When overexpressed or aberrantly expressed, such kinases may have oncogenic activity. Oncogenic kinases include but are not limited to c-Kit.

"Treatment" or "treating" means any treatment of a disease in a patient, including:
a) preventing the disease, that is, causing the clinical symptoms of the disease not to develop;
b) inhibiting the disease;
c) slowing or arresting the development of clinical symptoms; and/or
d) relieving the disease, that is, causing the regression of clinical symptoms.

"Diseases or disorders responsive to kinase modulation" refer to pathologic conditions that depend, at least in part, on the activity of one or more protein kinases, for example, angiogenic kinases and/or oncogenic kinases. Kinases either directly or indirectly participate in the signal transduction pathways of a variety of cellular activities including cell proliferation, differentiation, and invasion. Diseases responsive to kinase modulation include but are not limited to tumor growth, angiogenesis supporting solid tumor growth, and diseases characterized by excessive local vascularization such as diabetic retinopathy, macular degeneration, and inflammation.

"Change in angiogenesis" refers to a change in the vascular network or quality of vasculature. Change in angiogenesis may be measured by many parameters and, for instance, may be assessed by delayed appearance of neovascular structures, slowed development of neovascular structures, decreased occurrence of neovascular structures, changes in vascular permeability, changes in blood flow, slowed or decreased severity of angiogenesis-dependent disease effects, arrested angiogenic growth, or regression of previous angiogenic growth.

Provided herein is at least one chemical entity chosen from compounds of Formula 1 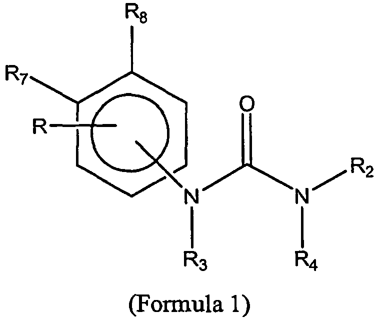 and pharmaceutically acceptable salts, solvates, crystal forms, chelates, non-covalent complexes, prodrugs, and mixtures thereof, wherein
R represents 0 to 2 substituents independently chosen from hydroxy, nitro, cyano, optionally substituted amino, aminocarbonyl, halo, carboxy, optionally substituted acyl, optionally substituted alkoxycarbonyl, optionally substituted C₁-C₆ alkyl, optionally substituted C₁-C₆ alkoxy, sulfanyl, sulfinyl, sulfonyl, optionally substituted aryl, optionally substituted heteroaryl, and optionally substituted heterocycloalkyl;
R₇ and R₈, taken together with the carbons to which they are bound, form a fused 5- to 7-membered heteroaryl ring substituted with a group -(Z₁)ₘR₁, wherein the fused 5- to 7-membered heteroaryl ring is optionally furrther substituted and wherein
R₁ is optionally substituted heteroaryl;
Z₁ is -CR₅R₆- wherein each R₅ and R₆ is independently chosen from hydrogen, optionally substituted C₁-C₆ alkyl, and halo; and
m is chosen from 0, 1, and 2;
R₂ is optionally substituted aryl; and
R₃ and R₄ are each independently chosen from hydrogen, optionally substituted C₁-C₆ alkyl, optionally substituted aryl, and optionally substituted heteroaryl, *provided that*
the compound of Formula 1 is not chosen from 5-(phenylcarbamoylamino)-3-(2-(4-pyridyl)ethyl)indole; 1-(4-chloro-3-(trifluoromethyl)phenyl)-3-(1-(pyridin-4-yl)-1H-indol-5-yl)urea; and 1-(2-methoxy-5-(trifluoromethyl)phenyl)-3-(1-(pyridin-4-yl)-1H-indol-5-yl)urea.

In certain embodiments, R represents 1 or 2 substituents independently chosen from halo, C₁-C₂ alkyl, and C₁-C₂ alkoxy.

In certain embodiments, R represents 1 or 2 substituents independently chosen from halo, methyl, and methoxy.

In certain embodiments, R represents a substituent chosen from halo, methyl, and methoxy.

In certain embodiments, R is absent.

In certain embodiments, R₇ and R₈, taken together with the carbons to which they are bound, form a fused 5-membered heteroaryl ring substituted with a group -(Z₁)ₘR₁ wherein the heteroaryl ring contains at least one nitrogen and optionally includes one or more additional heteroatoms, selected from N, O, and S in the ring.

In certain embodiments, R₇ and R₈, taken together with the carbons to which they are bound, form a fused ring chosen from pyrazolyl, imidazolyl, isoxazolyl,oxazolyl, thiazolyl, thiadiazolyl, triazolyl, and pyrrolyl, each of which is substituted with a group - (Z₁)ₘR₁. In certain embodiments, R₇ and R₈, taken together with the carbons to which they are bound, form a fused ring chosen from pyrazolyl, imidazolyl, isoxazolyl,oxazolyl, thiazolyl, triazolyl, and pyrrolyl, each of which is substituted with a group -(Z₁)ₘR₁.

In certain embodiments, R₇ and R₈, taken together with the carbons to which they are bound, form a fused heteroaryl ring chosen from 1H-pyrrolyl and 1H-pyrazolyl, each of which is substituted with a group -(Z₁)ₘR₁.

In certain embodiments, at least one of R₅ and R₆ is hydrogen. In certain embodiments, both of R₅ and R₆ are hydrogen.

In certain embodiments, m is 1 and at least one of R₅ and R₆ is hydrogen. In certain embodiments, both of R₅ and R₆ are hydrogen.

In certain embodiments, m is 0.

In certain embodiments, R₁ is chosen from pyridinyl and substituted pyridinyl wherein substituted pyridinyl is chosen from mono-, di-, and tri-substituted pyridinyls and wherein substituents on the substituted pyridinyl are independently chosen from hydroxy, nitro, cyano, optionally substituted amino, aminocarbonyl, halo, carboxy, optionally substituted acyl, optionally substituted alkoxycarbonyl, optionally substituted C₁-C₆ alkyl, optionally substituted C₁-C₆ alkoxy, sulfanyl, sulfinyl, sulfonyl, optionally substituted aryl, optionally substituted heteroaryl, and optionally substituted heterocycloalkyl.

In certain embodiments, the substituents on the substituted pyridinyl are independently chosen from hydroxy, nitro, cyano, optionally substituted amino, halo, carboxy, optionally substituted C₁-C₆ alkyl, optionally substituted C₁-C₆alkoxy, C₁-C₆ alkylsulfanyl, C₁-C₆ acyl, C₁-C₆alkoxycarbonyl, optionally substituted heteroaryl, and heterocycloalkyl.

In certain embodiments, the substituents on the substituted pyridinyl are independently chosen from hydroxy, cyano, halo, optionally substituted C₁-C₂ alkyl, optionally substituted C₁-C₂ alkoxy, and -NHR₁₀ wherein R₁₀ is chosen from hydrogen and optionally substituted acyl.

In certain embodiments, R₁ is chosen from pyridin-4-yl and substituted pyridin-4-yl wherein substituted pyridin-4-yl is chosen from mono-, di-, and tri-substituted pyridin-4-yls and wherein substituents on the substituted pyridin-4-yl are independently chosen from hydroxy, nitro, cyano, optionally substituted amino, aminocarbonyl, halo, carboxy, optionally substituted acyl, optionally substituted alkoxycarbonyl, optionally substituted C₁-C₆ alkyl, optionally substituted C₁-C₆ alkoxy, sulfanyl, sulfinyl, sulfonyl, optionally substituted aryl, optionally substituted heteroaryl, and optionally substituted heterocycloalkyl.

In certain embodiments, the substituents on the substituted pyridin-4-yl are independently chosen from hydroxy, nitro, cyano, optionally substituted amino, halo, carboxy, optionally substituted C₁-C₆ alkyl, optionally substituted C₁-C₆alkoxy, C₁-C₆ alkylsulfanyl, C₁-C₆ acyl, C₁-C₆alkoxycarbonyl, optionally substituted heteroaryl, and heterocycloalkyl.

In certain embodiments, the substituents on the substituted pyridin-4-yl are independently chosen from hydroxy, cyano, halo, optionally substituted C₁-C₂ alkyl, optionally substituted C₁-C₂ alkoxy, and -NHR₁₀ wherein R₁₀ is chosen from hydrogen and optionally substituted acyl.

In certain embodiments, R₁ is pyridin-4-yl.

In certain embodiments, R₂ is chosen from phenyl and substituted phenyl wherein substituted phenyl is chosen from mono-, di-, and tri-substituted phenyls and wherein substituents on the substituted phenyl are independently chosen from hydroxy, nitro, cyano, optionally substituted amino, aminocarbonyl, halo, carboxy, optionally substituted acyl, optionally substituted alkoxycarbonyl, optionally substituted C₁-C₆ alkyl, optionally substituted C₁-C₆ alkoxy, optionally substituted aryloxy, sulfanyl, sulfinyl, sulfonyl, optionally substituted aryl, optionally substituted heteroaryl, andoptionally substituted heterocycloalkyl.

In certain embodiments, the substituents on the substituted phenyl are independently chosen from hydroxy, nitro, cyano, optionally substituted amino, halo, carboxy, optionally substituted C₁-C₆ alkyl, optionally substituted C₁-C₆alkoxy, optionally substituted phenyl, optionally substituted phenoxy, C₁-C₆ alkylsulfanyl, C₁-C₆ acyl, C₁-C₆alkoxycarbonyl, optionally substituted heteroaryl, and heterocycloalkyl.

In certain embodiments, the substituents on the substituted phenyl are independently chosen from hydroxy, cyano, halo, optionally substituted C₁-C₂ alkyl, phenoxy, and optionally substituted C₁-C₂ alkoxy.

In certain embodiments, the substituents on the substituted phenyl are independently chosen from halo, methyl, ethyl, methoxy, ethoxy, difluoromethyl, trifluoromethyl, difluoromethoxy, and trifluoromethoxy.

In certain embodiments, R₃ and R₄ are each independently chosen from hydrogen and methyl.

In certain embodiments, R₃ and R₄ are hydrogen.

Provided herein is at least one chemical entity chosen from compounds of Formula 2 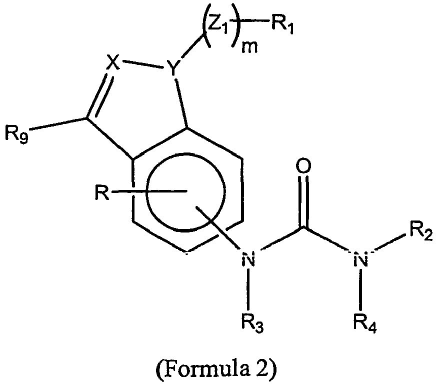 and pharmaceutically acceptable salts, solvates, crystal forms, chelates, non-covalent complexes, prodrugs, and mixtures thereof, wherein R₁, m, Z₁, R, R₂, R₃, and R₄ are as described for compounds of Formula 1 and wherein
X and Y are independently chosen from CH and N; and
R₉ is chosen from hydrogen and optionally substituted alkyl.

In certain embodiments, R₉ is chosen from hydrogen and optionally substituted lower alkyl. In certain embodiments, R₉ is chosen from hydrogen and lower alkyl. In certain embodiments, R₉ is hydrogen.

In certain embodiments, X and Y are N.

In certain embodiments, Y is N and X is CH.

Provided herein is at least one chemical entity chosen from compounds of Formula 3 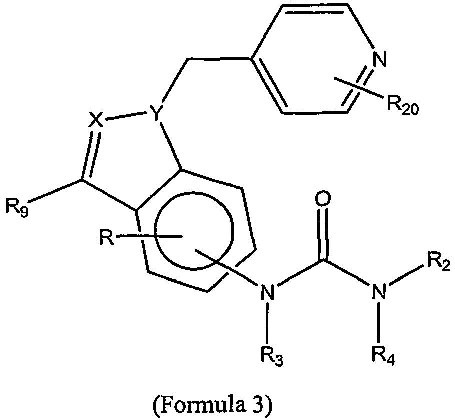 and pharmaceutically acceptable salts, solvates, crystal forms, chelates, non-covalent complexes, prodrugs, and mixtures thereof, wherein R, R₂, R₃, and R₄ are as described for compounds of Formula 1, wherein X, Y, and R₉ are as described for compounds of Formula 2, and wherein
R₂₀ represents 0 to 3 substituents independently chosen from hydroxy, nitro, cyano, optionally substituted amino, aminocarbonyl, halo, carboxy, optionally substituted acyl, optionally substituted alkoxycarbonyl, optionally substituted C₁-C₆ alkyl, optionally substituted C₁-C₆ alkoxy, sulfanyl, sulfinyl, sulfonyl, optionally substituted aryl, optionally substituted heteroaryl, and optionally substituted heterocycloalkyl.

In certain embodiments, R₂₀ is optionally substituted amino. In some embodiments, R₂₀ is amino.

Provided herein is at least one chemical entity chosen from compounds of Formula 4 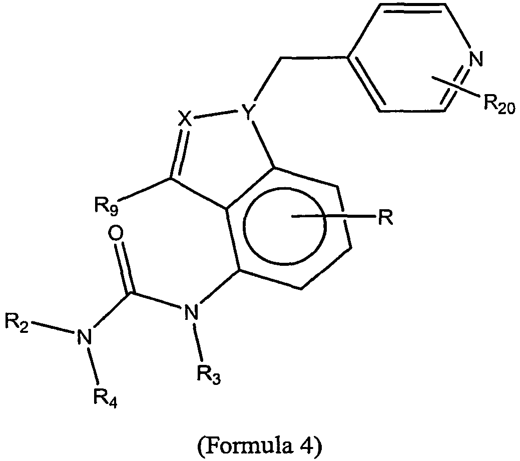 and pharmaceutically acceptable salts, solvates, crystal forms, chelates, non-covalent complexes, prodrugs, and mixtures thereof, wherein R, R₂, R₃, and R₄ are as described for compounds of Formula 1, wherein X, Y, and R₉ are as described for compounds of Formula 2, and wherein R₂₀ is as described for compounds of Formula 3.

Also provided is at least one chemical entity chosen from compounds of Formula 5 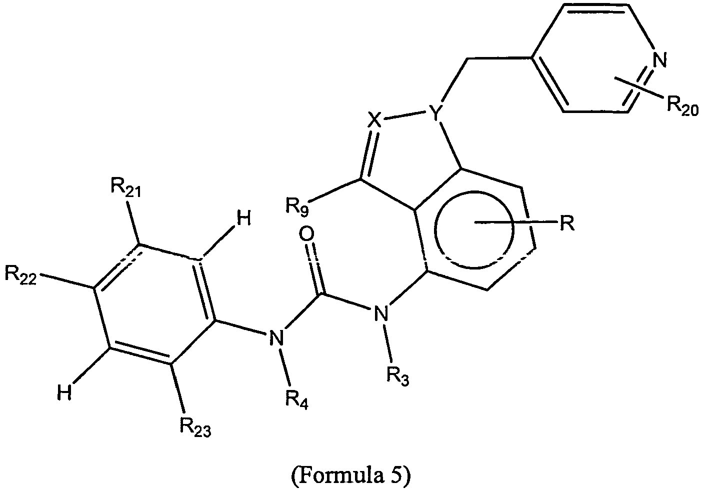 and pharmaceutically acceptable salts, solvates, crystal forms, chelates, non-covalent complexes, prodrugs, and mixtures thereof, wherein R, R₃, and R₄ are as described for compounds of Formula 1, wherein X, Y, and R₉ are as described for compounds of Formula 2, wherein R₂₀ is as described for compounds of Formula 3; and wherein
R₂₁ is chosen from hydrogen, halo and optionally substituted lower alkyl;
R₂₂ is chosen from hydrogen, halo, lower alkoxy, and lower alkyl; and
R₂₃ is chosen from hydrogen, lower alkyl, optionally substituted phenoxy, optionally substituted lower alkoxy, and halo.

In certain embodiments, R₂₁ is chosen from hydrogen, halo, methyl, and trifluoromethyl.

In certain embodiments, R₂₂ is chosen from hydrogen, halo, methoxy, and methyl.

In certain embodiments, R₂₂ is hydrogen.

In certain embodiments, R₂₃ is chosen from hydrogen, methyl, methoxy, difluoromethoxy, trifluoromethoxy, ethoxy, and halo.

In certain embodiments, at least one of R₂₁, R₂₂, and R₂₃ is not hydrogen.

Also provided is at least one chemical entity chosen from
1-(5-Bromo-2-methoxy-phenyl)-3-(1-pyridin-4-ylmethyl-1H-indol-4-yl)-urea;
1-(2-Methoxy-5-trifluoromethyl-phenyl)-3-(1-pyridin-4-ylmethyl-1H-indol-4-yl)-urea;
1-(2-Methoxy-5-trifluoromethyl-phenyl)-3-(1-pyridin-4-ylmethyl-1H-indol-6-yl)-urea;
1-(4-Chloro-2-methoxy-5-trifluoromethyl-phenyl)-3-(1-pyridin-4-ylmethyl-1H-indol-4-yl)-urea;
1-(2-Fluoro-5-trifluoromethyl-phenyl)-3-(1-pyridin-4-ylmethyl-1H-indol-4-yl)-urea;
1-(2,4-Dimethoxy-5-trifluoromethyl-phenyl)-3-(1-pyridin-4-ylmethyl-1H-indol-4-yl)-urea;
1-(2,4-Dimethyl-5-trifluoromethyl-phenyl)-3-(1-pyridin-4-ylmethyl-1H-indol-4-yl)-urea;
1-(2-Ethoxy-5-trifluoromethyl-phenyl)-3-(1-pyridin-4-yhnethyl-1H-indol-4-yl)-urea;
1-(5-Bromo-2-methoxy-phenyl)-3-(1-pyridin-4-ylmethyl-1H-indazol-4-yl)-urea;
1-(5-Bromo-2-ethoxy-phenyl)-3-(1-pyridin-4-ylmethyl-1 H-indol-4-yl)-urea;
1-(5-Bromo-2-methoxy-phenyl)-3-(1-pyridin-4-ylmethyl-1H-indol-5-yl)-urea;
1-(4-Methyl-3-trifluoromethyl-phenyl)-3-(1-pyridin-4-ylmethyl-1H-indol-4-yl)-urea;
1-(4-Chloro-3-trifluoromethyl-phenyl)-3-(1-pyridin-4-ylmethyl-1H-indol-4-yl)-urea;
1-(5-Chloro-2-methoxy-phenyl)-3-(1-pyridin-4-ylmethyl-1H-indol-5-yl)-urea;
1-(2-Methoxy-5-trifluoromethyl-phenyl)-3-(1-pyridin-4-ylmethyl-1H-indol-5-yl)-urea;
1-(4-Chloro-3-trifluoromethyl-phenyl)-3-(1-pyridin-4-ylmethyl-1H-indol-5-yl)-urea;
1-(2-Fluoro-5-trifluoromethyl-phenyl)-3-(1-pyridin-4-ylmethyl-1H-indol-5-yl)-urea;
1-(2-Chloro-5-trifluoromethyl-phenyl)-3-(1-pyridin-4-ylmethyl-1H-indol-5-yl)-urea;
1-(2-Fluoro-5-trifluoromethyl-phenyl)-3-(3-methyl-1-pyridin-4-ylmethyl-1H-indol-4-yl)-urea;
1-(2-Methoxy-5-trifluoromethyl-phenyl)-3-(3-methyl-1-pyridin-4-ylmethyl-1H-indol-4-yl)-urea;
1-(5-Bromo-2-methoxy-phenyl)-3-(3-methyl-1-pyridin-4-ylmethyl-1H-indol-4-yl)-urea;
1-(5-Bromo-2-methoxy-phenyl)-3-(1-pyridin-3-ylmethyl-1H-indol-4-yl)-urea;
1-(5-Bromo-2-methoxy-phenyl)-3-(1-pyridin-3-ylmethyl-1H-indol-5-yl)-urea;
1-(5-Bromo-2-methoxy-phenyl)-3-(5-methyl-1-pyridin-4-ylmethyl-1H-indol-4-yl)-urea;
1-(5-Bromo-2-methoxy-phenyl)-3-(7-methyl-1-pyridin-4-ylmethyl-1H-indol-4-yl)-urea;
1-(2-Methoxy-5-trifluoromethyl-phenyl)-3-(5-methyl-1-pyridin-4-ylmethyl-1H-indol-4-yl)-urea;
1-(2-Fluoro-5-trifluoromethyl-phenyl)-3-(5-methyl-1-pyridin-4-ylmethyl-1H-indol-4-yl)-urea;
1-(5-Chloro-2-methoxy-phenyl)-3-(5-methyl-1-pyridin-4-ylmethyl-1H-indol-4-yl)-urea;
1-(4-Methoxy-biphenyl-3-yl)-3-(1-pyridin-4-ylmethyl-1H-indol-4-yl)-urea;
1-(2-Methoxy-5-trifluoromethyl-phenyl)-3-(7-methyl-1-pyridin-4-ylmethyl-1H-indol-4-yl)-urea;
1-(2-Fluoro-5-trifluoromethyl-phenyl)-3-(7-methyl-1-pyridin-4-ylmethyl-1H-indol-4-yl)-urea;
1-(5-Chloro-2-methoxy-phenyl)-3-(7-methyl-1-pyridin-4-ylmethyl-1H-indol-4-yl)-urea;
4- {4-[3-(2-Methoxy-5-trifluoromethyl-phenyl)-ureido]-5-methyl-indol-1-ylmethyl}-pyridine-2-carboxylic acid methylamide;
1-(7-Methyl-1-pyridin-4-ylmethyl-1H-indol-4-yl)-3-(4-methyl-3-trifluoromethyl-phenyl)-urea;
1-(4-Chloro-3-trifluoromethyl-phenyl)-3-(7-methyl-1-pyridin-4-ylmethyl-1H-indol-4-yl)-urea;
1-[5-Chloro-2-([1,3]dioxolan-2-ylmethoxy)-phenyl]-3-(1-pyridin-4-ylmethyl-1H-indol-4-yl)-urea;
1-[5-Chloro-2-(3-hydroxy-propoxy)-phenyl]-3-(5-methyl-1-pyridin-4-ylmethyl-1H-indol-4-yl)-urea;
1-(3-Chloro-4-methoxy-phenyl)-3-(1-pyridin-4-ylmethyl-1H-indol-4-yl)-urea;
1-(4-Methoxy-3-trifluoromethyl-phenyl)-3-(1-pyridin-4-ylmethyl-1H-indol-4-yl)-urea;
1-(2-Methoxy-4-methyl-5-trifluoromethyl-phenyl)-3-(1-pyridin-4-ylmethyl-1H-indol-4-yl)-urea;
1-(3-Bromo-4-methyl-phenyl)-3-(1-pyridin-4-ylmethyl-1H-indol-4-yl)-urea;
1-(4-Fluoro-3-trifluoromethyl-phenyl)-3-(1-pyridin-4-ylmethyl-1H-indol-4-yl)-urea;
1-(5-Bromo-2-methoxy-4-methyl-phenyl)-3-(1-pyridin-4-ylmethyl-1H-indol-4-yl)-urea;
1-(5-Bromo-2-methoxy-phenyl)-3-[1-(2-fluoro-pyridin-4-ylmethyl)-1H-indol-4-yl]-urea;
1-(5-Bromo-2-methoxy-phenyl)-3-[1-(2-methyl-pyridin-4-ylmethyl)-1H-indol-4-yl]-urea;
1-(3-Bromo-4-methoxy-phenyl)-3-(1-pyridin-4-ylmetllyl-1H-indol-4-yl)-urea;
1-(5-Bromo-2-methoxy-phenyl)-3-[1-(3-fluoro-pyridin-4-ylmethyl)-1H-indol-4-yl]-urea;
1-[1-(3-Fluoro-pyridin-4-ylmethyl)-1H-indol-4-yl]-3-(4-methyl-3-trifluoromethyl-phenyl)-urea;
1-(3-Chloro-4-fluoro-phenyl)-3-(1-pyridin-4-ylmethyl-1H-indol-4-yl)-urea;
1-[1-(2-Amino-pyridin-4-ylmethyl)-1H-indol-4-yl]-3-(5-bromo-2-methoxy-phenyl)-urea;
1-(3,4-Dimethoxy-phenyl)-3-(1-pyridin-4-ylmethyl-1H-indol-4-yl)-urea;
1-(3-Chloro-4-methyl-phenyl)-3-(1-pyridin-4-ylmethyl-1H-indol-4-yl)-urea;
1-(5-Chloro-2-methoxy-4-methyl-phenyl)-3-(1-pyridin-4-ylmethyl-1H-indol-4-yl)-urea;
1-[1-(2-Amino-pyridin-4-ylmethyl)-1H-indol-4-yl]-3-(5-bromo-2-methoxy-4-methylphenyl)-urea;
1-[1-(2-Amino-pyridin-4-ylmethyl)-1H-indol-4-yl]-3-(3-chloro-4-methyl-phenyl)-urea;
1-[1-(2-Amino-pyridin-4-ylmethyl)-1H-indol-4-yl]-3-(5-chloro-2-methoxy-phenyl)-urea;
1-[1-(2-Amino-pyridin-4-ylmethyl)-1H-indol-4-yl]-3-(5-chloro-2,4-dimethoxy-phenyl)-urea;
1-[1-(2-Amino-pyridin-4-ylmethyl)-1H-indol-4-yl]-3-(5-chloro-2-methoxy-4-methylphenyl)-urea;
1-[1-(2-Amino-pyridin-4-ylmethyl)-1H-indol-4-yl]-3-(2,4-dimethyl-5-trifluoromethylphenyl)-urea;
i -(5-Bromo-2-methoxy-phenyl)-3-(5-methoxy-1-pyridin-4-ylmethyl-1H-indol-4-yl)-urea;
1-[1-(2-Amino-pyridin-4-ylmethyl)-1H-indol-4-yl]-3-(2-methoxy-5-trifluoromethyl-phenyl)-urea;
1-[1-(2-Amino-pyridin-4-ylmethyl)-1H-indol-4-yl]-3-(2,4-dimethoxy-5-trifluoromethylphenyl)-urea;
1-[1-(2-Amino-pyridin-4-ylmethyl)-1H-indol-4-yl]-3-(2-methoxy-4-methyl-5-trifluoromethyl-phenyl)-urea;
1-Benzo[1,3]dioxol-5-yl-3-(1-pyridin-4-ylmethyl-1H-indol-4-yl)-urea;
1-(5-Bromo-2-methoxy-phenyl)-3-(6-methyl-1-pyridin-4-ylmethyl-1H-indol-4-yl)-urea;
(4-{4-[3-(5-Bromo-2-methoxy-phenyl)-ureido]-indol-1-ylmethyl}-pyridin-2-yl)-carbamic acid methyl ester;
1-(5-Bromo-2-methoxy-phenyl)-3-[1-(3-chloro-pyridin-4-ylmethyl)-1H-indol-4-yl]-urea;
1-(5-Fluoro-2,4-dimethoxy-phenyl)-3-(1-pyridin-4-ylmethyl-1H-indol-4-yl)-urea;
1-(3-Bromo-4-fluoro-phenyl)-3-(1-pyridin-4-ylmethyl-1H-indol-4-yl)-urea;
N-(4- {4-[3-(5-Bromo-2-methoxy-phenyl)-ureido]-indol-1-ylmethyl}-pyridin-2-yl)-acetamide;
1-(5-Ethanesulfonyl-2-methoxy-phenyl)-3-(1-pyridin-4-ylmethyl-1H-indol-4-yl)-urea;
1-(5-Bromo-2,4-difluoro-phenyl)-3-(1-pyridin-4-ylmethyl-1H-indol-4-yl)-urea;
1-[1-(2-Amino-pyridin-4-ylmethyl)-1H-indol-4-yl]-3-(2-fluoro-5-trifluoromethyl-phenyl)-urea;
N-(4-{4-[3-(5-Chloro-2-methoxy-4-methyl-phenyl)-ureido]-indol-1-ylmethyl}-pyridin-2-yl)-acetamide;
1-(5-Bromo-2-methoxy-phenyl)-3-(7-fluoro-1-pyridin-4-ylmethyl-1H-indol-4-yl)-urea;
1-(5-Chloro-2-methoxy-4-methyl-phenyl)-3-(7-fluoro-1-pyridin-4-ylmethyl-1H-indol-4-yl)-urea;
N-(4-{4-[3-(5-Bromo-2-methoxy-phenyl)-ureido]-indol-1-ylmethyl}-pyridin-2-yl)-2-hydroxy-acetamide;
2-Amino-N-(4-{4-[3-(5-bromo-2-methoxy-phenyl)-ureido]-indol-1-ylmethyl}-pyridin-2-yl)-acetamide;
N-(4-{4-[3-(5-Bromo-2-methoxy-phenyl)-ureido]-indol-1-ylmethyl}-pyridin-2-yl)-formamide;
1-[1-(2-Amino-pyridin-4-ylmethyl)-1H-indol-4-yl]-3-(3-bromo-4-methoxy-phenyl)-urea;
N-(4-{4-[3-(3-Chloro-4-methyl-phenyl)-ureido]-indol-1-ylmethyl}-pyridin-2-yl)-acetamide;
1-(5-Bromo-2-methoxy-phenyl)-3-(7-methoxy-1-pyridin-4-ylmethyl-1 H-indol-4-yl)-urea;
1-(2,4-Dimethoxy-5-trifluoromethyl-phenyl)-3-(7-methoxy-1-pyridin-4-ylmethyl-1H-indol-4-yl)-urea;
1-(5-Bromo-2-methoxy-phenyl)-3-(5-fluoro-1-pyridin-4-ylmethyl-1H-indol-4-yl)-urea;
N-(4- {4-[3-(5-Chloro-2-methoxy-phenyl)-ureido]-indol-1-ylmethyl}-pyridin-2-yl)-acetamide;
1-Methyl-1H-imidazole-2-carboxylic acid (4-{4-[3-(5-bromo-2-methoxy-phenyl)-ureido]-indol-1-ylmethyl}-pyridin-2-yl)-amide;
N-(4- {4-[3-(5-Bromo-2-methoxy-phenyl)-ureido]-indol-1-ylmethyl}-pyridin-2-yl)-propionamide;
N-(4- {4-[3-(5-Bromo-2-methoxy-phenyl)-ureido]-indol-1-ylmethyl}-pyridin-2-yl)-isobutyramide;
N-(4- {4-[3-(5-Bromo-2-methoxy-phenyl)-ureido]-indol-1-ylmethyl}-pyridin-2-yl)-2-morpholin-4-yl-acetamide;
1-(5-Bromo-2-methoxy-phenyl)-3-[1-(2-methylamino-pyridin-4-ylmethyl)-1H-indol-4-yl]-urea;
1-(5-Bromo-2-methoxy-phenyl)-3-{1-[2-(2-methoxy-ethylamino)-pyridin-4-ylmethyl]-1H-indol-4-yl}-urea;
1-(5-Bromo-2-methoxy-phenyl)-3-[1-(2-morpholin-4-yl-pyridin-4-ylmethyl)-1H-indol-4-yl]-urea;
1-(5-Bromo-2-methoxy-phenyl)-3-[1-(2-methoxy-pyridin-4-ylmethyl)-1H-indol-4-yl]-urea;
1-(5-Bromo-2-methoxy-phenyl)-3-[1-(2-hydroxy-pyridin-4-ylmethyl)-1H-indol-4-yl]-urea;
N-(4- {4-[3-(2-Fluoro-5-trifluoromethyl-phenyl)-ureido]-indol-1-ylmethyl}-pyridin-2-yl)-acetamide;
N-(4- {4-[3-(5-Chloro-2-methoxy-phenyl)-ureido]-indol-1-ylmethyl}-pyridin-2-yl)-2-morpholin-4-yl-acetamide;
N-(4- {4-[3-(5-Bromo-2-methoxy-phenyl)-ureido]-indol-1-ylmethyl}-pyridin-2-yl)-2-methoxy-acetamide;
1-(5-Chloro-2-methoxy-phenyl)-3-[1-(3-methyl-pyridin-4-ylmethyl)-1H-indol-4-yl]-urea;
Cyclopropanecarboxylic acid (4-{4-[3-(5-chloro-2-methoxy-phenyl)-ureido]-indol-1-ylmethyl}-pyridin-2-yl)-amide;
1-[1-(2-Amino-pyridin-4-ylmethyl)-1H-indol-4-yl]-3-(5-bromo-2-difluoromethoxy-phenyl)-urea;
N-(4-{4-[3-(5-Bromo-2-difluoromethoxy-phenyl)-ureido]-indol-1-ylmethyl}-pyridin-2-yl)-acetamide;
N-(4-{4-[3-(5-Chloro-2-methoxy-phenyl)-ureido]-indol-1-ylmethyl}-pyridin-2-yl)-3-diethylamino-propionamide;
1-(5-Bromo-2-methoxy-phenyl)-3-(7-chloro-1-pyridin-4-ylmethyl-1H-indol-4-yl)-urea;
1-(5-Bromo-2-methoxy-phenyl)-3-{1-[2-(2-hydroxy-ethylamino)-pyridin-4-ylmethyl]-1H-indol-4-yl}-urea;
1-(5-Bromo-2-methoxy-phenyl)-3-(5-chloro-1-pyridin-4-ylmethyl-1H-indol-4-yl)-urea;
N-(4-{4-[3-(5-Bromo-2-methoxy-phenyl)-ureido]-indol-1-ylmethyl}-pyridin-2-yl)-2-pyrrolidin-1-yl-acetamide;
N-(4-{4-[3-(5-Bromo-2-methoxy-phenyl)-ureido]-indol-1-ylmethyl}-pyridin-2-yl)-2-dimethylamino-acetamide;
N-(4-{4-[3-(5-Bromo-2-methoxy-phenyl)-ureido]-indol-1-ylmethyl}-pyridin-2-yl)-2-methylamino-acetamide;
1-[1-(2-Amino-pyridin-4-ylmethyl)-5-fluoro-1H-indol-4-yl]-3-(5-bromo-2-methoxy-phenyl)-urea;
Cyclopropanecarboxylic acid (4-{4-[3-(5-bromo-2-methoxy-phenyl)-ureido]-indol-1-ylmethyl}-pyridin-2-yl)-amide;
Cyclopropanecarboxylic acid (4- {4-[3-(2-methoxy-5-trifluoromethyl-phenyl)-ureido]-indol-1-ylmethyl}-pyridin-2-yl)-amide;
Cyclopropanecarboxylic acid (4-{4-[3-(2-fluoro-5-trifluoromethyl-phenyl)-ureido]-indol-1-ylmethyl}-pyridin-2-yl)-amide;
1-[1-(2-Amino-pyridin-4-ylmethyl)-5-fluoro-1H-indol-4-yl]-3-(2-fluoro-5-trifluoromethylphenyl)-urea;
1-[1-(2-Amino-pyridin-4-ylmethyl)-5-fluoro-1H-indol-4-yl]-3-(2-methoxy-5-trifluoromethylphenyl)-urea;
Cyclopropanecarboxylic acid (4-{4-[3-(3-chloro-4-methyl-phenyl)-ureido]-indol-1-ylmethyl}-pyridin-2-yl)-amide;
N-(4-{4-[3-(5-Bromo-2-methoxy-phenyl)-ureido]-indol-1-ylmethyl}-pyridin-2-yl)-methanesulfonamide;
N-(4-{4-[3-(5-Chloro-2-methoxy-phenyl)-ureido]-indol-1-ylmethyl}-pyridin-2-yl)-methanesulfonamide;
1-[1-(2-Amino-pyridin-4-ylmethyl)-5-fluoro-1H-indol-4-yl]-3-(2,5-dichloro-phenyl)-urea;
1-[1-(2-Amino-pyridin-4-ylmethyl)-5-fluoro-1H-indol-4-yl]-3-(5-chloro-2-methoxy-phenyl)-urea;
1-(5-Chloro-2-methoxy-phenyl)-3-{1-[2-(pyrazin-2-ylamino)-pyridin-4-ylmethyl]-1H-indol-4-yl}-urea;
1-(5-Chloro-2-methoxy-phenyl)-3-{1-[2-(thiazol-2-ylamino)-pyridin-4-ylmethyl]-1H-indol-4-yl}-urea;
1-[1-(2-Amino-pyridin-4-ylmethyl)-7-fluoro-1H-indol-4-yl]-3-(5-bromo-2-methoxy-phenyl)-urea;
N-(4-{4-[3-(5-Chloro-2-methoxy-phenyl)-ureido]-indol-1-ylmethyl}-pyridin-2-yl)-2-methylamino-acetamide;
N-(4-{4-[3-(5-Bromo-2-methoxy-phenyl)-ureido]-indol-1-ylmethyl}-pyridin-2-yl)-3-diethylamino-propionamide;
1-[1-(2-Amino-pyridin-4-ylmethyl)-7-fluoro-1H-indol-4-yl]-3-(2-fluoro-5-trifluoromethylphenyl)-urea;
1-(2-Fluoro-5-trifluoromethyl-phenyl)-3-{1-[2-(pyridin-2-ylamino)-pyridin-4-ylmethyl]-1H-indol-4-yl}-urea;
1-(5-Bromo-2-methoxy-phenyl)-3-{1-[2-(3-isopropyl-ureido)-pyridin-4-ylmethyl]-1H-indol-4-yl}-urea;
3-(Acetyl-methyl-amino)-N-(4-{4-[3-(5-chloro-2-methoxy-phenyl)-ureido]-indol-1-ylmethyl}-pyridin-2-yl)-propionamide;
N-(4-{4-[3-(2-Fluoro-5-trifluoromethyl-phenyl)-ureido]-indol-1-ylmethyl}-pyridin-2-yl)-2-methylamino-acetamide;
1-[1-(2-Allylamino-pyridin-4-ylmethyl)-1H-indol-4-yl]-3-(5-bromo-2-methoxy-phenyl)-urea;
3-Amino-N-(4-{4-[3-(5-chloro-2-methoxy-phenyl)-ureido]-indol-1-ylmethyl}-pyridin-2-yl)-propionamide;
1-(2-Fluoro-5-trifluoromethyl-phenyl)-3-{1-[2-(pyridin-3-ylamino)-pyridin-4-ylmethyl]-1H-indol-4-yl}-urea;
1-[1-(2-Amino-5-fluoro-pyridin-4-ylmethyl)-1H-indol-4-yl]-3-(5-bromo-2-methoxy-phenyl)-urea;
1-[1-(2-Amino-pyridin-4-ylmethyl)-5-chloro-1H-indol-4-yl]-3-(5-bromo-2-methoxy-phenyl)-urea;
1-[1-(2-Amino-3-methyl-pyridin-4-ylmethyl)-1H-indol-4-yl]-3-(5-bromo-2-methoxyphenyl)-urea;
1-(2-Fluoro-5-trifluoromethyl-phenyl)-3-{1-[2-(1H-tetrazol-5-yl)-pyridin-4-ylmethyl]-1H-indol-4-yl}-urea;
1-(2-Fluoro-5-tritluoromethyl-phenyl)-3-{1-[2-(pyrimidin-2-ylamino)-pyridin-4-ylmethyl]-1H-indol-4-yl}-urea;
1-[1-(2-Bromo-pyridin-4-ylmethyl)-1H-indol-4-yl]-3-(5-chloro-2-methoxy-phenyl)-urea;
1-[1-(2-Bromo-pyridin-4-ylmethyl)-1H-indol-4-yl]-3-(2-fluoro-5-trifluoromethyl-phenyl)-urea;
1-[1-(2-Cyano-pyridin-4-ylmethyl)-1H-indol-4-yl]-3-(2-fluoro-5-trifluoromethyl-phenyl)-urea;
1-(5-Chloro-2-methoxy-phenyl)-3-[1-(2-cyano-pyridin-4-ylmethyl)-1H-indol-4-yl]-urea;
1-(2-Fluoro-5-trifluoromethyl-phenyl)-3-{1-[2-(1H-imidazol-2-yl)-pyridin-4-ylmethyl]-1H-indol-4-yl}-urea;
1-(5-Chloro-2-methoxy-phenyl)-3-{1-[2-(4-cyano-phenylmino)-pyridin-4-ylmethyl]-1H-indol-4-yl}-urea; and
1-(5-Chloro-2-methoxy-phenyl)-3-{1-[2-(6-cyano-pyridin-3-ylamino)-pyridin-4-ylmethyl]-1H-indol-4-yl}-urea;
and pharmaceutically acceptable salts, solvates, crystal forms, chelates, non-covalent complexes, prodrugs, and mixtures thereof.

Methods for obtaining the novel compounds described herein will be apparent to those of ordinary skill in the art, suitable procedures being described, for example, in the reaction scheme and example below, and in the references cited herein.

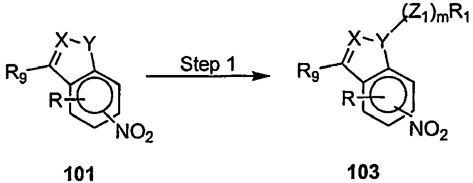

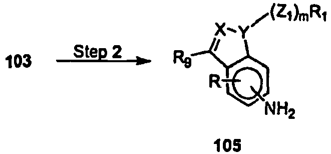

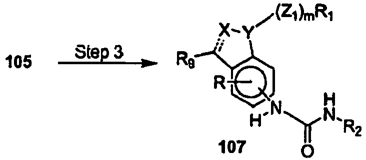

Referring to Reaction Scheme 1, Step 1, to a cooled solution of a compound of Formula 101wherein Y is NH and X is CH or N, in an inert solvent such as THF is added an excess, such as about 3 equivalents, of a base such as sodium hydride. The resulting reaction mixture is stirred at about 0° C for about 30 min and treated with an excess, such as about 1.5 equivalents, of a compound of Formula R₁-(Z₁)ₘ-X wherein X is a leaving group such as bromo. The reaction mixture is stirred for about 16 hrs while warming up to room temperature. The product, a compound of Formula 103, is isolated and optionally purified..

Referring to Reaction Scheme 1, Step 2, to a solution of a compound of Formula 103 in a polar, protic solvent such as EtOH/EtOAc is added catalytic amount of Pd/C and the reaction mixture is placed in a parr shaker with hydrogen gas. After about 3 hrs the H₂ gas uptake has ceased and the reaction mixture is filtered through a pad of Celite. The product, a compound of Formula 105, is isolated and optionally purified.

Referring to Reaction Scheme 1, Step 3, to a solution of a compound of Formula 105 in an inert solvent such as CH₂Cl₂ is added about one equivalent of an isocyanate of the formula R₂-NCO. The product, a compound of Formula 107, is isolated and optionally purified.

In some embodiments, the chemical entities described herein are administered as a pharmaceutical composition or formulation. Accordingly, the invention provides pharmaceutical formulations comprising at least one chemical entity chosen from compounds of Formula 1 and pharmaceutically acceptable salts, solvates, crystal forms, and mixtures thereof, together with at least one pharmaceutically acceptable vehicle chosen from carriers, adjuvants, and excipients.

Pharmaceutically acceptable vehicles must be of sufficiently high purity and sufficiently low toxicity to render them suitable for administration to the patient being treated. The vehicle can be inert or it can possess pharmaceutical benefits. The amount of vehicle employed in conjunction with the chemical entity is sufficient to provide a practical quantity of material for administration per unit dose of the chemical entity.

Exemplary pharmaceutically acceptable carriers or components thereof are sugars, such as lactose, glucose and sucrose; starches, such as corn starch and potato starch; cellulose and its derivatives, such as sodium carboxymethyl cellulose, ethyl cellulose, and methyl cellulose; powdered tragacanth; malt; gelatin; talc; solid lubricants, such as stearic acid and magnesium stearate; calcium sulfate; synthetic oils; vegetable oils, such as peanut oil, cottonseed oil, sesame oil, olive oil, and corn oil; polyols such as propylene glycol, glycerine, sorbitol, mannitol, and polyethylene glycol; alginic acid; phosphate buffer solutions; emulsifiers, such as the TWEENS; wetting agents, such sodium lauryl sulfate; coloring agents; flavoring agents; tableting agents; stabilizers; antioxidants; preservatives; pyrogen-free water; isotonic saline; and phosphate buffer solutions.

Optional active agents may be included in a pharmaceutical composition, which do not substantially interfere with the activity of the chemical entity of the present invention.

A therapeutically effective amount of at least one chemical entity chosen from compounds of Formula 1 and pharmaceutically acceptable salts, solvates, crystal forms, chelates, non-covalent complexes, prodrugs, and mixtures thereof, is mixed with a suitable pharmaceutical acceptable vehicle. In instances in which the chemical entity exhibits insufficient solubility, methods for solubilizing compounds may be used. Such methods are known to those of skill in this art, and include, but are not limited to, using cosolvents, such as dimethylsulfoxide (DMSO), using surfactants, such as TWEEN, or dissolution in aqueous sodium bicarbonate.

Upon mixing or addition of the chemical entity described herein, the resulting mixture may be a solution, suspension, emulsion or the like. The form of the resulting mixture depends upon a number of factors, including the intended mode of administration and the solubility of the chemical entity in the chosen vehicle. The therapeutically effective amount of the chemical entity may be empirically determined.

Chemical entities described herein may be administered orally, topically, parenterally, intravenously, by intramuscular injection, by inhalation or spray, sublingually, transdermally, via buccal administration, rectally, as an ophthalmic solution, or by other means, in dosage unit formulations.

Dosage formulations suitable for oral use, include, for example, tablets, troches, lozenges, aqueous or oily suspensions, dispersible powders or granules, emulsions, hard or soft capsules, or syrups or elixirs. Compositions intended for oral use may be prepared according to any method known to the art for the manufacture of pharmaceutical compositions and such compositions may contain one or more agents, such as sweetening agents, flavoring agents, coloring agents and preserving agents, in order to provide pharmaceutically elegant and palatable preparations. In some embodiments, oral formulations contain from 0.1 to 99% of at least one chemical entity described herein. In some embodiments, oral formulations contain at least 5% (weight %) of at least one chemical entity described herein. Some embodiments contain from 25% to 50% or from 5% to 75 % of at least one chemical entity described herein.

Orally administered compositions also include liquid solutions, emulsions, suspensions, powders, granules, elixirs, tinctures, syrups, and the like. The pharmaceutically acceptable carriers suitable for preparation of such compositions are well known in the art. Oral formulations may contain preservatives, flavoring agents, sweetening agents, such as sucrose or saccharin, taste-masking agents, and coloring agents.

Typical components of carriers for syrups, elixirs, emulsions and suspensions include ethanol, glycerol, propylene glycol, polyethylene glycol, liquid sucrose, sorbitol and water. Syrups and elixirs may be formulated with sweetening agents, for example glycerol, propylene glycol, sorbitol or sucrose. Such formulations may also contain a demulcent.

Chemical entities described herein can be incorporated into oral liquid preparations such as aqueous or oily suspensions, solutions, emulsions, syrups, or elixirs, for example. Moreover, formulations comprising these chemical entities can be presented as a dry product for constitution with water or other suitable vehicle before use. Such liquid preparations can contain conventional additives, such as suspending agents (e.g., sorbitol syrup, methyl cellulose, glucose/sugar, syrup, gelatin, hydroxyethyl cellulose, carboxymethyl cellulose, aluminum stearate gel, and hydrogenated edible fats), emulsifying agents (e.g., lecithin, sorbitan monsoleate, or acacia), non-aqueous vehicles, which can include edible oils (e.g., almond oil, fractionated coconut oil, silyl esters, propylene glycol and ethyl alcohol), and preservatives (e.g., methyl or propyl p-hydroxybenzoate and sorbic acid).

For a suspension, typical suspending agents include methylcellulose, sodium carboxymethyl cellulose, AVICEL RC-591, tragacanth and sodium alginate; typical wetting agents include lecithin and polysorbate 80; and typical preservatives include methyl paraben and sodium benzoate.

Aqueous suspensions contain the active material(s) in admixture with excipients suitable for the manufacture of aqueous suspensions. Such excipients are chosen form suspending agents, for example sodium carboxymethylcellulose, methylcellulose, hydropropylmethylcellulose, sodium alginate, polyvinylpyrrolidone, gum tragacanth and gum acacia; dispersing or wetting agents; naturally-occurring phosphatides, for example, lecithin, and condensation products of an alkylene oxide with fatty acids, for example polyoxyethylene stearate, and condensation products of ethylene oxide with long chain aliphatic alcohols, for example heptadecaethyleneoxycetanol, and condensation products of ethylene oxide with partial esters derived from fatty acids and a hexitol such as polyoxyethylene sorbitol substitute, or condensation products of ethylene oxide with partial esters derived from fatty acids and hexitol anhydrides, for example polyethylene sorbitan substitute. The aqueous suspensions may also contain one or more preservatives, for example ethyl, or n- propyl p-hydroxybenzoate.

Oily suspensions may be formulated by suspending the active ingredients in a vegetable oil, for example peanut oil, olive oil, sesame oil or coconut oil, or in a mineral oil such as liquid paraffin. The oily suspensions may contain a thickening agent, for example beeswax, hard paraffin or cetyl alcohol. Sweetening agents such as those set forth above, and flavoring agents may be added to provide palatable oral preparations. These compositions may be preserved by the addition of an anti-oxidant such as ascorbic acid.

Pharmaceutical compositions of the invention may also be in the form of oil-in-water emulsions. The oily phase may be a vegetable oil, for example olive oil or peanut oil, or a mineral oil, for example liquid paraffin or mixtures of these. Suitable emulsifying agents may be naturally-occurring gums, for example gum acacia or gum tragacanth, naturally-occurring phosphatides, for example soy bean, lecithin, and esters or partial esters derived from fatty acids and hexitol, anhydrides, for example sorbitan monoleate, and condensation products of the said partial esters with ethylene oxide, for example polyoxyethylene sorbitan monoleate.

Dispersible powders and granules suitable for preparation of an aqueous suspension by the addition of water provide the active ingredient in admixture with a dispersing or wetting agent, suspending agent and one or more preservatives. Suitable dispersing or wetting agents and suspending agents are exemplified by those already mentioned above.

Tablets typically comprise conventional pharmaceutically acceptable adjuvants as inert diluents, such as calcium carbonate, sodium carbonate, mannitol, lactose and cellulose; binders such as starch, gelatin and sucrose; disintegrants such as starch, alginic acid and croscarmelose; lubricants such as magnesium stearate, stearic acid and talc. Glidants such as silicon dioxide can be used to improve flow characteristics of the powder mixture. Coloring agents, such as the FD&C dyes, can be added for appearance. Sweeteners and flavoring agents, such as aspartame, saccharin, menthol, peppermint, and fruit flavors, can be useful adjuvants for chewable tablets. Capsules (including time release and sustained release formulations) typically comprise one or more solid diluents disclosed above. The selection of carrier components often depends on secondary considerations like taste, cost, and shelf stability.

Such compositions may also be coated by conventional methods, typically with pH or time-dependent coatings, such that the chemical entity is released in the gastrointestinal tract in the vicinity of the desired topical application, or at various times to extend the desired action. Such dosage forms typically include, but are not limited to, one or more of cellulose acetate phthalate, polyvinylacetate phthalate, hydroxypropyl methylcellulose phthalate, ethyl cellulose, Eudragit coatings, waxes and shellac.

Formulations for oral use may also be presented as hard gelatin capsules wherein the active ingredient is mixed with an inert solid diluent, for example, calcium carbonate, calcium phosphate or kaolin, or as soft gelatin capsules wherein the active ingredient is mixed with water or an oil medium, for example peanut oil, liquid paraffin or olive oil.

Pharmaceutical compositions may be in the form of a sterile injectable aqueous or oleaginous suspension. This suspension may be formulated according to the known art using those suitable dispersing or wetting agents and suspending agents that have been mentioned above. The sterile injectable preparation may also be sterile injectable solution or suspension in a non-toxic parentally acceptable vehicle, for example as a solution in 1,3-butanediol. Among the acceptable vehicles that may be employed are water, Ringer's solution, and isotonic sodium chloride solution. In addition, sterile, fixed oils are conventionally employed as a solvent or suspending medium. For this purpose any bland fixed oil may be employed including synthetic mono- or diglycerides. In addition, fatty acids such as oleic acid can be useful in the preparation of injectables.

Chemical entities described herein may be administered parenterally in a sterile medium. Parenteral administration includes subcutaneous injections, intravenous, intramuscular, intrathecal injection or infusion techniques. Chemical entities described herein, depending on the vehicle and concentration used, can either be suspended or dissolved in the vehicle. Advantageously, adjuvants such as local anesthetics, preservatives and buffering agents can be dissolved in the vehicle. In many compositions for parenteral administration the carrier comprises at least 90% by weight of the total composition. In some embodiments, the carrier for parenteral administration is chosen from propylene glycol, ethyl oleate, pyrrolidone, ethanol, and sesame oil.

Chemical entites described herein may also be administered in the form of suppositories for rectal administration of the drug. These compositions can be prepared by mixing the drug with a suitable non-irritating excipient that is solid at ordinary temperatures but liquid at rectal temperature and will therefore melt in the rectum to release the drug. Such materials include cocoa butter and polyethylene glycols.

Chemical entities described herein may be formulated for local or topical application, such as for topical application to the skin and mucous membranes, such as in the eye, in the form of gels, creams, and lotions and for application to the eye. Topical compositions may be in any form including, for example, solutions, creams, ointments, gels, lotions, milks, cleansers, moisturizers, sprays, skin patches, and the like.

Such solutions may be formulated as 0.01% -10% isotonic solutions, pH from 2 to 12, such as from 5 to 7, with appropriate salts. Chemical entities described herein may also be formulated for transdermal administration as a transdermal patch.

Topical compositions comprising at least one chemical entity described herein can be admixed with a variety of carrier materials well known in the art, such as, for example, water, alcohols, aloe vera gel, allantoin, glycerine, vitamin A and E oils, mineral oil, propylene glycol, PPG-2 myristyl propionate, and the like.

Other materials suitable for use in topical carriers include, for example, emollients, solvents, humectants, thickeners and powders. Examples of each of these types of materials, which can be used singly or as mixtures of one or more materials, are as follows:

Representative emollients include stearyl alcohol, glyceryl monoricinoleate, glyceryl monostearate, propane-1,2-diol, butane-1,3-diol, mink oil, cetyl alcohol, iso-propyl isostearate, stearic acid, iso-butyl palmitate, isocetyl stearate, oleyl alcohol, isopropyl laurate, hexyl laurate, decyl oleate, octadecan-2-ol, isocetyl alcohol, cetyl palmitate, dimethylpolysiloxane, di-n-butyl sebacate, iso-propyl myristate, iso-propyl palmitate, iso-propyl stearate, butyl stearate, polyethylene glycol, triethylene glycol, lanolin, sesame oil, coconut oil, arachis oil, castor oil, acetylated lanolin alcohols, petroleum, mineral oil, butyl myristate, isostearic acid, palmitic acid, isopropyl linoleate, lauryl lactate, myristyl lactate, decyl oleate, and myristyl myristate; propellants, such as propane, butane, iso-butane, dimethyl ether, carbon dioxide, and nitrous oxide; solvents, such as ethyl alcohol, methylene chloride, iso-propanol, castor oil, ethylene glycol monoethyl ether, diethylene glycol monobutyl ether, diethylene glycol monoethyl ether, dimethyl sulphoxide, dimethyl formamide, tetrahydrofuran; humectants, such as glycerin, sorbitol, sodium 2-pyrrolidone-5-carboxylate, soluble collagen, dibutyl phthalate, and gelatin; and powders, such as chalk, talc, fullers earth, kaolin, starch, gums, colloidal silicon dioxide, sodium polyacrylate, tetra alkyl ammonium smectites, trialkyl aryl ammonium smectites, chemically modified magnesium aluminium silicate, organically modified montmorillonite clay, hydrated aluminium silicate, fumed silica, carboxyvinyl polymer, sodium carboxymethyl cellulose, and ethylene glycol monostearate.

Chemical entities described herein may also be topically administered in the form of liposome delivery systems, such as small unilamellar vesicles, large unilamellar vesicles, and multilamellar vesicles. Liposomes can be formed from a variety of phospholipids, such as cholesterol, stearylamine or phosphatidylcholines.

Other compositions useful for attaining systemic delivery of the chemical entity include sublingual, buccal and nasal dosage forms. Such compositions typically comprise one or more of soluble filler substances such as sucrose, sorbitol and mannitol, and binders such as acacia, microcrystalline cellulose, carboxymethyl cellulose, and hydroxypropyl methylcellulose. Glidants, lubricants, sweeteners, colorants, antioxidants and flavoring agents disclosed above may also be included.

Compositions for inhalation typically can be provided in the form of a solution, suspension or emulsion that can be administered as a dry powder or in the form of an aerosol using a conventional propellant (e.g., dichlorodifluoromethane or trichlorofluoromethane).

The compositions of the present invention may also optionally comprise an activity enhancer. The activity enhancer can be chosen from a wide variety of molecules that function in different ways to enhance or be independent of therapeutic effects of the chemical entities described herein. Particular classes of activity enhancers include skin penetration enhancers and absorption enhancers.

Pharmaceutical compositions of the invention may also contain additional active agents that can be chosen from a wide variety of molecules, which can function in different ways to enhance the therapeutic effects of at least one chemical entity described herein. These optional other active agents, when present, are typically employed in the compositions of the invention at a level ranging from 0.01% to 15%. Some embodiments contain from 0.1% to 10% by weight of the composition. Other embodiments contain from 0.5% to 5% by weight of the composition.

The invention can include packaged pharmaceutical formulations. Such packaged formulations include a pharmaceutical composition comprising at least one chemical entity chosen from compounds of Formula 1 and pharmaceutically acceptable salts, solvates, crystal forms, and mixtures thereof, in a container and instructions for using the composition to treat a mammal (typically a human patient). In some embodiments, the instructions are for using the pharmaceutical composition to treat a patient suffering from a disease responsive to kinase inhibition. The invention includes providing prescribing information; for example, to a patient or health care provider, or as a label in a packaged pharmaceutical formulation. Prescribing information may include for example efficacy, dosage and administration, contraindication and adverse reaction information pertaining to the pharmaceutical formulation.

In all of the foregoing the chemical entities can be administered alone, as mixtures, or in combination with other active agents.

The compounds of the present invention can be useful for the treatment of diseases and disorders responsive to kinase modulation.

In certain embodiments, compounds described herein are modulators of protein kinases. In certain embodiments, the compounds described herein are inhibitors of the protein kinases. In certain embodiments, the compounds inhibit at least one kinase chosen from EphB₄, c-Kit, PDGFRβ, and VEGFR2 kinases. In certain embodiments, the compounds inhibit more than one kinase chosen from EphB₄, c-Kit, PDGFRβ, and VEGFR2

Accordingly, the invention includes chemical entities for use in a method of treating a patient, such as a human patient, having a disease or disorder responsive to kinase modulation, said method comprising administering to the patient a therapeutically effective amount of at least one chemical entity described herein.

Compound for use in a method of treating a patient having a disease or disorder responsive to kinase modulation, particularly VEGFR2 modulation, said method comprising administering to the patient a therapeutically effective amount of one or more of the compounds of Formula I is provided.

Also provided is the use of at least one chemical entity described herein for the manufacture of a medicament for the treatment of a patient having a disease or disorder responsive to kinase modulation, particularly VEGFR2 modulation. Also provided is the use of at least one chemical entity described herein for the manufacture of a medicament for the treatment of a patient having angiogenesis.

In some embodiments, the compounds of Formula I inhibit at least one kinase chosen from EphB₄, c-Kit, PDGFRβ, and VEGFR2 and can be useful for the treatment of diseases and disorders responsive to modulation of at least one of such kinases. In some embodiments, the disease or disorder is characterized by angiogenesis supporting solid tumor growth or dysregulated local vascularization.

Methods of treatment also include modulating kinase activity, by inhibiting ATP binding or hydrolysis by a kinase or by some other mechanism, in vivo, in a patient suffering from a disease or disorder responsive to kinase modulation, by administering a therapeutically effective amount of at least one chemical entity described herein to inhibit kinase activity *in vitro.*

In some embodiments, the condition responsive to kinase modulation is cancer or a disease or disorder characterized by a change in angiogenesis.

The invention includes chemical entities for use in a method of treating a patient having cancer or a disease or disorder characterized by a change in angiogenesis by administering at least one chemical entity described herein. The invention provides compound for use in methods of treatment in which a compound of the invention is the only active agent given to a patient and also includes chemical entities for use in methods of treatment in which at least one chemical entity described herein is given to a patient in combination with one or more additional active agents.

Certain compounds described herein can be useful for treating a patient suffering from a disease or disorder responsive to kinase modulation.

In certain embodiments, the conditions, diseases and/or disorders that are affected using compounds of Formula I and compositions comprising such compounds include, but are not limited to, psoriasis, angiogenesis, cancer (for example, chronic myelogenous leukemia, gastrointestinal stromal tumors, non-small cell lung cancer, breast cancer, ovarian cancer, recurrent ovarian cancer, prostate cancer such as hormonal refractory prostate cancer, kidney cancer, head and neck cancer, or colorectal cancer), immunoregulation (graft rejection), atherosclerosis, rheumatoid arthritis, Parkinson's disease, Alzheimer's disease, diabetes (for example insulin resistance or diabetic retinopathy), septic shock, and the like.

In certain embodiments, the conditions, diseases and/or disorders that are affected using at least one chemical entity described herein and compositions comprising such chemical entities are female reproductive female reproductive disorders and conditions. In certain embodiments, the female reproductive disorders and conditions are chosen from endometriosis, endometrial carcinoma, gynecologic bleeding disorders, irregular menstrual cycles, ovulation, premenstrual syndrome (PMS), and menopausal dysfunction.

Because kinases play an active role in angiogenesis certain compounds described herein can be useful for modulating angiogenesis. Angiogenesis, the formation of new blood vessels from preexisting ones, plays a critical role in many pathological settings, including cancer, chronic inflammation, diabetic retinopathy and macular degeneration. Angiogenesis is regulated by multiple cell-signaling pathways, including pathways controlled by cellular kinases. Blocking angiogenesis, through the modulation of cell kinases, therefore, can represent effective approach to the treatment of diseases such as cancer. Thus methods of treatment include administering a therapeutically effective amount of at least one chemical entity described herein to treat these diseases or disorders, e.g., to decrease the symptoms or slow the progression of these diseases or disorders by inhibiting the rate of angiogenesis in a tissue.

The invention further includes compounds for use in methods for combination drug therapy, in which a compound of the invention is given to a patient together with one or more other active agents. Thus in one embodiment the invention provides chemical entities for use in a method of treating cancer, which comprises administering to a patient in need thereof an effective amount of at least one chemical entity described herein together with a second active agent, which can be useful for treating cancer. For example the second agent may be an antitumor agent. Treatment with the second active agent may be prior to, concomitant with, or following treatment with at least one chemical entity described herein.

In certain embodiments, at least one chemical entity chosen from compounds of Formula 1, and pharmaceutically acceptable salts, solvates, crystal forms, and mixtures thereof, is combined with at least one second active agent in a single dosage form. Radiotherapeutic anti-tumor agents may also be used alone or in combination with chemotherapeutic agents. Suitable anti-tumor therapeutics that may be used in combination with at least one chemical entity described herein. Examples of anti-tumor therapeutics include, in general, microtubule-stabilizing agents (such as paclitaxel (also known as Taxol), docetaxel (also known as Taxotere), epothilone A, epothilone B, desoxyepothilone A, desoxyepothilone B or their derivatives); microtubule-disruptor agents; alkylating agents, anti-metabolites; epidophyllotoxin; an antineoplastic enzyme; a topoisomerase inhibitor; procarbazine; mitoxantrone; platinum coordination complexes; biological response modifiers and growth inhibitors; hormonal/anti-hormonal therapeutic agents and haematopoietic growth factors.

Example classes of anti-tumor therapeutics include, for example, the anthracycline family of drugs, the vinca drugs, the mitomycins, the bleomycins, the cytotoxic nucleosides, the taxanes, the epothilones, discodermolide, the pteridine family of drugs, diynenes and the podophyllotoxins. Particularly useful members of those classes include, for example, doxorubicin, carminomycin, daunorubicin, aminopterin, methotrexate, methopterin, dichloro-methotrexate, mitomycin C, porfiromycin, herceptin, 5-fluorouracil, 6-mercaptopurine, gemcitabine, cytosine arabinoside, podophyllotoxin or podo-phyllotoxin derivatives such as etoposide, etoposide phosphate or teniposide, melphalan, vinblastine, vincristine, leurosidine, vindesine, leurosine, paclitaxel and the like. Other useful antineoplastic agents include estramustine, cisplatin, carboplatin, cyclophosphamide, bleomycin, tamoxifen, ifosamide, melphalan, hexamethyl melamine, thiotepa, cytarabin, idatrexate, trimetrexate, dacarbazine, L-asparaginase, camptothecin, CPT-11, topotecan, ara-C, bicalutamide, flutamide, leuprolide, pyridobenzoindole derivatives, interferons and interleukins.

In certain embodiments, at least one chemical entity chosen from compounds of Formula 1, and pharmaceutically acceptable salts, solvates, crystal forms, and mixtures thereof, can be administered in combination with an anti-inflammatory agent. Anti-inflammatory agents include NSAIDs, non-specific and COX-2 specific cyclooxgenase enzyme inhibitors, gold-containing compounds, corticosteroids, methotrexate, tumor necrosis factor receptor (TNF) receptors antagonists, immunosuppressants and methotrexate. Examples of NSAIDs include ibuprofen, flurbiprofen, naproxen and naproxen sodium, diclofenac, combinations of diclofenac sodium and misoprostol, sulindac, oxaprozin, diflunisal, piroxicam, indomethacin, etodolac, fenoprofen calcium, ketoprofen, sodium nabumetone, sulfasalazine, tolmetin sodium, and hydroxychloroquine. Examples of NSAIDs also include COX-2 specific inhibitors (i.e., a compound that inhibits COX-2 with an IC₅₀ that is at least 50-fold lower than the IC₅₀ for COX-1) such as celecoxib, valdecoxib, lumiracoxib, etoricoxib and/or rofecoxib. In certain embodiments, the anti-inflammatory agent can be a salicylate. Salicylates include acetylsalicylic acid or aspirin, sodium salicylate, and choline and magnesium salicylates. The anti-inflammatory agent can also be a corticosteroid. For example, the corticosteroid may be cortisone, dexamethasone, methylprednisolone, prednisolone, prednisolone sodium phosphate, and prednisone. In certain embodiments, the anti-inflammatory agent can be a gold-containing compound such as gold, sodium thiomalate or auranofin. In certain embodiments, the anti-inflammatory agent can be a metabolic inhibitor such as a dihydrofolate reductase inhibitor, such as methotrexate or a dihydroorotate dehydrogenase inhibitor, such as leflunomide. Certain embodiments of the present disclosure include combinations in which at least one anti-inflammatory compound can be an anti-C5 monoclonal antibody (such as eculizumab or pexelizumab), a TNF antagonist, such as entanercept, or infliximab, which is an anti-TNF alpha monoclonal antibody, and combinations in which at least one active agent is an immunosuppressant compound such as methotrexate, leflunomide, cyclosporine, tacrolimus, azathioprine, or mycophenolate mofetil.

Dosage levels of the order of from 0.1 mg to 140 mg per kilogram, such as 1 to 50 mg per kilogram, of body weight per day can be useful in the treatment of the above-indicated conditions (0.5 mg to 7 g per patient per day). The amount of active ingredient that may be combined with the carrier materials to produce a single dosage form will vary depending upon the host treated and the particular mode of administration. Dosage unit forms will generally contain from 1 mg to 500 mg of an active ingredient.

Frequency of dosage may also vary depending on the compound used and the particular disease treated. In certain embodiments, a dosage regimen of 4 times daily or less is used. In certain embodiments, a dosage regimen of 1 or 2 times daily is used.

It will be understood, however, that the specific dose level for any particular patient will depend upon a variety of factors including the activity of the specific compound employed, the age, body weight, general health, sex, diet, time of administration, route of administration, and rate of excretion, drug combination and the severity of the particular disease in the patient undergoing therapy.

### EXAMPLES

The invention is further illustrated by the following non-limiting examples.

In the examples below, the following abbreviations have the following meanings. If an abbreviation is not defined, it has its generally accepted meaning.
- DME: = dimethyl ether
- DMEM: = Dulbecco's modified Eagle's medium
- DMF: = N,N-dimethylformamide
- DMSO: = dimethylsulfoxide
- g: = gram
- h: = hour
- mg: = milligram
- min: = minutes
- mL: = milliliter
- mmol: = millimoles
- mM: = millimolar
- ng: = nanogram
- nm: = nanometer
- nM: = nanomolar
- PBS: = phosphate buffered saline
- µL: = microliter
- µM: = micromolar

### EXAMPLE 1.

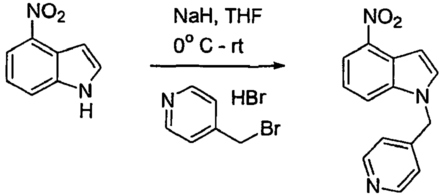

**4-Nitro-1-pyridin-4-ylmethyl-1H-indole:** To a cooled solution of 1.0 g (6.2 mmol, leq.) of 4-nitroindole in 25 mL of THF is added 0.44 g (19 mmol, 3 eq.) of NaH (95% dry). The resulting reaction mixture is stirred at 0° C for 30 min and treated with 2.3 g (9.3 mmol, 1.5 eq.) of 4-bromomethylpyridine hydrobromide and the reaction mixture is stirred for 16 hrs while warming up to rt. Water is added and the aqueous layer was extracted with EtOAc (3 x 75 mL), the organic layers combined and dried over anhydrous MgSO₄. The resulting solid is then purified by column chromatography (5% MeOH : 95% EtOAc) affording 4-nitro-1-pyridin-4-ylmethyl-1H-indole as a yellow solid.

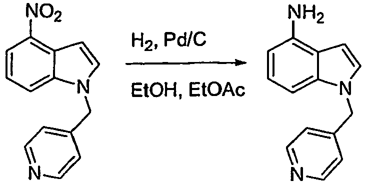

**1-Pyridine-4-ylmethyl-1H-indol-4-ylamine:** To a solution of 1.2 g (4.7 mmol, 1 eq.) of 4-nitro-1-pyridin-4-ylmethyl-1H-indole in EtOH/EtOAc (20 ml, 1:1) is added catalytic amount of Pd/C and the reaction mixture is placed in a Parr hydrogenation apparatus. After 3 hrs the H₂ gas uptake has ceased and the reaction mixture is filtered through a pad of Celite. The crude solution is then concentrated under reduced pressure and the resulting crude is purified by column chromatography (5% MeOH : 95% DCM) to afford 1-pyridine-4-ylmethyl-1H-indol-4-ylamine as an off-white solid.

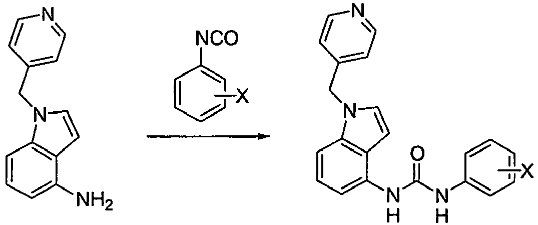

**Preparation of the ureas**: To a solution of 1-pyridine-4-ylmethyl-1H-indol-4-ylamine (1 eq.) in CH₂Cl₂ is added 1 eq. of the phenyl isocyanate and the reaction is monitored by LCMS. In the cases where the urea precipitated, the reaction mixture is diluted with Et₂O, the product is filtered and no further purification was needed. Otherwise, the resulting crude was purified by column chromatography using CH₂Cl₂/MeOH affording the desired urea as a white solid.

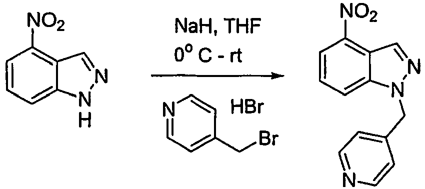

**4-Nitro-1-pyridin-4-ylmethyl-1H-indazole:** To a cooled solution of 4-nitroindazole (1.0 g, 6.10 mmol) in THF (25 mL) is added NaH (0.44 g, 95%, 18.0 mmol). The resulting reaction mixture is stirred at 0° C for 30 min then treated with 4-bromomethylpyridine hydrobromide (2.3 g, 9.2 mmol, 1.5 eq.) and the reaction mixture is stirred for 16 hrs while warming up to rt. Water (25 mL) is added and the aqueous layer is extracted with EtOAc (3 x 75 mL), and the organic layers are combined and dried over anhydrous MgSO₄. The resulting crude is then purified by column chromatography (5% MeOH : 95% EtOAc) to afford 4-nitro-1-pyridin-4-ylmethyl-1H-indazole as a yellow solid.

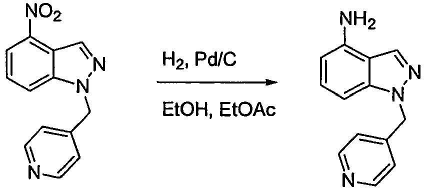

**1-Pyridine-4-ylmethyl-1H-indazol-4-ylamine:** To a solution of 4-nitro-1-pyridin-4-ylmethyl-1H-indazole (0.90 g, 3.60 mmol, 1.0 equiv) in EtOH/EtOAc (20 mL, 1:1) is added catalytic amount of Pd/C and the reaction mixture is placed in a Parr hydrogenation apparatus. After 4 days the H₂ gas uptake has ceased and the reaction mixture is filtered through a pad of Celite. The crude solution is then concentrated under reduced pressure and the resulting crude is purified by column chromatography (5% MeOH : 95% DCM) to afford 1-pyridine-4-ylmethyl-1H-indazol-4-ylamine as an orange oil. 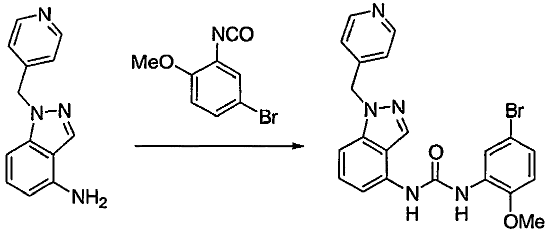 **1-(5-Bromo-2-methoxy-phenyl)-3-(1-pyridin-4-ylmethyl-1H-indazol-4-yl)-urea:** To a solution of 1-pyridine-4-ylmethyl-1H-indazol-4-ylamine (0.06 g, 0.3 mmol, 1 eq.) in DCM (2.0 mL) is added 5-bromo-2-methoxyphenyl isocyanate (0.06 g, 0.3 mmol, 1 eq.) and the reaction mixture is stirred for 16 hrs. The solvent is then removed under reduced pressure and the crude sample is purified by column chromatography (5% MeOH : 95% DCM) to afford 1-(5-bromo-2-methoxy-phenyl)-3-(1-pyridin-4-ylmethyl-1H-indazol-4-yl)-urea as an off-white solid.

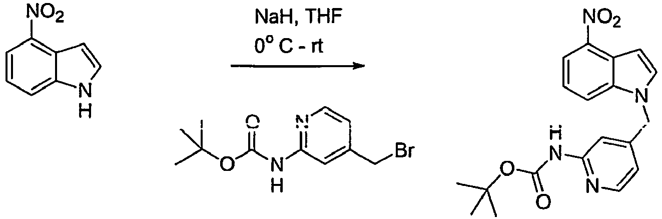

**[4-(4-Nitro-indol-1-ylmethyl)-pyridin-2-yl]-carbamic acid tert-butyl ester:** To a cooled solution of 4-nitroindole (1.0 g, 6.2 mmol, 1eq.) in THF (25 mL) is added NaH (0.44g, 95%, 19.0 mmol, 3.0 equivs). The resulting reaction mixture is stirred at 0° C for 30 min and treated with (4-Bromomethyl-pyridin-2-yl)-carbamic acid tert-butyl ester (2.30 g, 9.3 mmol, 1.5 eq.) and the reaction mixture is stirred for 16 hrs while warming up to RT. Water (50 mL) is added and the aqueous layer is extracted with EtOAc (3 x 75 mL), the organic layers are then combined and dried over anhydrous MgSO₄. The resulting crude sample is purified by column chromatography (5% MeOH : 95% EtOAc) to afford [4-(4-nitro-indol-1-ylmethyl)-pyridin-2-yl]-carbamic acid tert-butyl ester as a yellow solid.

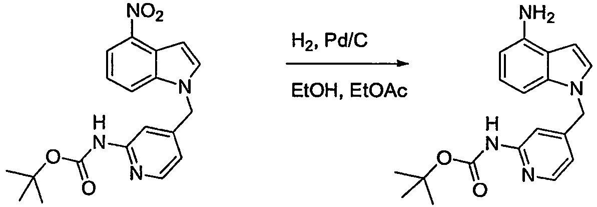

**[4-(4-Amino-indol-1-ylmethyl)-pyridin-2-yl]-carbamic acid tert-butyl ester:** To a solution of [4-(4-Nitro-indol-1-ylmethyl)-pyridin-2-yl]-carbamic acid tert-butyl ester (1.2 g, 4.7 mmol, 1 eq.) in EtOH/EtOAc (20 mL, 1:1) is added catalytic amount of Pd/C and the reaction mixture is placed in a Parr hydrogenation apparatus. After 3 hrs the H₂ gas uptake has ceased and the reaction mixture is filtered through a pad of Celite. The resulting crude sample is purified by column chromatography (5% MeOH : 95% DCM) to afford [4-(4-amino-indol-1-ylmethyl)-pyridin-2-yl]-carbamic acid tert-butyl ester as a cream-colored solid.

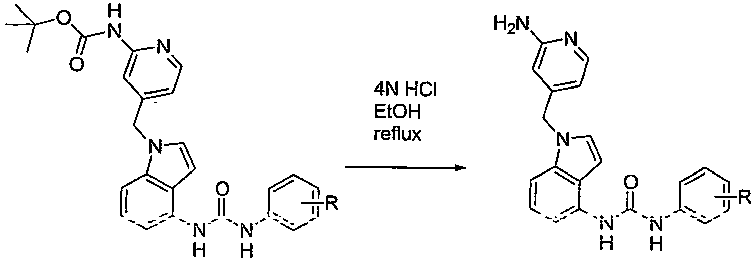

**Boc Deprotection:** A solution of Boc-protected urea (1 eq.) in 4N HCl (20 mL) and EtOH (20 mL) is refluxed and the reaction is monitored by LCMS. Upon full deprotection the solution is made basic with 2N NaOH and extracted with EtOAc (3 x 50 mL). The organic solution is dried over Na₂SO₄, filtered and concentrated under reduced pressure to a crude solid. Purification by column chromatography (MeOH:DCM) affords the desired urea as a white to off-white solid. 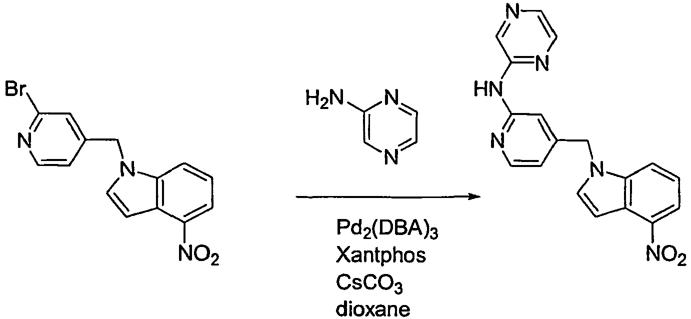 **[4-(4-Nitro-indol-1-ylmethyl)-pyridin-2-yl]-pyrazin-2-yl-amine:** To a solution of 1-(2-Bromo-pyridin-4-ylmethyl)-4-nitro-1H-indole (119 mg, 0.358 mmol) in dioxane (10 mL) is added CsCO₃ (175 mg, 0.538 mmol) and 2-aminopyrazine (51 mg, 0.538 mmol) at RT. The mixture is degassed by bubbling argon under sonication for 15 min. Pd₂(DBA)₃ (16
mg, 0.018 mmol) and Xantphos (31 mg, 0.054 mmol) are sequentially added and the solution is heated to 120°C for 6 h. Crude reaction products are adsorbed onto silica gel, and chromatographed (hexanes / ethyl acetate) to yield [4-(4-nitro-indol-1-ylmethyl)-pyridin-2-yl]-pyrazin-2-yl-amine as a yellow solid. 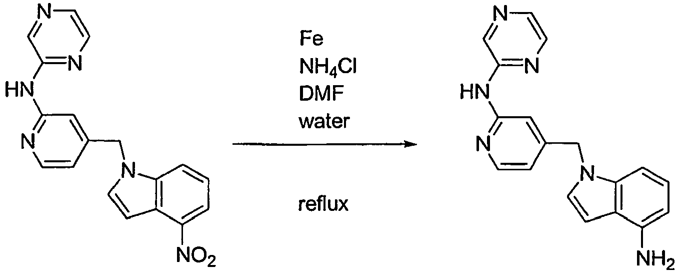 **1-[2-(Pyrazin-2-ylamino)-pyridin-4-ylmethyl]-1H-indol-4-ylamine:** [4-(4-Nitro-indol-1-ylmethyl)-pyridin-2-yl]-pyrazin-2-yl-amine is slurried with ethanol (5 mL), water (5 mL) and DMF (3 mL). Iron (90 mg), and ammonium chloride are added and the mixture is heated to reflux for 30 min. The crude mixture is filtered and the resulting filtrate is adsorbed onto silica gel and purified by column chromatography (DCM / MeOH) to afford 1-[2-(pyrazin-2-ylamino)-pyridin-4-ylmethyl]-1H-indol-4-ylamine as an off-white solid. 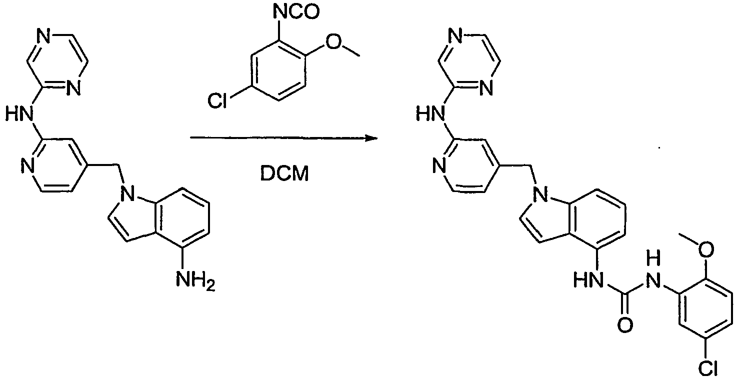 **1-(5-Chloro-2-methoxy-phenyl)-3-{1-[2-(pyrazin-2-ylamino)-pyridin-4-ylmethyl]-1H-indol-4-yl}-urea:** 1-[2-(Pyrazin-2-ylamino)-pyridin-4-ylmethyl]-1H-indol-4-ylamine (45 mg) is dissolved in DCM (5.0 mL) and 4-Chloro-2-isocyanato-1-methoxy-benzene (30 mg) is added and allowed stir for 1 h. Ether (20 mL) is added to the mixture and the precipated solid is collected by vacuum filtration affording 1-(5-chloro-2-methoxy-phenyl)-3-{1-[2-(pyrazin-2-ylamino)-pyridin-4-ylmethyl]-1H-indol-4-yl}-urea as a white solid.

### EXAMPLE 2.

The following compounds were prepared using procedures similar to those described above. Those of ordinary skill in the art of organic synthesis will recognize when starting materials or reaction conditions should be varied to obtain the desired compound.

MS data reported in this example was obtained as follows:
MS conditions: Electrospray MS is performed on a MICROMASS LCT equipped with a LockSpray source for accurate mass measurements. Spectra are acquired in positive ion mode from 100-1000 Da at an acquisition rate of 1 spectrum/0.9s with a 0.1s interscan delay.
The instrument is tuned for a resolution of 5000 (FWHM). Every 5^{th} scan is taken from the reference position of the Lockspray source. Leucine enkephalin (556.2771 [M+H]⁺) is used as the reference, or lock mass.

| **Structure** | **Name MF MW MS m/z** | **[M + H]** |
|---|---|---|
| 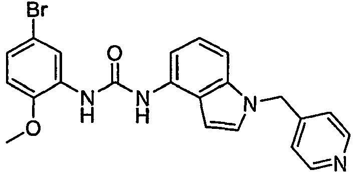 | 1-(5-Bromo-2-methoxy-phenyl)-3-(1-pyridin-4-ylmethyl-1H-indol-4-yl)-urea C22H19BrN402 450.07 | 450.91 |
| 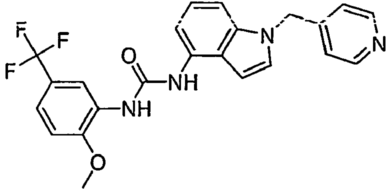 | 1-(2-Methoxy-5-trifluoromethyl-phenyl)-3-(1-pyridin-4-ylmethyl-1H-indol-4-yl)-urea C23H19F3N402 440.15 | 440.92 |
| 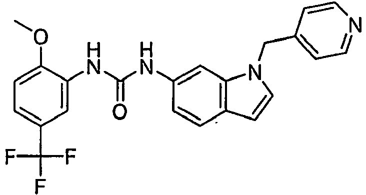 | 1-(2-Methoxy-5-trifluoromethyl-phenyl)-3-(1-pyridin-4-ylmethyl-1H-indol-6-yl)-urea C23H19F3N4O2 440.15 | 441.09 |
| 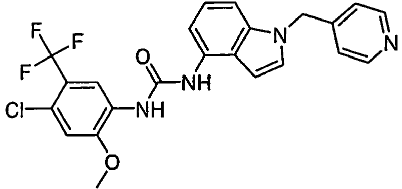 | 1-(4-Chloro-2-methoxy-5-trifluoromethyl-phenyl)-3-(1-pyridin-4-ylmethyl-1H-indol-4-yl)-urea C23H18ClF3N4O2 474.11 | 475.06 |
| 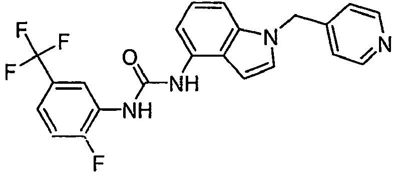 | 1-(2-Fluoro-5-trifluoromethyl-phenyl)-3-(1-pyridin-4-ylmethyl-1H-indol-4-yl)-urea C22H16F4N4O 428.13 | 429.07 |
| 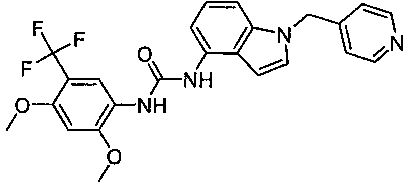 | 1-(2,4-Dimethoxy-5-trifluoromethyl-phenyl)-3-(1-pyridin-4-ylmethyl-1H-indol-4-yl)-urea C24H21F3N403 470.16 | 471.15 |
| 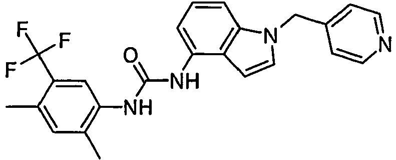 | 1-(2,4-Dimethyl-5-trifluoromethyl-phenyl)-3-(1-pyridin-4-ylmethyl-1H-indol-4-yl)-urea C24H21F3N4O 438.17 | 439.10 |
| 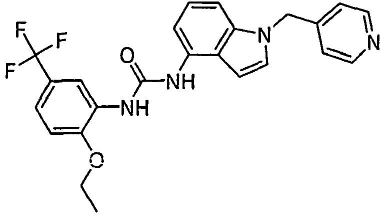 | 1-(2-Ethoxy-5-trifluoromethyl-phenyl)-3-(1-pyridin-4-ylmethyl-1H-indol-4-yl)-urea C24H21F3N402 454.16 | 455.09 |
| 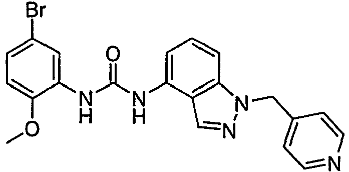 | 1-(5-Bromo-2-methoxy-phenyl)-3-(1-pyridin-4-ylmethyl-1H-indazol-4-yl)-urea C21H18BrN5O2 451.06 | 452.06 |
| 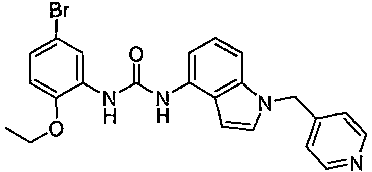 | 1-(5-Bromo-2-ethoxy-phenyl)-3-(1-pyridin-4-ylmethyl-1H-indol-4-yl)-urea C23H21BrN4O2 464.08 | 465.34 |
| 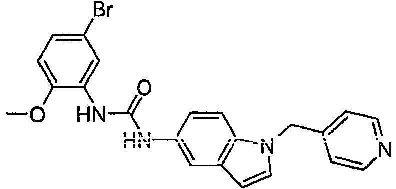 | 1-(5-Bromo-2-methoxy-phenyl)-3-(1-pyridin-4-ylmethyl-1H-indol-5-yl)-urea C22H19BrN402 450.07 | 451.14 |
| 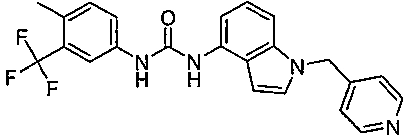 | 1-(4-Methyl-3-trifluoromethyl-phenyl)-3-(1-pyridin-4-ylmethyl-1H-indol-4-yl)-urea C23H19F3N4O 424.15 | 425.16 |
| 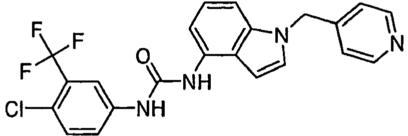 | 1-(4-Chloro-3-trifluoromethyl-phenyl)-3-(1-pyridin-4-ylmethyl-1H-indol-4-yl)-urea C22H16ClF3N4O 444.10 | 445.19 |
| 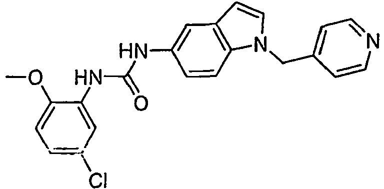 | 1-(5-Chloro-2-methoxy-phenyl)-3-(1-pyridin-4-ylmethyl-1H-indol-5-yl)-urea C22H19ClN4O2 406.12 | 407.05 |
| 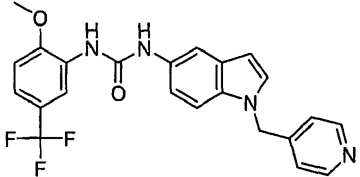 | 1-(2-Methoxy-5-trifluoromethyl-phenyl)-3-(1-pyridin-4-ylmethyl-1H-indol-5-yl)-urea C23H19F3N4O2 440.15 | 441.04 |
| 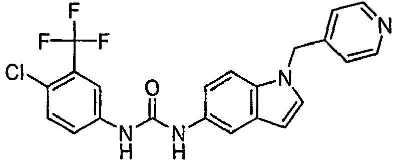 | 1-(4-Chloro-3-trifluoromethyl-phenyl)-3-(1-pyridin-4-ylmethyl-1H-indol-5-yl)-urea C22H16ClF3N4O 444.10 | 445.01 |
| 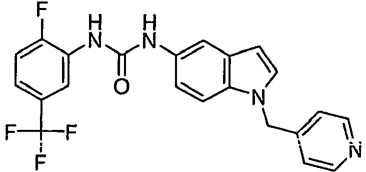 | 1-(2-Fluoro-5-trifluoromethyl-phenyl)-3-(1-pyridin-4-ylmethyl-1H-indol-5-yl)-urea C22H16F4N40 428.13 | 429.08 |
| 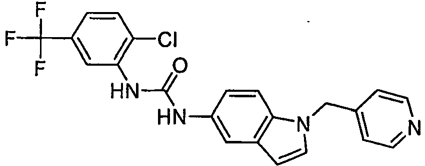 | 1-(2-Chloro-5-trifluoromethyl-phenyl)-3-(1-pyridin-4-ylmethyl-1H-indol-5-yl)-urea C22H16ClF3N4O 444.10 | 445.05 |
| 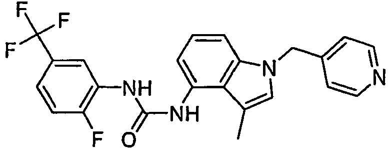 | 1-(2-Fluoro-5-trifluoromethyl-phenyl)-3-(3-methyl-1-pyridin-4-ylmethyl-1H-indol-4-yl)-urea C23H18F4N40 442.14 | 443.04 |
| 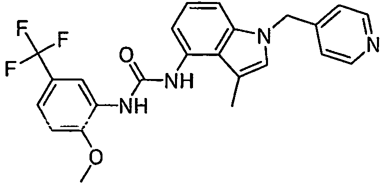 | 1-(2-Methoxy-5-trifluoromethyl-phenyl)-3-(3-methyl-1-pyridin-4-ylmethyl-1H-indol-4-yl)-urea C24H21F3N402 454.16 | 455.07 |
| 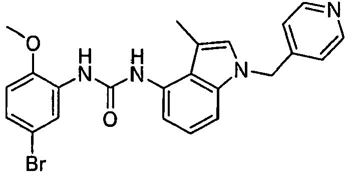 | 1-(5-Bromo-2-methoxy-phenyl)-3-(3-methyl-1-pyridin-4-ylmethyl-1H-indol-4-yl)-urea C23H21BrN402 464.08 | 465.03 |
| 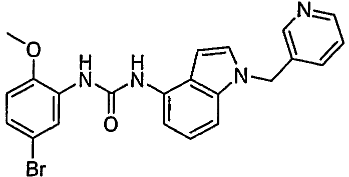 | 1-(5-Bromo-2-methoxy-phenyl)-3-(1-pyridin-3-ylmethyl-1H-indol-4-yl)-urea C22H19BrN4O2 450.07 | 451.05 |
| 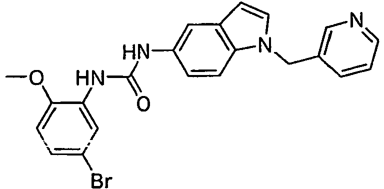 | 1-(5-Bromo-2-methoxy-phenyl)-3-(1-pyridin-3-ylmethyl-1H-indol-5-yl)-urea C22H19BrN4O2 450.07 | 451.05 |
| 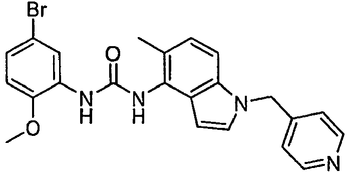 | 1-(5-Bromo-2-methoxy-phenyl)-3-(5-methyl-1-pyridin-4-ylmethyl-1H-indol-4-yl)-urea C23H21BrN4O2 464.08 | 465.04 |
| 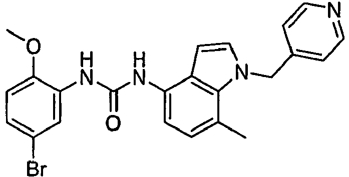 | 1-(5-Bromo-2-methoxy-phenyl)-3-(7-methyl-1-pyridin-4-ylmethyl-1H-indol-4-yl)-urea C23H21BrN402 464.08 | 465.03 |
| 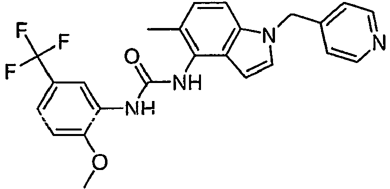 | 1-(2-Methoxy-5-trifluoromethyl-phenyl)-3-(5-methyl-1-pyridin-4-ylmethyl-1H-indol-4-yl)-urea C24H21F3N402 454.16 | 455.04 |
| 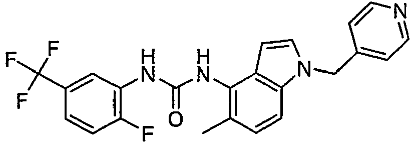 | 1-(2-Fluoro-5-trifluoromethyl-phenyl)-3-(5-methyl-1-pyridin-4-ylmethyl-1H-indol-4-yl)-urea C23H18F4N40 442.14 | 443.02 |
| 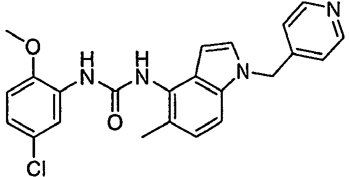 | 1-(5-Chloro-2-methoxy-phenyl)-3-(5-methyl-1-pyridin-4-ylmethyl-1H-indol-4-yl)-urea C23H21ClN4O2 420.14 | 421.05 |
| 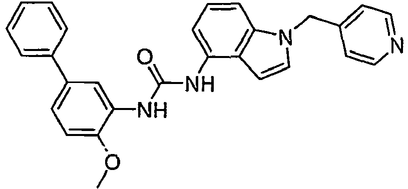 | 1-(4-Methoxy-biphenyl-3-yl)-3-(1-pyridin-4-ylmethyl-1H-indol-4-yl)-urea C28H24N402 448.19 | 449.34 |
| 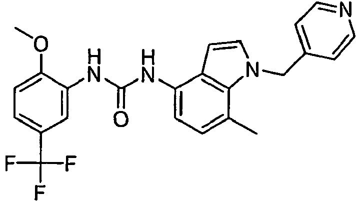 | 1-(2-Methoxy-5-trifluoromethyl-phenyl)-3-(7-methyl-1-pyridin-4-ylmethyl-1H-indol-4-yl)-urea C24H21F3N402 454.16 | 455.25 |
| 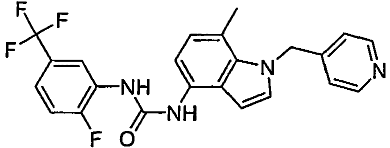 | 1-(2-Fluoro-5-trifluoromethyl-phenyl)-3-(7-methyl-1-pyridin-4-ylmethyl-1H-indol-4-yl)-urea C23H18F4N4O 442.14 | 443.19 |
| 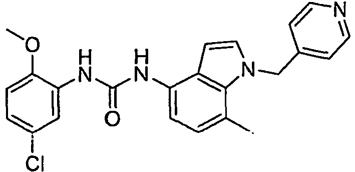 | 1-(5-Chloro-2-methoxy-phenyl)-3-(7-methyl-1-pyridin-4-ylmethyl-1H-indol-4-yl)-urea C23H21ClN4O2 420.14 | 421.26 |
| 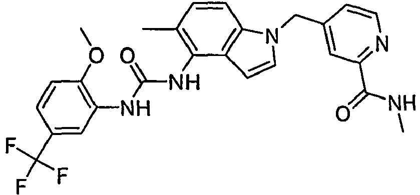 | 4- {4-[3-(2-Methoxy-5-trifluoromethyl-phenyl)-ureido]-5-methyl-indol-1-ylmethyl}-pyridine-2-carboxylic acid methylamide C26H24F3N503 511.18 | 512.36 |
| 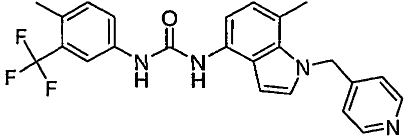 | 1-(7-Methyl-1-pyridin-4-ylmethyl-1H-indol-4-yl)-3-(4-methyl-3-trifluoromethyl-phenyl)-urea C24H21F3N4O 438.17 | 439.23 |
| 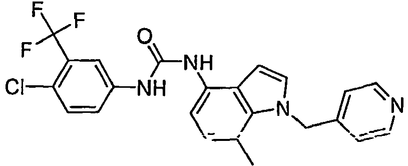 | 1-(4-Chloro-3-trifluoromethyl-phenyl)-3-(7-methyl-1-pyridin-4-ylmethyl-1 H-indol-4-yl)-urea C23H18ClF3N4O 458.11 | 459.19 |
| 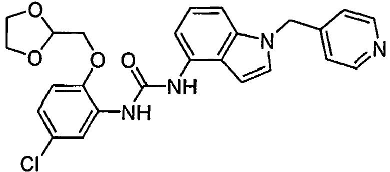 | 1-[5-Chloro-2-([1,3]dioxolan-2-ylmethoxy)-phenyl]-3-(1-pyridin-4-ylmethyl-1H-indol-4-yl)-urea C25H23ClN4O4 478.14 | 479.22 |
| 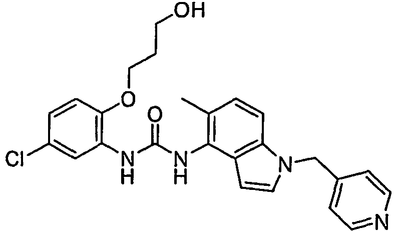 | 1-[5-Chloro-2-(3-hydroxy-propoxy)-phenyl]-3-(5-methyl-1-pyridin-4-ylmethyl-1H-indol-4-yl)-urea C25H25ClN4O3 464.16 | 465.24 |
| 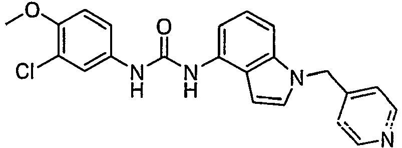 | 1-(3-Chloro-4-methoxy-phenyl)-3-(1-pyridin-4-ylmethyl-1H-indol-4-yl)-urea C22H19ClN4O2 406.12 | 407.20 |
| 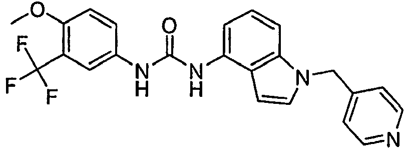 | 1-(4-Methoxy-3-trifluoromethyl-phenyl)-3-(1-pyridin-4-ylmethyl-1H-indol-4-yl)-urea C23H19F3N402 440.15 | 441.20 |
| 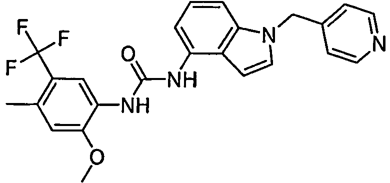 | 1-(2-Methoxy-4-methyl-5-trifluoromethyl-phenyl)-3-(1-pyridin-4-ylmethyl-1 H-indol-4-yl)-urea C24H21F3N402 454.16 | 455.22 |
| 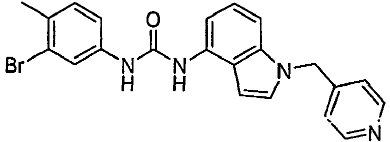 | 1-(3-Bromo-4-methyl-phenyl)-3-(1-pyridin-4-ylmethyl-1H-indol-4-yl)-urea C22H19BrN4O 434.07 | 435.20 |
| 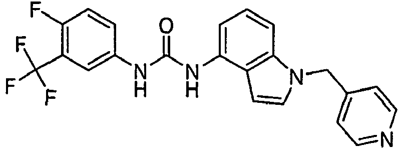 | 1-(4-Fluoro-3-trifluoromethyl-phenyl)-3-(1-pyridin-4-ylmethyl-1H-indol-4-yl)-urea C22H16F4N4O 428.13 | 429.18 |
| 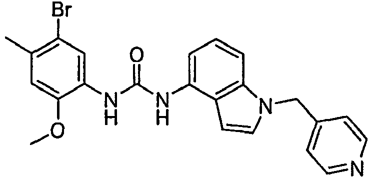 | 1-(5-Bromo-2-methoxy-4-methyl-phenyl)-3-(1-pyridin-4-ylmethyl-1H-indol-4-yl)-urea C23H21BrN4O2 464.08 | 465.17 |
| 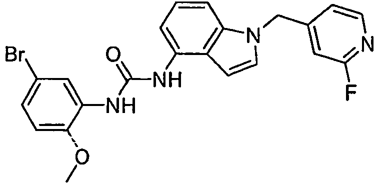 | 1-(5-Bromo-2-methoxy-phenyl)-3-[1-(2-fluoro-pyridin-4-ylmethyl)-1H-indol-4-yl]-urea C22H18BrFN4O2 468.06 | 469.29 |
| 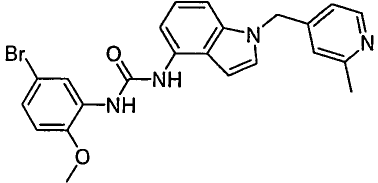 | 1-(5-Bromo-2-methoxy-phenyl)-3-[1-(2-methyl-pyridin-4-ylmethyl)-1H-indol-4-yl]-urea C23H21BrN4O2 464.08 | 465.13 |
| 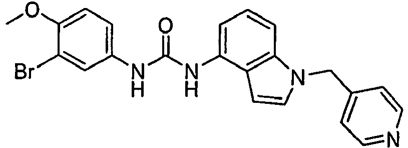 | 1-(3-Bromo-4-methoxy-phenyl)-3-(1-pyridin-4-ylmethyl-1H-indol-4-yl)-urea C22H19BrN4O2 450.07 | 451.13 |
| 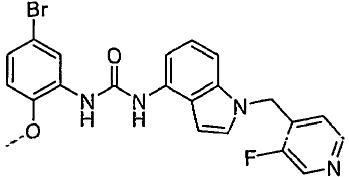 | 1-(5-Bromo-2-methoxy-phenyl)-3-[1-(3-fluoro-pyridin-4-ylmethyl)-1H-indol-4-yl]-urea C22H18BrFN4O2 468.06 | 469.23 |
| 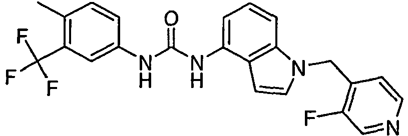 | 1-[1-(3-Fluoro-pyridin-4-ylmethyl)-1H-indol-4-yl]-3-(4-methyl-3-trifluoromethyl-phenyl)-urea C23H18F4N40 442.14 | 443.23 |
| 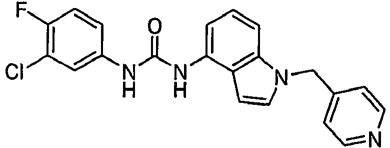 | 1-(3-Chloro-4-fluoro-phenyl)-3-(1-pyridin-4-ylmethyl-1H-indol-4-yl)-urea C21H16ClFN4O 394.10 | 395.14 |
| 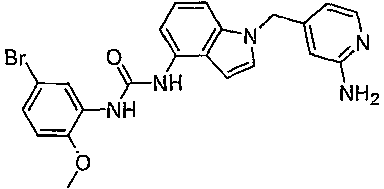 | 1-[1-(2-Amino-pyridin-4-ylmethyl)-1H-indol-4-yl]-3-(5-bromo-2-methoxy-phenyl)-urea C22H20BrN502 465.08 | 466.18 |
| 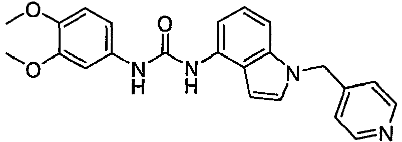 | 1-(3,4-Dimethoxy-phenyl)-3-(1-pyridin-4-ylmethyl-1H-indol-4-yl)-urea C23H22N403 402.17 | 403.13 |
| 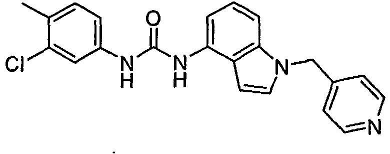 | 1-(3-Chloro-4-methyl-phenyl)-3-(1-pyridin-4-ylmethyl-1H-indol-4-yl)-urea C22H19ClN4O 390.12 | 391.08 |
| 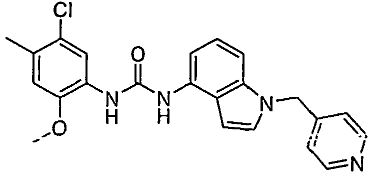 | 1-(5-Chloro-2-methoxy-4-methyl-phenyl)-3-(1-pyridin-4-ylmethyl-1H-indol-4-yl)-urea C23H21ClN4O2 420.14 | 421.06 |
| 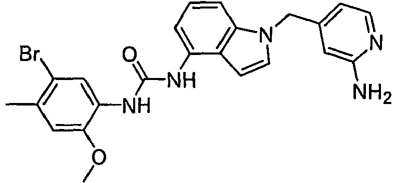 | 1-[1-(2-Amino-pyridin-4-ylmethyl)-1H-indol-4-yl]-3-(5-bromo-2-methoxy-4-methyl-phenyl)-urea C23H22BrN502 479.10 | 480.03 |
| 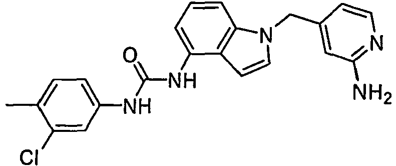 | 1-[1-(2-Amino-pyridin-4-ylmethyl)-1H-indol-4-yl]-3-(3-chloro-4-methyl-phenyl)-urea C22H20ClN5O 405.14 | 406.07 |
| 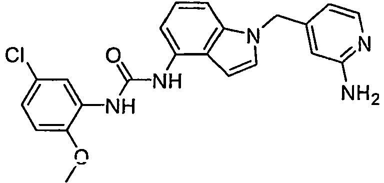 | 1-[1-(2-Amino-pyridin-4-ylmethyl)-1H-indol-4-yl]-3-(5-chloro-2-methoxy-phenyl)-urea C22H20ClN5O2 421.13 | 422.07 |
| 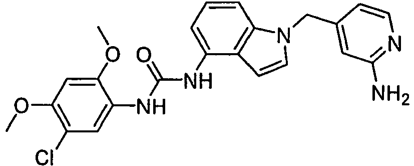 | 1-[1-(2-Amino-pyridin-4-ylmethyl)-1H-indol-4-yl]-3-(5-chloro-2,4-dimethoxy-phenyl)-urea C23H22ClN5O3 451.14 | 452.10 |
| 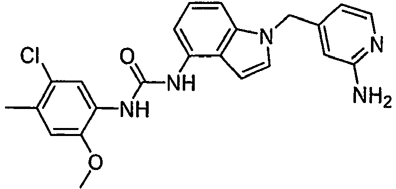 | 1-[1-(2-Amino-pyridin-4-ylmethyl)-1H-indol-4-yl]-3-(5-chloro-2-methoxy-4-methyl-phenyl)-urea C23H22ClN5O2 435.15 | 436.11 |
| 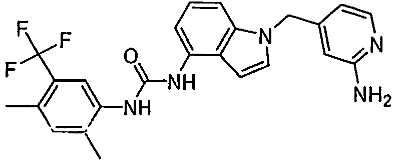 | 1-[1-(2-Amino-pyridin-4-ylmethyl)-1H-indol-4-yl]-3-(2,4-dimethyl-5-trifluoromethyl-phenyl)-urea C24H22F3N50 453.18 | 454.08 |
| 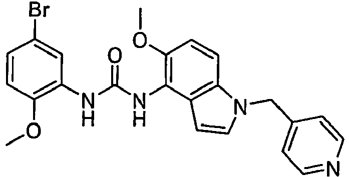 | 1-(5-Bromo-2-methoxy-phenyl)-3-(5-methoxy-1-pyridin-4-ylmethyl-1H-indol-4-yl)-urea C23H21BrN403 480.08 | 481.05 |
| 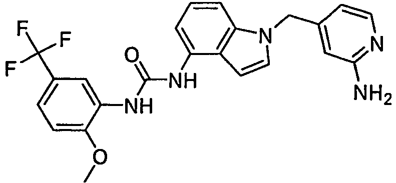 | 1-[1-(2-Amino-pyridin-4-ylmethyl)-1H-indol-4-yl]-3-(2-methoxy-5-trifluoromethyl-phenyl)-urea C23H20F3N502 455.16 | 456.07 |
| 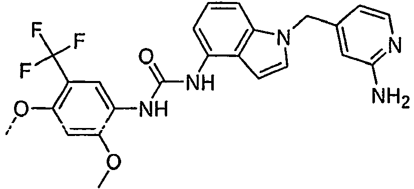 | 1-[1-(2-Amino-pyridin-4-ylmethyl)-1H-indol-4-yl]-3-(2,4-dimethoxy-5-trifluoromethyl-phenyl)-urea C24H22F3N503 485.17 | 486.08 |
| 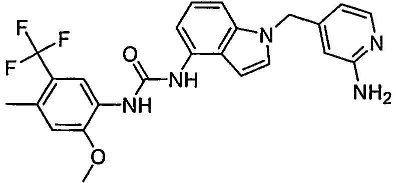 | 1-[1-(2-Amino-pyridin-4-ylmethyl)-1H-indol-4-yl]-3-(2-methoxy-4-methyl-5-trifluoromethyl-phenyl)-urea C24H22F3N502 469.17 | 470.10 |
| 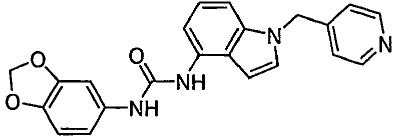 | 1-Benzo[1,3]dioxol-5-yl-3-(1-pyridin-4-ylmethyl-1H-indol-4-yl)-urea C22H18N403 386.14 | 387.11 |
| 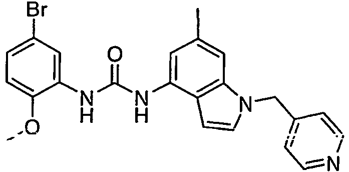 | 1-(5-Bromo-2-methoxy-phenyl)-3-(6-methyl-1-pyridin-4-ylmethyl-1H-indol-4-yl)-urea C23H21BrN4O2 464.08 | 465.08 |
| 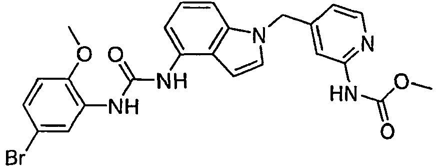 | (4- {4-[3-(5-Bromo-2-methoxy-phenyl)-ureido]-indol-1-ylmethyl}-pyridin-2-yl)-carbamic acid methyl ester C24H22BrN504 523.09 | 524.13 |
| 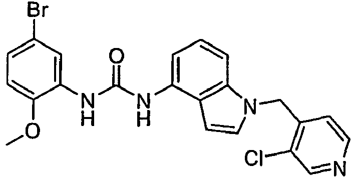 | 1-(5-Bromo-2-methoxy-phenyl)-3-[1-(3-chloro-pyridin-4-ylmethyl)-1H-indol-4-yl]-urea C22H18BrClN4O2 484.03 | 485.10 |
| 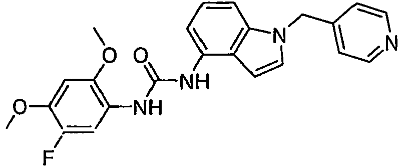 | 1-(5-Fluoro-2,4-dimethoxy-phenyl)-3-(1-pyridin-4-ylmethyl-1H-indol-4-yl)-urea C23H21FN403 420.16 | 421.10 |
| 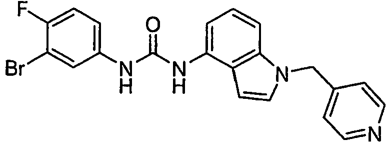 | 1-(3-Bromo-4-fluoro-phenyl)-3-(1-pyridin-4-ylmethyl-1H-indol-4-yl)-urea C21H16BrFN4O 438.05 | 439.04 |
| 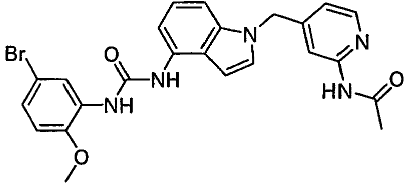 | N-(4{4-[3-(5-Bromo-2-methoxy-phenyl)-ureido]-indol-1-ylmethyl}-pyridin-2-yl)-acetamide C24H22BrN503 507.09 | 508.06 |
| 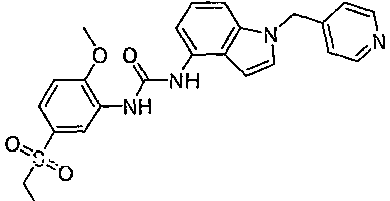 | 1-(5-Ethanesulfonyl-2-methoxy-phenyl)-3-(1-pyridin-4-ylmethyl-1H-indol-4-yl)-urea C24H24N404S 464.15 | 465.03 |
| 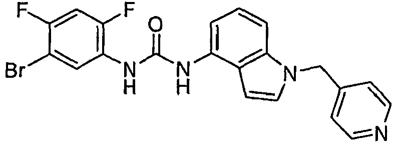 | 1-(5-Bromo-2,4-difluoro-phenyl)-3-(1-pyridin-4-ylmethyl-1H-indol-4-yl)-urea C21H15BrF2N4O 456.04 | 456.95 |
| 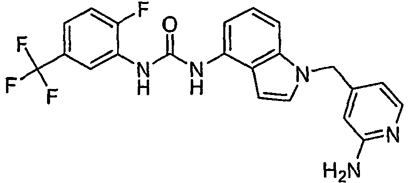 | 1-[1-(2-Amino-pyridin-4-ylmethyl)-1H-indol-4-yl]-3-(2-fluoro-5-trifluoromethyl-phenyl)-urea C22H17F4N5O 443.14 | 444.02 |
| 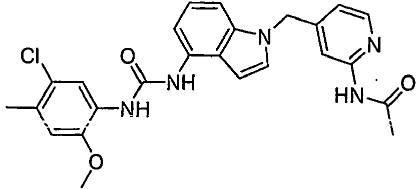 | N-(4-{4-[3-(5-Chloro-2-methoxy-4-methyl-phenyl)-ureido]-indol-1-ylmethyl}-pyridin-2-yl)-acetamide C25H24ClN5O3 477.16 | 478.07 |
| 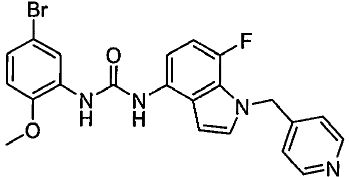 | 1-(5-Bromo-2-methoxy-phenyl)-3-(7-fluoro-1-pyridin-4-ylmethyl-1H-indol-4-yl)-urea C22H18BrFN4O2 468.06 | 468.98 |
| 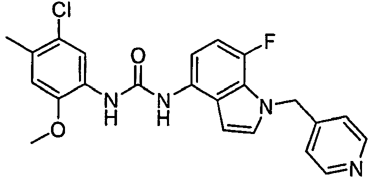 | 1-(5-Chloro-2-methoxy-4-methyl-phenyl)-3-(7-fluoro-1-pyridin-4-ylmethyl-1H-indol-4-yl)-urea C23H20ClFN4O2 438.13 | 439.03 |
| 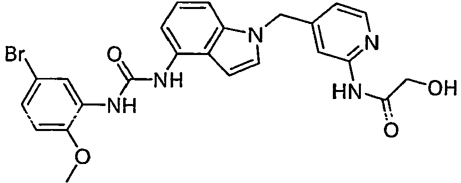 | N-(4-{4-[3-(5-Bromo-2-methoxy-phenyl)-ureido]-indol-1-ylmethyl}-pyridin-2-yl)-2-hydroxy-acetamide C24H22BrN504 523.09 | 524.11 |
| 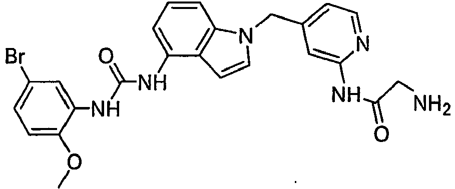 | 2-Amino-N-(4-{4-[3-(5-bromo-2-methoxy-phenyl)-ureido]-indol-1-ylmethyl}-pyridin-2-yl)-acetamide C24H23BrN603 522.10 | 523.36 |
| 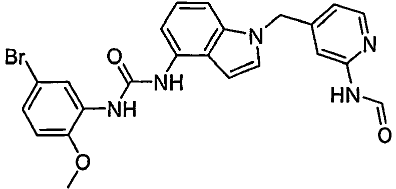 | N-(4-{4-[3-(5-Bromo-2-methoxy-phenyl)-ureido]-indol-1-ylmethyl}-pyridin-2-yl)-formamide C23H20BrN503 493.07 | 494.38 |
| 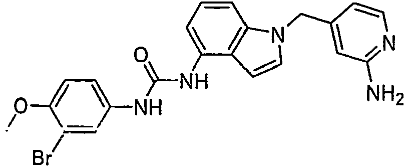 | 1-[1-(2-Amino-pyridin-4-ylmethyl)-1H-indol-4-yl]-3-(3-bromo-4-methoxy-phenyl)-urea C22H20BrN502 465.08 | 466.33 |
| 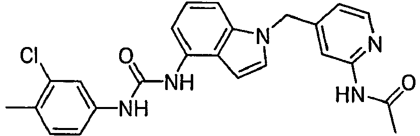 | N-(4-{4-[3-(3-Chloro-4-methyl-phenyl)-ureido]-indol-1-ylmethyl}-pyridin-2-yl)-acetamide C24H22ClN5O2 447.15 | 448.36 |
| 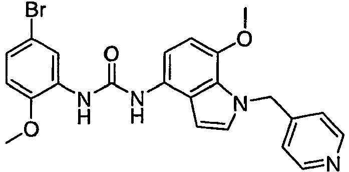 | 1-(5-Bromo-2-methoxy-phenyl)-3-(7-methoxy-1-pyridin-4-ylmethyl-1H-indol-4-yl)-urea C23H21BrN4O3 480.08 | 481.29 |
| 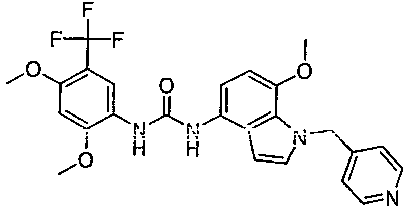 | 1-(2,4-Dimethoxy-5-trifluoromethyl-phenyl)-3-(7-methoxy-1-pyridin-4-ylmethyl-1H-indol-4-yl)-urea C25H23F3N404 500.17 | 501.28 |
| 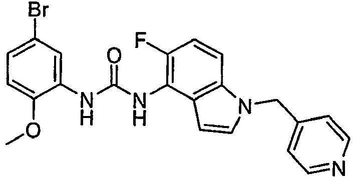 | 1-(5-Bromo-2-methoxy-phenyl)-3-(5-fluoro-1-pyridin-4-ylmethyl-1H-indol-4-yl)-urea C22H18BrFN4O2 468.06 | 469.27 |
| 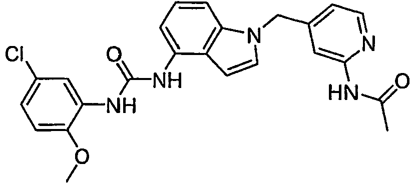 | N-(4-{4-[3-(5-Chloro-2-methoxy-phenyl)-ureido]-indol-1-ylmethyl}-pyridin-2-yl)-acetamide C24H22ClN5O3 463.14 | 464.12 |
| 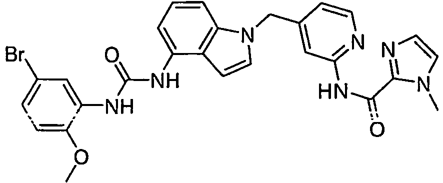 | 1-Methyl-1H-imidazole-2-carboxylic acid (4-{4-[3-(5-bromo-2-methoxy-phenyl)-ureido]-indol-1-ylmethyl}-pyridin-2-yl)-amide C27H24BrN703 573.11 | 574.11 |
| 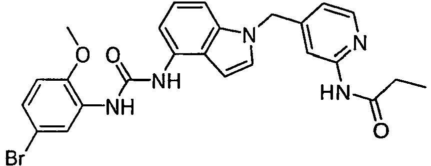 | N-(4-{4-[3-(5-Bromo-2-methoxy-phenyl)-ureido]-indol-1-ylmethyl}-pyridin-2-yl)-propionamide C25H24BrN503 521.11 | 522.16 |
| 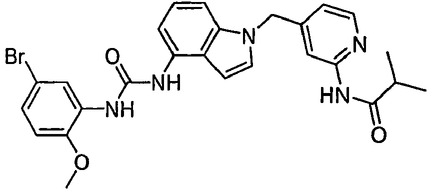 | N-(4-{4-[3-(5-Bromo-2-methoxy-phenyl)-ureido]-indol-1-ylmethyl}-pyridin-2-yl)-isobutyramide C26H26BrN503 535.12 | 536.17 |
| 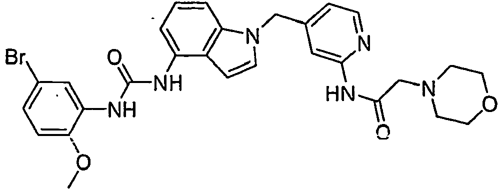 | N-(4-{4-[3-(5-Bromo-2-methoxy-phenyl)-ureido]-indol-1-ylmethyl}-pyridin-2-yl)-2-morpholin-4-yl-acetamide C28H29BrN604 592.14 | 593.10 |
| 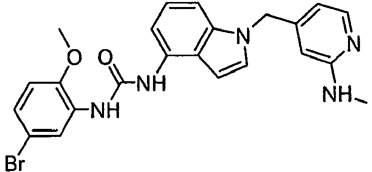 | 1-(5-Bromo-2-methoxy-phenyl)-3-[1-(2-methylamino-pyridin-4-ylmethyl)-1H-indol-4-yl]-urea C23H22BrN502 479.10 | 480.09 |
| 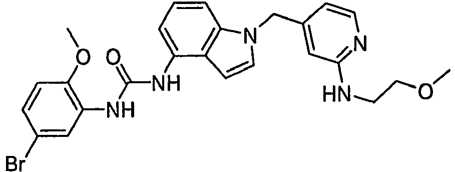 | 1-(5-Bromo-2-methoxy-phenyl)-3-{1-[2-(2-methoxy-ethylamino)-pyridin-4-ylmethyl]-1H-indol-4-yl}-urea C25H26BrN503 523.12 | 524.11 |
| 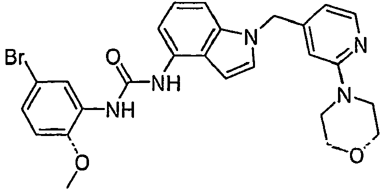 | 1-(5-Bromo-2-methoxy-phenyl)-3-[1-(2-morpholin-4-yl-pyridin-4-ylmethyl)-1H-indol-4-yl]-urea C26H26BrN503 535.12 | 536.11 |
| 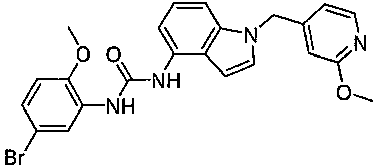 | 1-(5-Bromo-2-methoxy-phenyl)-3-[1-(2-methoxy-pyridin-4-ylmethyl)-1H-indol-4-yl]-urea C23H21BrN403 480.08 | 481.10 |
| 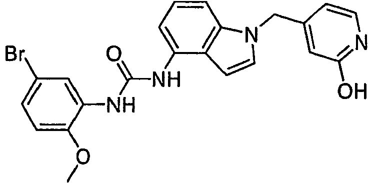 | 1-(5-Bromo-2-methoxy-phenyl)-3-[1-(2-hydroxy-pyridin-4-ylmethyl)-1H-indol-4-yl]-urea C22H19BrN403 466.06 | 467.14 |
| 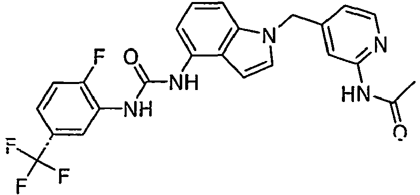 | N-(4-{4-[3-(2-Fluoro-5-trifluoromethyl-phenyl)-ureido]-indol-1-ylmethyl}-pyridin-2-yl)-acetamide C24H19F4N5O2 485.15 | 486.18 |
| 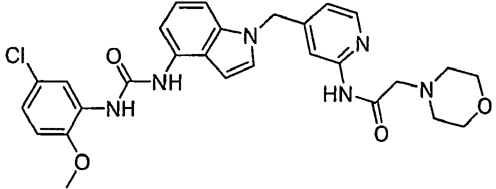 | N-(4-{4-[3-(5-Chloro-2-methoxy-phenyl)-ureido]-indol-1-ylmethyl}-pyridin-2-yl)-2-morpholin-4-yl-acetamide C28H29ClN6O4 548.19 | 549.25 |
| 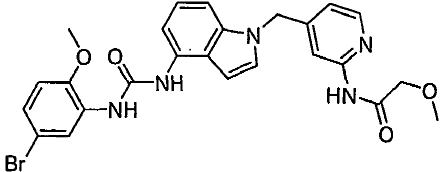 | N-(4-{4-[3-(5-Bromo-2-methoxy-phenyl)-ureido]-indol-1-ylmethyl}-pyridin-2-yl)-2-methoxy-acetamide C25H24BrN504 537.10 | 538.17 |
| 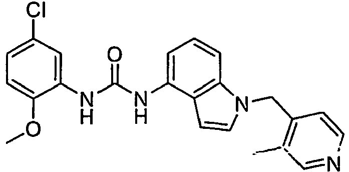 | 1-(5-Chloro-2-methoxy-phenyl)-3-[1-(3-methyl-pyridin-4-ylmethyl)-1H-indol-4-yl]-urea C23H21ClN4O2 420.14 | 421.18 |
| 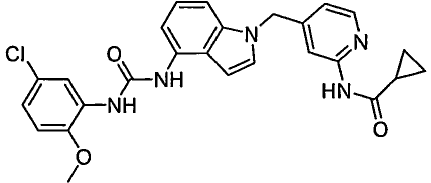 | Cyclopropanecarboxylic acid (4- {4-[3-(5-chloro-2-methoxy-phenyl)-ureido]-indol-1-ylmethyl}-pyridin-2-yl)-amide C26H24ClN5O3 489.16 | 490.23 |
| 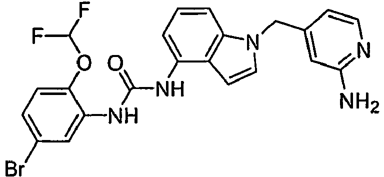 | 1-[1-(2-Amino-pyridin-4-ylmethyl)-1H-indol-4-yl]-3-(5-bromo-2-difluoromethoxy-phenyl)-urea C22H18BrF2N502 501.06 | 502.10 |
| 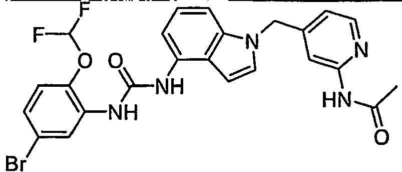 | N-(4-{4-[3-(5-Bromo-2-difluoromethoxy-phenyl)-ureido]-indol-1-ylmethyl}-pyridin-2-yl)-acetamide C24H20BrF2N5O3 543.07 | 544.08 |
| 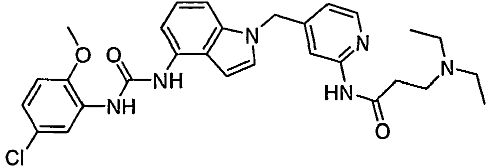 | N-(4-{4-[3-(5-Chloro-2-methoxy-phenyl)-ureido]-indol-1-ylmethyl}-pyridin-2-yl)-3-diethylamino-propionamide C29H33ClN6O3 548.23 | 549.28 |
| 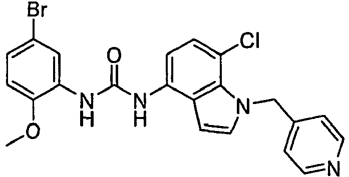 | 1-(5-Bromo-2-methoxy-phenyl)-3-(7-chloro-1-pyridin-4-ylmethyl-1H-indol-4-yl)-urea C22H18BrClN4O2 484.03 | 485.10 |
| 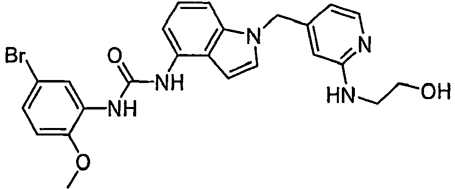 | 1-(5-Bromo-2-methoxy-phenyl)-3- {1-[2-(2-hydroxy-ethylamino)-pyridin-4-ylmethyl]-1H-indol-4-yl}-urea C24H24BrN503 509.11 | 510.05 |
| 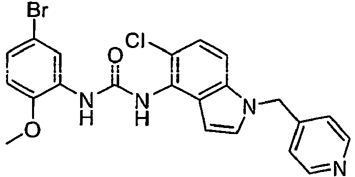 | 1-(5-Bromo-2-methoxy-phenyl)-3-(5-chloro-1-pyridin-4-ylmethyl-1H-indol-4-yl)-urea C22H18BrClN4O2 484.03 | 484.97 |
| 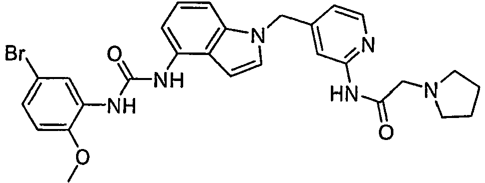 | N-(4-{4-[3-(5-Bromo-2-methoxy-phenyl)-ureido]-indol-1-ylmethyl}-pyridin-2-yl)-2-pyrrolidin-1-yl-acetamide C28H29BrN603 576.15 | 577.22 |
| 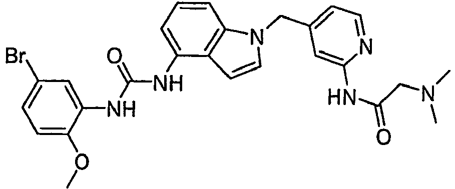 | N-(4-{4-[3-(5-Bromo-2-methoxy-phenyl)-ureido]-indol-1-ylmethyl}-pyridin-2-yl)-2-dimethylamino-acetamide C26H27BrN603 550.13 | 551.21 |
| 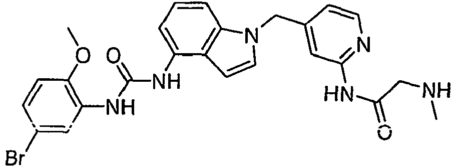 | N-(4-{4-[3-(5-Bromo-2-methoxy-phenyl)-ureido]-indol-1-ylmethyl}-pyridin-2-yl)-2-methylamino-acetamide C25H25BrN603 536.12 | 537.18 |
| 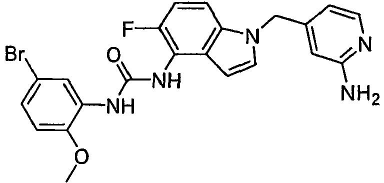 | 1-[1-(2-Amino-pyridin-4-ylmethyl)-5-fluoro-1H-indol-4-yl]-3-(5-bromo-2-methoxy-phenyl)-urea C22H19BrFN5O2 483.07 | 484.13 |
| 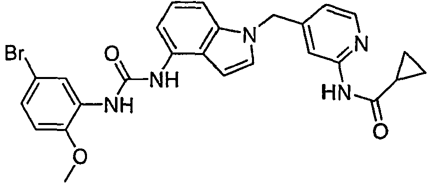 | Cyclopropanecarboxylic acid (4-{4-[3-(5-bromo-2-methoxy-phenyl)-ureido]-indol-1-ylmethyl}-pyridin-2-yl)-amide C26H24BrN503 533.11 | 534.19 |
| 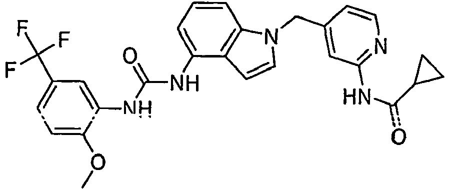 | Cyclopropanecarboxylic acid (4-{4-[3-(2-methoxy-5-trifluoromethyl-phenyl)-ureido]-indol-1-ylmethyl}-pyridin-2-yl)-amide C27H24F3N503 523.18 | 524.21 |
| 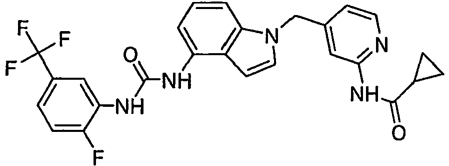 | Cyclopropanecarboxylic acid (4- {4-[3-(2-fluoro-5-trifluoromethyl-phenyl)-ureido]-indol-1-ylmethyl}-pyridin-2-yl)-amide C26H21F4N5O2 511.16 | 512.18 |
| 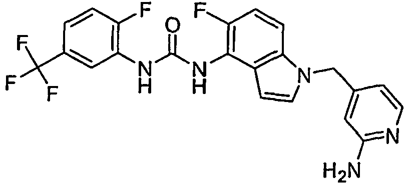 | 1-[1-(2-Amino-pyridin-4-ylmethyl)-5-fluoro-1H-indol-4-yl]-3-(2-fluoro-5-trifluoromethyl-phenyl)-urea C22H16F5N5O 461.13 | 462.16 |
| 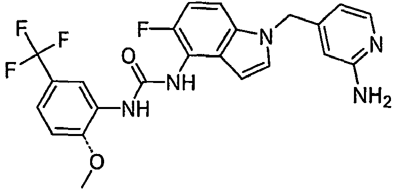 | 1-[1-(2-Amino-pyridin-4-ylmethyl)-5-fluoro-1H-indol-4-yl]-3-(2-methoxy-5-trifluoromethyl-phenyl)-urea C23H19F4N502 473.15 | 474.19 |
| 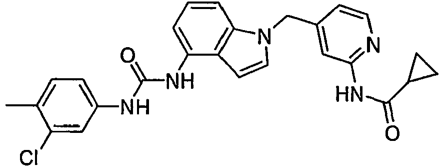 | Cyclopropanecarboxylic acid (4-{4-[3-(3-chloro-4-methyl-phenyl)-ureido]-indol-1-ylmethyl}-pyridin-2-yl)-amide C26H24ClN5O2 473.16 | 474.21 |
| 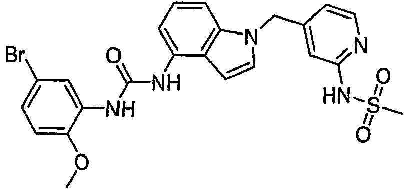 | N-(4- {4-[3-(5-Bromo-2-methoxy-phenyl)-ureido]-indol-1-ylmethyl}-pyridin-2-yl)-methanesulfonamide C23H22BrN504S 543.06 | 544.15 |
| 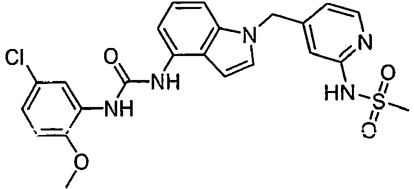 | N-(4-{4-[3-(5-Chloro-2-methoxy-phenyl)-ureido]-indol-1-ylmethyl}-pyridin-2-yl)-methanesulfonamide C23H22ClN5O4S 499.11 | 500.19 |
| 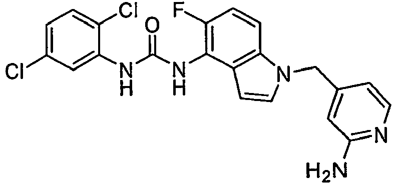 | 1-[1-(2-Amino-pyridin-4-ylmethyl)-5-fluoro-1H-indol-4-yl]-3-(2,5-dichloro-phenyl)-urea C21H16Cl2FN5O 443.07 | 444.11 |
| 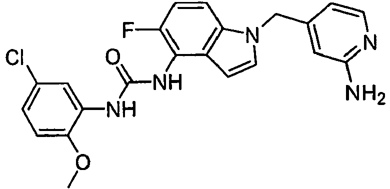 | 1-[1-(2-Amino-pyridin-4-ylmethyl)-5-fluoro-1H-indol-4-yl]-3-(5-chloro-2-methoxy-phenyl)-urea C22H19ClFN5O2 439.12 | 440.17 |
| 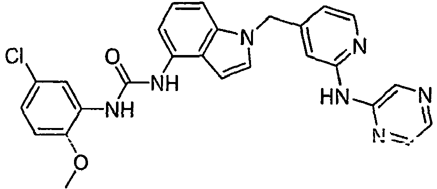 | 1-(5-Chloro-2-methoxy-phenyl)-3-{1-[2-(pyrazin-2-ylamino)-pyridin-4-ylmethyl]-1H-indol-4-yl}-urea C26H22ClN7O2 499.15 | 500.20 |
| 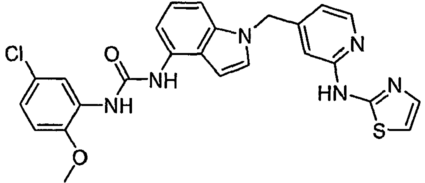 | 1-(5-Chloro-2-methoxy-phenyl)-3-{1-[2-(thiazol-2-ylamino)-pyridin-4-ylmethyl]-1H-indol-4-yl}-urea C25H21ClN6O2S 504.11 | 505.18 |
| 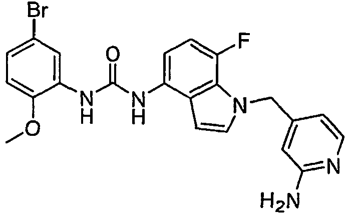 | 1-[1-(2-Amino-pyridin-4-ylmethyl)-7-fluoro-1H-indol-4-yl]-3-(5-bromo-2-methoxy-phenyl)-urea C22H19BrFN5O2 483.07 | 484.16 |
| 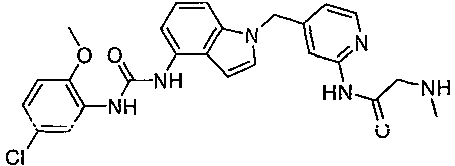 | N-(4-{4-[3-(5-Chloro-2-methoxy-phenyl)-ureido]-indol-1-ylmethyl}-pyridin-2-yl)-2-methylamino-acetamide C25H25ClN6O3 492.17 | 493.22 |
| 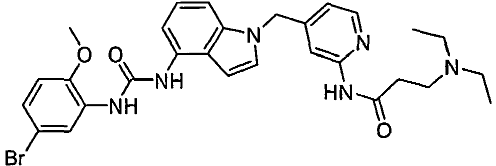 | N-(4-{4-[3-(5-Bromo-2-methoxy-phenyl)-ureido]-indol-1-ylmethyl}-pyridin-2-yl)-3-diethylamino-propionamide C29H33BrN603 592.18 | 593.24 |
| 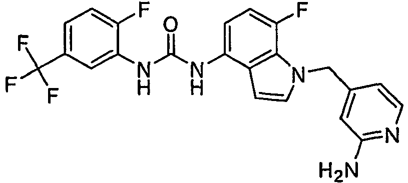 | 1-[1-(2-Amino-pyridin-4-ylmethyl)-7-fluoro-1H-indol-4-yl]-3-(2-fluoro-5-trifluoromethyl-phenyl)-urea C22H16F5N5O 461.13 | 462.17 |
| 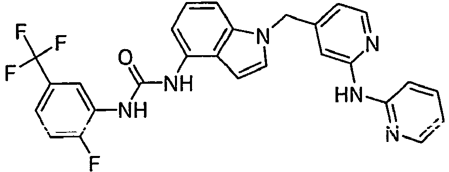 | 1-(2-Fluoro-5-trifluoromethyl-phenyl)-3-{1-[2-(pyridin-2-ylamino)-pyridin-4-ylmethyl]-1H-indol-4-yl}-urea C27H20F4N6O 520.16 | 521.20 |
| 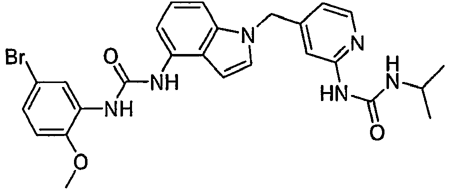 | 1-(5-Bromo-2-methoxy-phenyl)-3- {1-[2-(3-isopropyl-ureido)-pyridin-4-ylmethyl]-1H-indol-4-yl}-urea C26H27BrN603 550.13 | 551.20 |
| 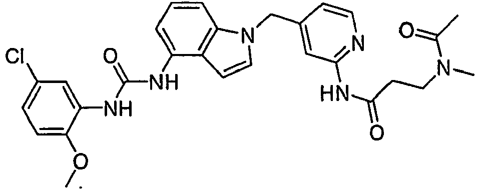 | 3-(Acetyl-methyl-amino)-N-(4-{4-[3-(5-chloro-2-methoxy-phenyl)-ureido]-indol-1-ylmethyl}-pyridin-2-yl)-propionamide C28H29ClN6O4 548.19 | 549.19 |
| 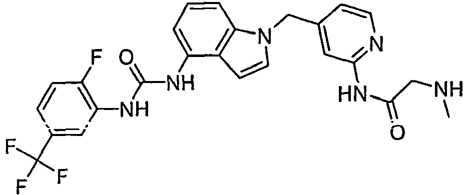 | N-(4-{4-[3-(2-Fluoro-5-trifluoromethyl-phenyl)-ureido]-indol-1-ylmethyl}-pyridin-2-yl)-2-methylamino-acetamide C25H22F4N602 514.17 | 515.17 |
| 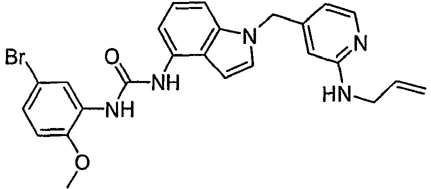 | 1-[1-(2-Allylamino-pyridin-4-ylmethyl)-1H-indol-4-yl]-3-(5-bromo-2-methoxy-phenyl)-urea C25H24BrN502 505.11 | 506.11 |
| 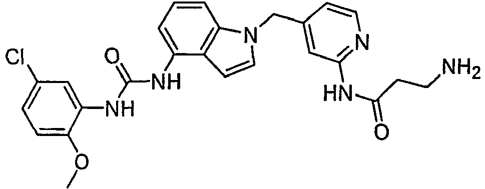 | 3-Amino-N-(4-{4-[3-(5-chloro-2-methoxy-phenyl)-ureido]-indol-1-ylmethyl}-pyridin-2-yl)-propionamide C25H25ClN6O3 492.17 | 493.17 |
| 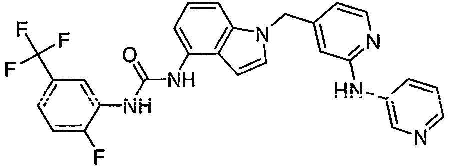 | 1-(2-Fluoro-5-trifluoromethyl-phenyl)-3-{1-[2-(pyridin-3-ylamino)-pyridin-4-ylmethyl]-1H-indol-4-yl}-urea C27H20F4N6O 520.16 | 520.32 |
| 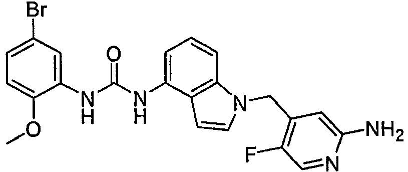 | 1-[1-(2-Amino-5-fluoro-pyridin-4-ylmethyl)-1H-indol-4-yl]-3-(5-bromo-2-methoxy-phenyl)-urea C22H19BrFN5O2 483.07 | 483.28 |
| 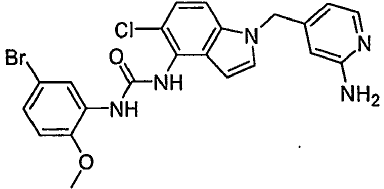 | 1-[1-(2-Amino-pyridin-4-ylmethyl)-5-chloro-1H-indol-4-yl]-3-(5-bromo-2-methoxy-phenyl)-urea C22H19BrClN5O2 499.04 | 499.24 |
| 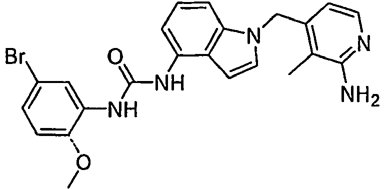 | 1-[1-(2-Amino-3-methyl-pyridin-4-ylmethyl)-1H-indol-4-yl]-3-(5-bromo-2-methoxy-phenyl)-urea C23H22BrN502 479.10 | 479.29 |
| 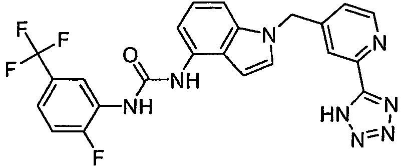 | 1-(2-Fluoro-5-trifluoromethyl-phenyl)-3-{1-[2-(1H-tetrazol-5-yl)-pyridin-4-ylmethyl]-1H-indol-4-yl}-urea C23H16F4N80 496.14 | 496.35 |
| 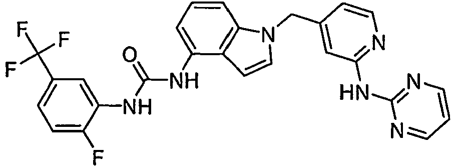 | 1-(2-Fluoro-5-trifluoromethyl-phenyl)-3-{1-[2-(pyrimidin-2-ylamino)-pyridin-4-ylmethyl]-1H-indol-4-yl}-urea C26H19F4N70 521.16 | 521.36 |
| 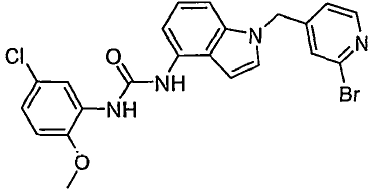 | 1-[1-(2-Bromo-pyridin-4-ylmethyl)-1H-indol-4-yl]-3-(5-chloro-2-methoxy-phenyl)-urea C22H18BrClN4O2 484.03 | 484.26 |
| 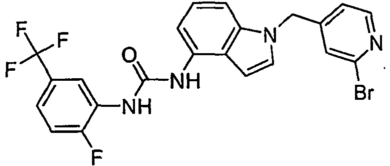 | 1-[1-(2-Bromo-pyridin-4-ylmethyl)-1H-indol-4-yl]-3-(2-fluoro-5-trifluoromethyl-phenyl)-urea C22H15BrF4N4O 506.04 | 506.24 |
| 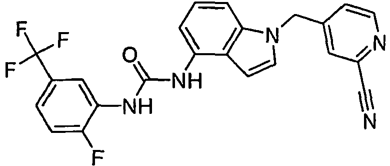 | 1-[1-(2-Cyano-pyridin-4-ylmethyl)-1H-indol-4-yl]-3-(2-fluoro-5-trifluoromethyl-phenyl)-urea C23H15F4N50 453.12 | 453.36 |
| 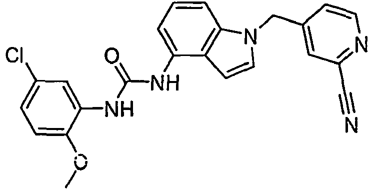 | 1-(5-Chloro-2-methoxy-phenyl)-3-[1-(2-cyano-pyridin-4-ylmethyl)-1H-indol-4-yl]-urea C23H18ClN5O2 431.11 | 431.38 |
| 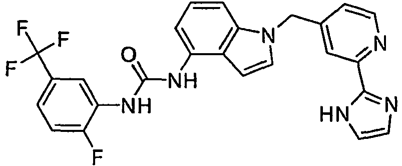 | 1-(2-Fluoro-5-trifluoromethyl-phenyl)-3-{1-[2-(1H-imidazol-2-yl)-pyridin-4-ylmethyl]-1H-indol-4-yl}-urea C25H18F4N60 494.15 | 494.32 |
| 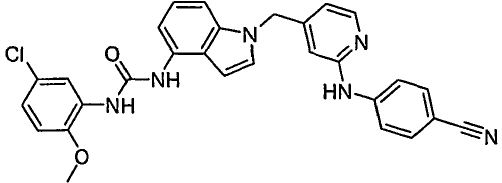 | 1-(5-Chloro-2-methoxy-phenyl)-3-{1-[2-(4-cyano-phenylamino)-pyridin-4-ylmethyl]-1H-indol-4-yl}-urea C29H23ClN6O2 522.16 | 523.12 |
| 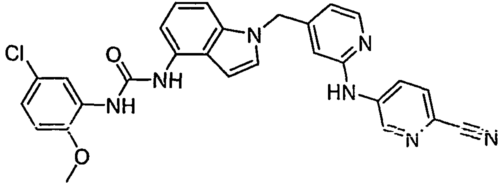 | 1-(5-Chloro-2-methoxy-phenyl)-3-{1-[2-(6-cyano-pyridin-3-ylamino)-pyridin-4-ylmethyl]-1H-indol-4-yl}-urea C28H22ClN7O2 523.15 | 524.12 |

### EXAMPLE 3. ASSAY FOR EPHB₄ KINASE ACTIVTY

The following is a procedure for a standard biochemical assay for EphB₄ Kinase Activity that can be used to test compounds disclosed in this application.

### Materials:

96-well, ½ area flat bottom, white polystyrene plates are purchased from Costar, cat #3693.

The cytoplasmic domain of recombinant EphB₄ kinase (amino acids 596-987, *Homo sapiens* EphB₄, GENBANK Accession No. AY056047.1) with a C-terminal V5-(his)₆ tag is purified from Sf9 cells. Purity of >95% is assessed by Sypro-Ruby staining of SDS gels.

PTK Biotinylated Peptide Substrate 2, is purchased from Promega, cat #V288A.

LANCE Eu-W1024 labeled anti-phosphotyrosine antibody (PT66) is purchased from Perkin-Elmer, cat #AD0068. Kinase Buffer is purchased from Cell Signaling, cat #9802.

Dilutions of compounds are made in 100% DMSO at 20X the final desired concentration. Compounds in 100% DMSO are transferred (1.25 µL) to the 96 well assay plate. A 18.75 µL volume of master mix containing the final concentrations (in 25 ul) of 0.01% BSA, 1X Cell Signaling Kinase Buffer, 0.5 µM PTK Biotinylated Peptide Substrate 2, and 18.6 ng/well ng/well of EphB₄ kinase is added to all wells, except the four negative control wells (which contain no kinase), and mixed. To initiate the reaction, 5 µL of 550 uM ATP is added to each well. (Final Concentration of ATP = 110 µM). The reactions are incubated for 1 hour at room temperature (RT). After incubation a quantity of 8.35 µL of a 4X SA-APC Detection Mix is added to each well. The final concentration of Eu-labelled PT66 antibody is 1 nM and the SA-APC is 20 nM (based on the SA moiety). The reaction plates are incubated at RT for at least 15 minutes after SA-APC Detection Mix addition. The reaction plates are read on an Envision plate reader (Perkin-Elmer) with 605nm Excitation at 605nm and 640nm Emission wavelengths. Values are corrected for the fluorescence in the absence of enzyme and inhibition curves are fit to the data using a Logit curve-fitting algorithm. IC₅₀ values are determined from these inhibition curves.

### EXAMPLE 4. EPHB4 CELLULAR ASSAY

The following is a procedure for a standard cell-based assay for EphB₄ kinase activity that can be used to test compounds disclosed in this application.

HEK293 cells stably expressing V5-epitope tagged EphB₄ are grown to ~75% confluency, and then incubated for 90 min at 37 °C in low serum media (Optimem) containing test compound. Cells are stimulated for 10 minutes at 37 °C with 500ng/ml EphrinB₂/Fc chimera and 50ng/ml goat-anti-human IgG (FC-specific) in low serum media containing test compound. Cells are washed in ice-cold PBS, lysed, and protein assays are performed on the cleared lysates. Equal protein amounts of each sample are subjected to SDS-PAGE and western blotting with either an anti-phosphotyrosine antibody or an anti-V5 antibody to control for total amounts of V5-epitope-tagged EphB₄ in each lysate.

### EXAMPLE 5 BIOCHEMICAL ASSAYS

The following is a procedure for a standard biochemical assay that can be used to test activity of compounds disclosed herein as inhibitors of c-Kit, PDGFRβ, and VEGFR2, kinase activity.

Test compounds are diluted 1:20 from an original 200 µM DMSO stock and incubated with recombinant c-Kit (10 ng), or VEGFR2 (1 ng) enzyme (ProQinase GmbH, Germany), biotinylated peptide (PTK peptide 2, Promega) in Cell Signalling kinase buffer (c-Kit) or Upstate Kinase buffer (VEGFR2) and 5 ul of ATP (final concentrations: 85 uM for the VEGFR2 assayand 150 µM for the c-Kit assay) for 60 minutes at room temperature. For PDGFRβ, test compounds are diluted 1:20 from an original 200 µM DMSO stock and incubated with recombinant PDGFRβ (2 ng) (ProQinase GmbH, Germany), biotinylated peptide (PTK peptide 2, Promega), 1 uM poly-L-lysine (Sigma) in Upstate Kinase buffer and 5 ul of ATP (final concentration: 2.5 uM) for at least 15 minutes at room temperature. The final assay volume is 25 µl. After incubation a detection Mix, which includes 1 nM LANCE Eu-W1024 labeled anti-phosphotyrosine antibody PT66 (Perkin-Elmer, cat #AD0068) and 20 nM SA-APC (based on the SA moiety), is added. The reaction plates are incubated at room temperature for at least 15 minutes after SA-APC detection mix addition. The reaction plates are then read on an Envision plate reader (Perkin-Elmer) with 605nm excitation 615 nM and 640nm emission wavelengths. 2

For a negative control, i.e. a readout in which the kinases are not inhibited, the assay is run without any test compound added. Staurosporine, a general kinase inhibitor, is used as a positive control.

IC₅₀ values are determined from an 11-point saturation binding curve for test compounds that show significant inhibition of one of the tyrosine kinases. In these assays concentration of test compound ranges from 10 µM to 20nM. Equilibrium binding parameters are determined by fitting the allosteric Hill equation to the measured values with the aid of the computer program, such as FitP^{™} (BIOSOFT, Ferguson, MO).

### EXAMPLE 6. TEST RESULTS

Certain compounds described in Example 2 were tested in the assays for EphB₄ kinase activity (as outlined in Examples 3 and 4), and found to exhibit an IC₅₀ of 1 micromolar or less. Certain of those compounds exhibited an IC₅₀ of 500 nM or less in these assays. Certain of those compounds exhibited an IC₅₀ of 50 nM or less in these assays.

Certain compounds of Example 2 were tested in the assay for PDGFRβ kinase activity (as outlined in example 5), and found to exhibit an IC₅₀ of 1 micromolar or less. Certain of those compounds exhibited an IC₅₀ of 500 nM or less in the assay for PDGFRβ kinase activity. Certain of those compounds exhibited an IC₅₀ of 100 nM or less in this assay.

Certain compounds described in Example 2 were tested in the assay for c-Kit kinase activity (as outlined in example 5) and found to exhibit an IC₅₀ of 1 micromolar or less. Certain of those compounds exhibited an IC₅₀ of 500 nM or less in the assay for c-Kit kinase activity. Certain of those compounds exhibited an IC₅₀ of 50 nM or less in this assay.

Certain compounds described in Example 2 were also tested in the assay for VEGFR2 kinase activity (as outlined in example 5). Certain of those compounds were found to exhibit an IC₅₀ of 1 micromolar or less. Certain of those compounds exhibited an IC₅₀ of 100 nM or less in this assay. Certain of those compounds exhibited an IC₅₀ of 50 nM or less in this assay.

Certain compounds described in Example 2 were also tested in the assays described herein and were found to exhibit an IC₅₀ of 1 micromolar or less against two or more kinases chosen from EphB₄, PDGFRβ, c-Kit, and VEGFR2. Certain compounds described in Example 2 were also tested in the assays described herein and were found to exhibit an IC₅₀ of 100 nm or less against two or more kinases chosen from EphB₄, PDGFRβ, c-Kit, and VEGFR2.

Certain compounds described in Example 2 were also tested in the assays described herein and were found to exhibit an IC₅₀ of 1 micromolar or less against three or more kinases chosen from EphB₄, PDGFRβ, c-Kit, and VEGFR2. Certain compounds described in Example 2 were also tested in the assays described herein and were found to exhibit an IC₅₀ of 100 nm or less against three or more kinases chosen from EphB₄, PDGFRβ, c-Kit, and VEGFR2.

Certain compounds described in Example 2 were also tested in the assays described herein and were found to exhibit an IC₅₀ of 1 micromolar or less against each of EphB₄, PDGFRβ, c-Kit, and VEGFR2. Certain compounds described in Example 2 were also tested in the assays described herein and were found to exhibit an IC₅₀ of 100 nm or less against each of EphB₄, PDGFRβ, c-Kit, and VEGFR2.

While certain embodiments have been shown and described, various modifications and substitutions may be made thereto without departing from the scope of the invention as defined by the claims. Accordingly, it is to be understood that the present invention has been described by way of illustration and not limitations.

## Claims

1. At least one chemical entity chosen from compounds of Formula 1 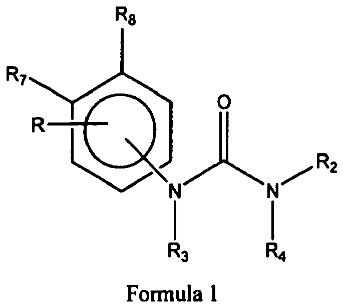 and pharmaceutically acceptable salts, solvates, crystal forms and mixtures thereof, wherein
R represents 0 to 2 substituents independently chosen from hydroxy, nitro, cyano, optionally substituted amino, aminocarbonyl, halo, carboxy, optionally substituted acyl, optionally substituted alkoxycarbonyl, optionally substituted C₁-C₆alkyl, optionally substituted C₁-C₆ alkoxy, sulfanyl, sulfinyl, sulfonyl, optionally substituted aryl, optionally substituted heteroaryl, and optionally substituted heterocycloalkyl;
R₇ and R₈, taken together with the carbons to which they are bound, form a fused 5- to 7-membered heteroaryl ring substituted with a group -(Z₁)ₘR₁, wherein the fused 5- to 7-membered heteroaryl ring is optionally furrther substituted and wherein
R₁ is optionally substituted heteroaryl;
Z₁ is -CR₅R₆- wherein each R₅ and R₆ is independently chosen from hydrogen, optionally substituted C₁-C₆ alkyl, and halo; and
m is chosen from 0, 1, and 2;
R₂ is optionally substituted aryl; and
R₃ and R₄ are each independently chosen from hydrogen, optionally substituted C₁-C₆ alkyl, optionally substituted aryl, and optionally substituted heteroaryl,
wherein
the terms "substituted" alkyl, cycloalkyl, aryl, heterocycloalkyl, and heteroaryl refer respectively to alkyl, cycloalkyl, aryl, heterocycloalkyl, and heteroaryl wherein one or more hydrogen atoms are replaced by a substituent independently chosen from:
-R^{a}, -OR^{b}, -SR^{b}, guanidine, guanidine wherein one or more of the guanidine hydrogens are replaced with a lower-alkyl group, -NR^{b}R^{c}, halo, cyano, nitro, oxo (as a substitutent for cycloalkyl, heterocycloalkyl, and heteroaryl), -COR^{b}, -CO₂R^{b}, -CONR^{b}R^{c}, -OCOR^{b}, -OCO₂R^{a}, -OCONR^{b}R^{c}, -NR^{c}COR^{b}, -NR^{c}CO₂R^{a}, -NR^{c}CONR^{b}R^{c}, -CO₂R^{b}, -CONR^{b}R^{c}, -NR^{c}COR^{b}, -SOR^{a}, -SO₂R^{a}, -SO₂NR^{b}R^{c}, and -NR^{c}SO₂R^{a},
where R^{a} is chosen from optionally substituted C₁-C₆ alkyl, optionally substituted alkenyl, optionally substituted alkynyl, optionally substituted aryl, and optionally substituted heteroaryl;
R^{b} is chosen from H, optionally substituted C₁-C₆ alkyl, optionally substituted cycloalkyl, optionally substituted heterocycloalkyl, optionally substituted aryl, and optionally substituted heteroaryl; and
R^{c} is independently chosen from hydrogen and optionally substituted C₁-C₄ alkyl; or
R^{b} and R^{c}, and the nitrogen to which they are attached, form an optionally substituted heterocycloalkyl group; and
where each optionally substituted group is unsubstituted or independently substituted with one or more, such as one, two, or three, substituents independently selected from C₁-C₄ alkyl, aryl, heteroaryl, aryl-C₁-C₄ alkyl-, heteroaryl-C₁-C₄ alkyl-, C₁-C₄haloalkyl-, -OC₁-C₄ alkyl, -OC₁-C₄ alkylphenyl, -C₁-C₄ allcyl-OH, -OC₁-C₄ haloalkyl, halo, -OH, -NH₂, -C₁-C₄ alkyl-NH₂, -N(C₁-C₄alkyl)(C₁-C₄alkyl), -NH(C₁-C₄alkyl), -N(C₁-C₄ alkyl)(C₁-C₄ alkylphenyl), -NH(C₁-C₄ alkylphenyl), cyano, nitro, oxo (as a substitutent for cycloalkyl, heterocycloalkyl, or heteroaryl), -CO₂H, -C(O)OC₁-C₄ alkyl, -CON(C₁-C₄ alkyl)(C₁-C₄ alkyl), -CONH(C₁-C₄ alkyl), -CONH₂, -NHC(O)(C₁-C₄ alkyl), -NHC(O)(phenyl), -N(C₁-C₄ alkyl)C(O)(C₁-C₄ alkyl), -N(C₁-C₄ alkyl)C(O)(phenyl), -C(O)C₁-C₄ alkyl, -C(O)C₁-C₄ alkylphenyl, -C(O)C₁-C₄ haloalkyl, -OC(O)C₁-C₄ alkyl, - SO₂(C₁-C₄ alkyl), -SO₂(phenyl), -SO₂(C₁-C₄ haloalkyl), -SO₂NH₂, -SO₂NH(C₁-C₄ alkyl), -SO₂NH(phenyl), -NHSO₂(C₁-C₄ alkyl), -NHSO₂(phenyl), and -NHSO₂(C₁-C₄ haloalkyl),
the term "substituted acyl" refers to the groups (substituted alkyl)-C(O)-; (substituted cycloalkyl)-C(O)-; (substituted aryl)-C(O)-; (substituted heteroaryl)-C(O)-; and (substituted heterocycloalkyl)-C(O)-, wherein the group is attached to the parent structure through the carbonyl functionality and wherein substituted alkyl, cycloalkyl, aryl, heteroaryl, and heterocycloalkyl, refer respectively to alkyl, cycloalkyl, aryl, heteroaryl, and heterocycloalkyl wherein one or more hydrogen atoms are replaced by a substituent independently chosen from:
-R^{a}, -OR^{b}, -SR^{b}, guanidine, guanidine wherein one or more of the guanidine hydrogens are replaced with a lower-alkyl group, -NR^{b}R^{c}, halo, cyano, nitro, -COR^{b}, -CO₂R^{b}, -CONR^{b}R^{c}, -OCOR^{b}, -OCO₂R^{a}, -OCONR^{b}R^{c}, -NR^{c}COR^{b}, -NR^{c}CO₂R^{a}, -NR^{c}CONR^{b}R^{c}, -CO₂R^{b}, -CONR^{b}R^{c}, -NR^{c}COR^{b}, -SOR^{a}, -SO₂R^{a}, -SO₂NR^{b}R^{c}, and -NR^{c}SO₂R^{a},
The term "substituted alkoxy" refers to alkoxy wherein the alkyl constituent is subsfituted (i.e., -O-(substituted alkyl)) wherein "substituted alkyl" refers to alkyl wherein one or more hydrogen atoms are replaced by a substituent independently chosen from:
-R^{a}, -OR^{b}, -SR^{b}, guanidine, guanidine wherein one or more of the guanidine hydrogens are replaced with a lower-alkyl group, -NR^{b}R^{c}, halo, cyano, nitro, -COR^{b}, -CO₂R^{b}, -CONR^{b}R^{c}, -OCOR^{b}, -OCO₂R^{a}, -OCONR^{b}R^{c}, -NR^{c}COR^{b}, -NR^{c}CO₂R^{a}, -NR^{c}CONR^{b}R^{c}, -CO₂R^{b}, -CONR^{b}R^{c}, -NR^{c}COR^{b}, -SOR^{a}, -SO₂R^{a}, -SO₂NR^{b}R^{c}, and -NR^{c}SO₂R^{a},
a substituted alkoxy group may be "polyalkoxy" or -O-(optionally substituted alkylene)-(optionally substituted alkoxy) or hydroxyalkoxy or -OCH₂(CH₂)_{y}OH, where y is an integer of 1-10, such as 1-4,
the term "substituted alkoxycarbonyl" refers to the group (substituted alkyl)-O-C(O)- wherein the group is attached to the parent structure through the carbonyl functionality and wherein substituted refers to alkyl wherein one or more
hydrogen atoms are replaced by a substituent independently chosen from:
-R^{a}, -OR^{b}, -SR^{b}, guanidine, guanidine wherein one or more of the guanidine hydrogens are replaced with a lower-alkyl group, -NR^{b}R^{c}, halo, cyano, nitro, -COR^{b}, -CO₂R^{b}, -CONR^{b}R^{c}, -OCOR^{b}, -OCO₂R^{a}, -OCONR^{b}R^{c}, -NR^{c}COR^{b}, -NR^{c}CO₂R^{a}, -NR^{c}CONR^{b}R^{c}, -CO₂R^{b}, -CONR^{b}R^{c}, -NR^{c}COR^{b}, -SOR^{a}, -SO₂R^{a}, -SO₂NR^{b}R^{c}, and -NR^{c}SO₂R^{a},
the term "substituted amino" refers to the group -NHR^{d} or -NR^{d}R^{e} wherein R^{d} is chosen from: hydroxy, optionally substitued alkoxy, optionally substituted alkyl, optionally substituted cycloalkyl, optionally substituted acyl, aminocarbonyl, optionally substituted aryl, optionally substituted heteroaryl, optionally substituted heterocycloalkyl, optionally substituted alkoxycarbonyl, sulfinyl and sulfonyl, and wherein R^{e} is chosen from: optionally substituted alkyl, optionally substituted cycloalkyl, optionally substituted aryl, optionally substituted heteroaryl, and optionally substituted heterocycloalkyl, and wherein substituted alkyl, cycloalkyl, aryl, heterocycloalkyl, and heteroaryl refer respectively to alkyl, cycloalkyl, aryl, heterocycloalkyl, and heteroaryl wherein one or more
hydrogen atoms are replaced by a substituent independently chosen from:
-R^{a}, -OR^{b}, -SR^{b}, guanidine, guanidine wherein one or more of the guanidine hydrogens are replaced with a lower-alkyl group, -NR^{b}R^{c}, halo, cyano, nitro, -COR^{b}, -CO₂R^{b}, -CONR^{b}R^{c}, -OCOR^{b}, -OCO₂R^{a}, -OCONR^{b}R^{c}, -NR^{c}COR^{b} -NR^{c}CO₂R^{a}, -NR^{c}CONR^{b}R^{c}, -CO₂R^{b}, -CONR^{b}R^{c}, -NR^{c}COR^{b}, -SOR^{a}, -SO₂R^{a}, -SO₂NR^{b}R^{c}, and -NR^{c}SO₂R^{a},
the term "substituted amino" also refers to N-oxides of the groups -NHR^{d}, and NR^{d}R^{d}
*provided that*
the compound of Formula 1 is not chosen from 5-(phenylcarbamoylamino)-3-(2-(4-pyridyl)ethyl)indole; 1-(4-chloro-3-(trifluoromethyl)phenyl)-3-(1-(pyridin-4-yl)-1H-indol-5-yl)urea; and 1-(2-methoxy-5-(trifluoromethyl)phenyl)-3-(1-(pyridin-4-yl)-1H-indol-5-yl)urea.

2. At least one chemical entity of claim 1 wherein R₇ and R₈, taken together with the carbons to which they are bound, form a fused 5-membered heteroaryl ring substituted with a group -(Z₁)ₘR₁ wherein the heteroaryl ring contains at least one nitrogen and optionally includes one or more additional heteroatoms, selected from N, O, and S in the ring.

3. At least one chemical entity of claim 2 wherein R₇ and R₈, taken together with the carbons to which they are bound, form a fused ring chosen from pyrazolyl, imidazolyl, isoxazolyl,oxazolyl, thiazolyl, triazolyl, and pyrrolyl, each of which is substituted with a group - (Z₁)ₘR₁.

4. At least one chemical entity of claim 3 wherein R₇ and R₈, taken together with the carbons to which they are bound, form a fused heteroaryl ring chosen from 1H-pyrrolyl and 1H-pyrazolyl, each of which is substituted with a group -(Z₁))ₘR₁.

5. At least one chemical entity of claim 1 wherein the compound of Formula 1 is chosen from compounds of Formula 2 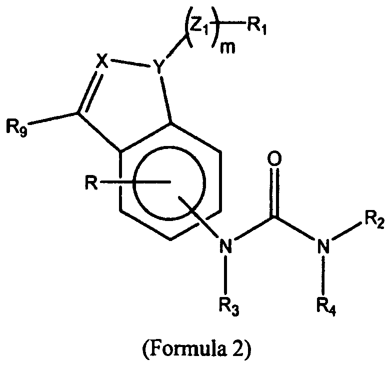 wherein
X and Y are independently chosen from CH and N; and
R₉ is chosen from hydrogen and optionally substituted alkyl.

6. At least one chemical entity of any one of claims 1 to 5 wherein R₁ is chosen from pyridinyl and substituted pyridinyl wherein substituted pyridinyl is chosen from mono-, di-, and tri-substituted pyridinyls and wherein substituents on the substituted pyridinyl are independently chosen from hydroxy, nitro, cyano, optionally substituted amino, aminocarbonyl, halo, carboxy, optionally substituted acyl, optionally substituted alkoxycarbonyl, optionally substituted C₁-C₆ alkyl, optionally substituted C₁-C₆ alkoxy, sulfanyl, sulfinyl, sulfonyl, optionally substituted aryl, optionally substituted heteroaryl, and optionally substituted heterocycloalkyl.

7. At least one chemical entity of claim 6 wherein the substituents on the substituted pyridinyl are independently chosen from hydroxy, nitro, cyano, optionally substituted amino, halo, carboxy, optionally substituted C₁-C₆ alkyl, optionally substituted C₁-C₆alkoxy, C₁-C₆ alkylsulfanyl, C₁-C₆ acyl, C₁-C₆alkoxycarbonyl, optionally substituted heteroaryl, and heterocycloalkyl.

8. At least one chemical entity of claim 7 wherein the substituents on the substituted pyridinyl are independently chosen from hydroxy, cyano, halo, optionally substituted C₁-C₂ alkyl, optionally substituted C₁-C₂ alkoxy, and -NHR₁₀ wherein R₁₀ is chosen from hydrogen and optionally substituted acyl.

9. At least one chemical entity of claim 6 wherein R₁ is pyridin-4-yl.

10. At least one chemical entity of any one of claims 1 to 9 wherein at least one of R₅ and R₆ is hydrogen.

11. At least one chemical entity of claim 10 wherein both of R₅ and R₆ are hydrogen.

12. At least one chemical entity of any one of claims 1 to 9 wherein m is 1 and at least one of R₅ and R₆ is hydrogen.

13. At least one chemical entity of claim 10 wherein both of R₅ and R₆ are hydrogen.

14. At least one chemical entity of any one of claims 1 to 9 wherein m is 0.

15. At least one chemical entity of claim 5 wherein the compound of Formula 2 is chosen from compounds of Formula 3 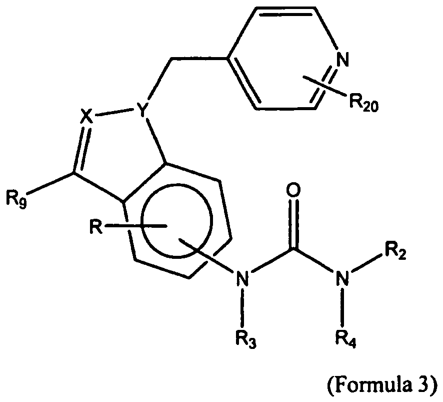 wherein
R₂₀ represents 0 to 3 substituents independently chosen from hydroxy, nitro, cyano, optionally substituted amino, aminocarbonyl, halo, carboxy, optionally substituted acyl, optionally substituted alkoxycarbonyl, optionally substituted C₁-C₆alkyl, optionally substituted C₁-C₆ alkoxy, sulfanyl, sulfinyl, sulfonyl, optionally substituted aryl, optionally substituted heteroaryl, and optionally substituted heterocycloalkyl.

16. At least one chemical entity of claim 15 wherein the compound of Formula 3 is chosen from compounds of Formula 4

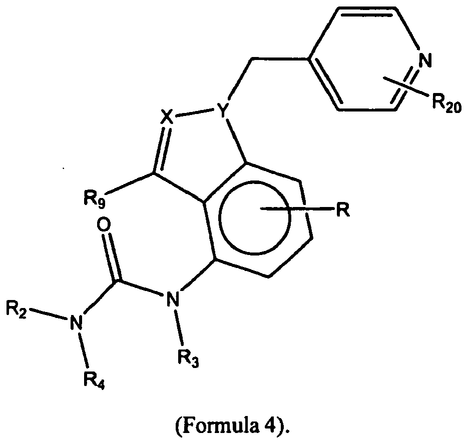

17. At least one chemical entity of any one of claims 5 to 16 wherein X and Y are N.

18. At least one chemical entity of any one of claims 5 to 16 wherein Y is N and X is CH.

19. At least one chemical entity of any one of claims 1 to 18 wherein R₂ is chosen from phenyl and substituted phenyl wherein substituted phenyl is chosen from mono-, di-, and tri-substituted phenyls and wherein substituents on the substituted phenyl are independently chosen from hydroxy, nitro, cyano, optionally substituted amino, aminocarbonyl, halo, carboxy, optionally substituted acyl, optionally substituted alkoxycarbonyl, optionally substituted C₁-C₆ alkyl, optionally substituted C₁-C₆ alkoxy, optionally substituted aryloxy, sulfanyl, sulfinyl, sulfonyl, optionally substituted aryl, optionally substituted heteroaryl, andoptionally substituted heterocycloalkyl.

20. At least one chemical entity of claim 19 wherein the substituents on the substituted phenyl are independently chosen from hydroxy, nitro, cyano, optionally substituted amino, halo, carboxy, optionally substituted C₁-C₆ alkyl, optionally substituted C₁-C₆ alkoxy, optionally substituted phenyl, optionally substituted phenoxy, C₁-C₆ alkylsulfanyl, C₁-C₆ acyl, C₁-C₆alkoxycarbonyl, optionally substituted heteroaryl, and heterocycloalkyl.

21. At least one chemical entity of claim 20 wherein the substituents on the substituted phenyl are independently chosen from hydroxy, cyano, halo, optionally substituted C₁-C₂ alkyl, phenoxy, and optionally substituted C₁-C₂ alkoxy.

22. At least one chemical entity of claim 21 wherein the substituents on the substituted phenyl are independently chosen from halo, methyl, ethyl, methoxy, ethoxy, difluoromethyl, trifluoromethyl, difluoromethoxy, and trifluoromethoxy.

23. At least one chemical entity of claim 16 wherein the compounds of Formula 4 are chosen from compounds of Formula 5 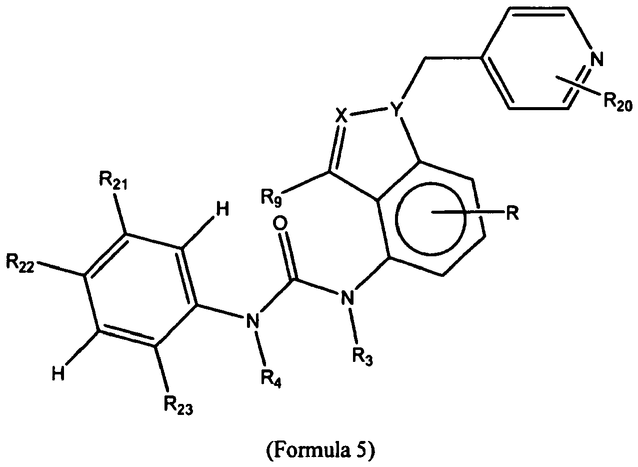 wherein
R₂₁ is chosen from hydrogen, halo and optionally substituted lower alkyl;
R₂₂ is chosen from hydrogen, halo, lower alkoxy, and lower alkyl; and
R₂₃ is chosen from hydrogen, lower alkyl, optionally substituted phenoxy, optionally substituted lower alkoxy, and halo.

24. At least one chemical entity of claim 23 wherein R₂₁ is chosen from hydrogen, halo, methyl, and trifluoromethyl.

25. At least one chemical entity of claim 23 or 24 wherein R₂₂ is chosen from hydrogen, halo, methoxy, and methyl.

26. At least one chemical entity of claim 25 wherein R₂₂ is hydrogen.

27. At least one chemical entity of any one of claims 23 to 25 wherein R₂₃ is chosen from hydrogen, methyl, methoxy, difluoromethoxy, trifluoromethoxy, ethoxy, and halo.

28. At least one chemical entity of any one of claims 15 to 27 wherein R₂₀ is optionally substituted amino.

29. At least one chemical entity of any one of claims 5 to 28 wherein R₉ is chosen from hydrogen and optionally substituted lower alkyl.

30. At least one chemical entity of claim 29 wherein R₉ is chosen from hydrogen and lower alkyl.

31. At least one chemical entity of claim 30 wherein R₉ is hydrogen.

32. At least one chemical entity of any one of claims 1 to 31 wherein R represents 1 or 2 substituents independently chosen from halo, C₁-C₂ alkyl, and C₁-C₂ alkoxy.

33. At least one chemical entity of claim 32 wherein R represents 1 or 2 substituents independently chosen from halo, methyl, and methoxy.

34. At least one chemical entity of claim 33 wherein R represents a substituent chosen from halo, methyl, and methoxy.

35. At least one chemical entity of any one of claims 1 to 34 wherein R is absent.

36. At least one chemical entity of any one of claims 1 to 35 wherein R₃ and R₄ are each independently chosen from hydrogen and methyl.

37. At least one chemical entity of claim 36 wherein R₃ and R₄ are hydrogen.

38. At least one chemical entity of claim 1 wherein the compound of Formula 1 is chosen from
1-(5-Bromo-2-methoxy-phenyl)-3-(1-pyridin-4-ylmethyl-1H-indol-4-yl)-urea;
1-(2-Methoxy-5-trifluoromethyl-phenyl)-3-(1-pyridin-4-ylmethyl-1H-indol-4-yl)-urea;
1-(2-Methoxy-5-trifluoromethyl-phenyl)-3-(1-pyridin-4-ylmethyl-1H-indol-6-yl)-urea;
1-(4-Chloro-2-methoxy-5-trifluoromethyl-phenyl)-3-(1-pyridin-4-ylmethyl-1H-indol-4-yl)-urea;
1-(2-Fluoro-5-trifluoromethyl-phenyl)-3-(1-pyridin-4-ylmethyl-1H-indol-4-yl)-urea;
1-(2,4-Dimethoxy-5-trifluoromethyl-phenyl)-3-(1-pyridin-4-ylmethyl-1H-indol-4-yl)-urea;
1-(2,4-Dimethyl-5-trifluoromethyl-phenyl)-3-(1-pyridin-4-ylmethyl-1H-indol-4-yl)-urea;
1-(2-Ethoxy-5-trifluoromethyl-phenyl)-3-(1-pyridin-4-ylmethyl-1H-indol-4-yl)-urea;
1-(5-Bromo-2-methoxy-phenyl)-3-(1-pyridin-4-ylmethyl-1H-indazol-4-yl)-urea;
1-(5-Bromo-2-ethoxy-phenyl)-3-(1-pyridin-4-ylmethyl-1H-indol-4-yl)-urea;
1-(5-Bromo-2-methoxy-phenyl)-3-(1-pyridin-4-ylmethyl-1H-indol-5-yl)-urea;
1-(4-Methyl-3-trifluoromethyl-phenyl)-3-(1-pyridin-4-ylmethyl-1H-indol-4-yl)-urea;
1-(4-Chloro-3-trifluoromethyl-phenyl)-3-(1-pyridin-4-ylmethyl-H-indol-4-yl)-urea;
1-(5-Chloro-2-methoxy-phenyl)-3-(1-pyridin-4-ylmethyl-1H-indol-5-yl)-urea;
1-(2-Methoxy-5-trifluoromethyl-phenyl)-3-(1-pyridin-4-ylmethyl-1H-indol-5-yl)-urea;
1-(4-Chloro-3-trifluoromethyl-phenyl)-3-(1-pyridin-4-ylmethyl-1H-indol-5-yl)-urea;
1-(2-Fluoro-5-trifluoromethyl-phenyl)-3-(1-pyridin-4-ylmethyl-1H-indol-5-yl)-urea;
1-(2-Chloro-5-trifluoromethyl-phenyl)-3-(1-pyridin-4-ylmethyl-1H-indol-5-yl)-urea;
1-(2-Fluoro-5-trifluoromethyl-phenyl)-3-(3-methyl-1-pyridin-4-ylmethyl-1H-indol-4-yl)-urea;
1-(2-Methoxy-5-trifluoromethyl-phenyl)-3-(3-methyl-1-pyridin-4-ylmethyl-1H-indol-4-yl)-urea;
1-(5-Bromo-2-methoxy-phenyl)-3-(3-methyl-1-pyridin-4-ylmethyl-1H-indol-4-yl)-urea;
1-(5-Bromo-2-methoxy-phenyl)-3-(1-pyridin-3-ylmethyl-1H-indol-4-yl)-urea;
1-(5-Bromo-2-methoxy-phenyl)-3-(1-pyridin-3-ylmethyl-1H-indol-5-yl)-urea;
1-(5-Bromo-2-methoxy-phenyl)-3-(5-methyl-1-pyridin-4-ylmethyl-1H-indol-4-yl)-urea;
1-(5-Bromo-2-methoxy-phenyl)-3-(7-methyl-1-pyridin-4-ylmethyl-1H-indol-4-yl)-urea;
1-(2-Methoxy-5-trifluoromethyl-phenyl)-3-(5-methyl-1-pyridin-4-ylmethyl-1H-indol-4-yl)-urea;
1-(2-Fluoro-5-trifluoromethyl-phenyl)-3-(5-methyl-l-pyridin-4-ylmethyl-1H-indol-4-yl)-urea;
1-(5-Chloro-2-methoxy-phenyl)-3-(5-methyl-1-pyridin-4-ylmethyl-1 H-indol-4-yl)-urea;
1-(4-Methoxy-biphenyl-3-yl)-3-(1-pyridin-4-ylmethyl-1H-indol-4-yl)-urea;
1-(2-Methoxy-5-trifluoromethyl-phenyl)-3-(7-methyl-1-pyridin-4-ylmethyl-1H-indol-4-yl)-urea;
1-(2-Fluoro-5-trifluoromethyl-phenyl)-3-(7-methyl-1-pyridin-4-ylmethyl-1H-indol-4-yl)-urea;
1-(5-Chloro-2-methoxy-phenyl)-3-(7-methyl-1-pyridin-4-ylmethyl-1H-indol-4-yl)-urea;
4-{4-[3-(2-Methoxy-5-trifluoromethyl-phenyl)-ureido]-5-methyl-indol-1-ylmethyl}-pyridine-2-carboxylic acid methylamide;
1-(7-Methyl-1-pyridin-4-ylmethyl-1H-indol-4-yl)-3-(4-methyl-3-trifluoromethyl-phenyl)-urea;
1-(4-Chloro-3-trifluoromethyl-phenyl)-3-(7-methyl-1-pyridin-4-ylmethyl-1H-indol-4-yl)-urea;
1-[5-Chloro-2-([1,3]dioxolan-2-ylmethoxy)-phenyl]-3-(1-pyridin-4-ylmethyl-1H-indol-4-yl)-urea;
1-[5-Chloro-2-(3-hydroxy-propoxy)-phenyl]-3-(5-methyl-1-pyridin-4-ylmethyl-1H-indol-4-yl)-urea;
1-(3-Chloro-4-methoxy-phenyl)-3-(1-pyridin-4-ylmethyl-1H-indol-4-yl)-urea;
1-(4-Methoxy-3-trifluoromethyl-phenyl)-3-(1-pyridin-4-ylmethyl-1H-indol-4-yl)-urea;
1-(2-Methoxy-4-methyl-5-trifluoromethyl-phenyl)-3-(1-pyridin-4-ylmethyl-1H-indol-4-yl)-urea;
1-(3-Bromo-4-methyl-phenyl)-3-(1-pyridin-4-ylmethyl-1H-indol-4-yl)-urea;
1-(4-Fluoro-3-trifluoromethyl-phenyl)-3-(1-pyridin-4-ylmethyl-1H-indol-4-yl)-urea;
1-(5-Bromo-2-methoxy-4-methyl-phenyl)-3-(1-pyridin-4-ylmethyl-1H-indol-4-yl)-urea;
1-(5-Bromo-2-methoxy-phenyl)-3-[1-(2-fluoro-pyridin-4-ylmethyl)-1H-indol-4-yl]-urea;
1-(5-Bromo-2-methoxy-phenyl)-3-[1-(2-methyl-pyridin-4-ylmethyl)-1H-indol-4-yl]-urea;
1-(3-Bromo-4-methoxy-phenyl)-3-(1-pyridin-4-ylmethyl-1H-indol-4-yl)-urea;
1-(5-Bromo-2-methoxy-phenyl)-3-[1-(3-fluoro-pyridin-4-ylmethyl)-1H-indol-4-yl]-urea;
1-[1-(3-Fluoro-pyridin-4-ylmethyl)-1H-indol-4-yl]-3-(4-methyl-3-trifluoromethyl-phenyl)-urea;
1-(3-Chloro-4-fluoro-phenyl)-3-(1-pyridin-4-ylmethyl-1H-indol-4-yl)-urea;
1-[1-(2-Amino-pyridin-4-ylmethyl)-1H-indol-4-yl]-3-(5-bromo-2-methoxy-phenyl)-urea;
1-(3,4-Dimethoxy-phenyl)-3-(1-pyridin-4-ylmethyl-1H-indol-4-yl)-urea;
1-(3-Chloro-4-methyl-phenyl)-3-(1-pyridin-4-ylmethyl-1H-indol-4-yl)-urea;
1-(5-Chloro-2-methoxy-4-methyl-phenyl)-3-(1-pyridin-4-ylmethyl-1H-indol-4-yl)-urea;
1-[1-(2-Amino-pyridin-4-ylmethyl)-1H-indol-4-yl]-3-(5-bromo-2-methoxy-4-methyl-phenyl)-urea;
1-[1-(2-Amino-pyridin-4-ylmethyl)-1H-indol-4-yl]-3-(3-chloro-4-methyl-phenyl)-urea;
1-[1-(2-Amino-pyridin-4-ylmethyl)-1H-indol-4-yl]-3-(5-chloro-2-methoxy-phenyl)-urea;
1-[1-(2-Amino-pyridin-4-ylmethyl)-1H-indol-4-yl]-3-(5-chloro-2,4-dimethoxy-phenyl)-urea;
1-[1-(2-Amino-pyridin-4-ylmethyl)-1H-indol-4-yl]-3-(5-chloro-2-methoxy-4-methyl-phenyl)-urea;
1-[1-(2-Amino-pyridin-4-ylmethyl)-1H-indol-4-yl]-3-(2,4-dimethyl-5-trifluoromethyl-phenyl)-urea;
1-(5-Bromo-2-methoxy-phenyl)-3-(5-methoxy-1-pyridin-4-ylmethyl-1H-indol-4-yl)-urea;
1-[1-(2-Amino-pyridin-4-ylmethyl)-1H-indol-4-yl]-3-(2-methoxy-5-trifluoromethyl-phenyl)-urea;
1-[1-(2-Amino-pyridin-4-ylmethyl)-1H-indol-4-yl]-3-(2,4-dimethoxy-5-trifluoromethyl-phenyl)-urea;
1-[1-(2-Amino-pyridin-4-ylmethyl)-1H-indol-4-yl]-3-(2-methoxy-4-methyl-5-trifluoromethylphenyl)-urea;
1-Benzo[1,3]dioxol-5-yl-3-(1-pyridin-4-ylmethyl-1H-indol-4-yl)-urea;
1-(5-Bromo-2-methoxy-phenyl)-3-(6-methyl-1-pyridin-4-ylmethyl-1H-indol-4-yl)-urea;
(4-{4-[3-(5-Bromo-2-methoxy-phenyl)-ureido]-indol-1-ylmethyl}-pyridin-2-yl)-carbamicacid methyl ester;
1-(5-Bromo-2-methoxy-phenyl)-3-[1-(3-chloro-pyridin-4-ylmethyl)-1H-indol-4-yl]-urea;
1-(5-Fluoro-2,4-dimethoxy-phenyl)-3-(1-pyridin-4-ylmethyl-1H-indol-4-yl)-urea;
1-(3-Bromo-4-fluoro-phenyl)-3-(1-pyridin-4-ylmethyl-1H-indol-4-yl)-urea;
N-(4-{4-[3-(5-Bromo-2-methoxy-phenyl)-ureido]-indol-1-ylmethyl}-pyridin-2-yl)-acetamide;
1-(5-Ethanesulfonyl-2-methoxy-phenyl)-3-(1-pyridin-4-ylmethyl-1H-indol-4-yl)-urea;
1-(5-Brcmo-2,4-difluoro-phenyl)-3-(1-pyridin-4-ylmethyl-1H-indol-4-yl)-urea;
1-[1-(2-Amino-pyridin-4-ylmethyl)-1H-indol-4-yl]-3-(2-fluoro-5-trifluoromethyl-phenyl)-urea;
N-(4-{4-[3-(5-Chloro-2-methoxy-4-methyl-phenyl)-ureido]-indol-1-ylmethyl}-pyridin-2-yl)-acetamide;
1-(5-Bromo-2-methoxy-phenyl)-3-(7-fluoro-1-pyridin-4-ylmethyl-1H-indol-4-yl)-urea;
1-(5-Chloro-2-methoxy-4-methyl-phenyl)-3-(7-fluoro-1-pyridin-4-ylmethyl-1H-indol-4-yl)-urea;
N-(4-{4-[3-(5-Bromo-2-methoxy-phenyl)-ureido]-indol-1-ylmethyl}-pyridin-2-yl)-2-hydroxy-acetamide;
2-Amino-N-(4-{4-[3-(5-bromo-2-methoxy-phenyl)-ureido]-indol-1-ylmethyl}-pyridin-2-yl)-acetamide;
N-(4-{4-[3-(5-Bromo-2-methoxy-phenyl)-ureido]-indol-1-ylmethyl}-pyridin-2-yl)-formamide;
1-[1-(2-Amino-pyridin-4-ylmethyl)-1H-indol-4-yl]-3-(3-bromo-4-methoxy-phenyl)-urea;
N-(4-{4-[3-(3-Chloro-4-methyl-phenyl)-ureido]-indol-1-ylmethyl}-pyridin-2-yl)-acetamide;
1-(5-Bromo-2-methoxy-phenyl)-3-(7-methoxy-1-pyridin-4-ylmethyl-1H-indol-4-yl)-urea;
1-(2,4-Dimethoxy-5-trifluoromethyl-phenyl)-3-(7-methoxy-1-pyridin-4-ylmethyl-1H-indol-4-yl)-urea;
1-(5-Bromo-2-methoxy-phenyl)-3-(5-fluoro-1-pyridin-4-ylmethyl-1H-indol-4-yl)-urea;
N-(4- {4-[3-(5-Chloro-2-methoxy-phenyl)-ureido]-indol-1-ylmethyl}-pyridin-2-yl)-acetamide;
1-Methyl-1H-imidazole-2-carboxylic acid (4-{4-[3-(5-bromo-2-methoxy-phenyl)-ureido]-indol-1-ylmethyl}-pyridin-2-yl)-amide;
N-(4-{4-[3-(5-Bromo-2-methoxy-phenyl)-ureido]-indol-1-ylmethyl}-pyridin-2-yl)-propionamide;
N-(4-{4-[3-(5-Bromo-2-methoxy-phenyl)-ureido]-indol-1-ylmethyl}-pyridin-2-yl)-isobutyramide;
N-(4-{4-[3-(5-Bromo-2-methoxy-phenyl)-ureido]-indol-1-ylmethyl}-pyridin-2-yl)-2-morpholin-4-yl-acetamide;
1-(5-Bromo-2-methoxy-phenyl)-3-[1-(2-methylamino-pyridin-4-ylmethyl)-1H-indol-4-yl]-urea;
1-(5-Bromo-2-methoxy-phenyl)-3-{1-[2-(2-methoxy-ethylamino)-pyridin-4-ylmethyl]-1H-indol-4-yl}-urea;
1-(5-Bromo-2-methoxy-phenyl)-3-[1-(2-morpholin-4-yl-pyridin-4-ylmethyl)-1H-indol-4-yl]-urea;
1-(5-Bromo-2-methoxy-phenyl)-3-[1-(2-methoxy-pyridin-4-ylmethyl)-1H-indol-4-yl]-urea;
1-(5-Bromo-2-methoxy-phenyl)-3-[1-(2-hydroxy-pyridin-4-ylmethyl)-1H-indol-4-yl]-urea;
N-(4-{4-[3-(2-Fluoro-5-trifluoromethyl-phenyl)-ureido]-indol-1-ylmethyl}-pyridin-2-yl)-acetamide;
N-(4-{4-[3-(5-Chloro-2-methoxy-phenyl)-ureido]-indol-1-ylmethyl}-pyridin-2-yl)-2-morpholin-4-yl-acetamide;
N-(4-{4-[3-(5-Bromo-2-methoxy-phenyl)-ureido]-indol-1-ylmethyl}-pyridin-2-yl)-2-methoxyacetamide;
1-(5-Chloro-2-methoxy-phenyl)-3-[1-(3-methyl-pyridin-4-ylmethyl)-1H-indol-4-yl]-urea;
Cyclopropanecarboxylic acid (4-{4-[3-(5-chloro-2-methoxy-phenyl)-ureido]-indol-1-ylmethyl}-pyridin-2-yl)-amide;
1-[1-(2-Amino-pyridin-4-ylmethyl)-1H-indol-4-yl]-3-(5-bromo-2-difluoromethoxy-phenyl)-urea;
N-(4- {4-[3-(5-Bromo-2-difluoromethoxy-phenyl)-ureido]-indol-1-ylmethyl}-pyridin-2-yl)-acetamide;
N-(4-{4-[3-(5-Chloro-2-methoxy-phenyl)-ureido]-indol-1-ylmethyl}-pyridin-2-yl)-3-diethylamino-propionamide;
1-(5-Bromo-2-methoxy-phenyl)-3-(7-chloro-1-pyridin-4-ylmethyl-1H-indol-4-yl)-urea;
1-(5-Bromo-2-methoxy-phenyl)-3-{1-[2-(2-hydroxy-ethylamino)-pyridin-4-ylmethyl]-1H-indol-4-yl}-urea;
1-(5-Bromo-2-methoxy-phenyl)-3-(5-chloro-1-pyridin-4-ylmethyl-1H-indol-4-yl)-urea;
N-(4- {4-[3-(5-Bromo-2-methoxy-phenyl)-ureido]-indol-1-ylmethyl}-pyridin-2-yl)-2-pyrrolidin-1-yl-acetamide;
N-(4-{4-[3-(5-Bromo-2-methoxy-phenyl)-ureido]-indol-1-ylmethyl}-pyridin-2-yl)-2-dimethylamino-acetamide;
N-(4-{4-[3-(5-Bromo-2-methoxy-phenyl)-ureido]-indol-1-ylmethyl}-pyridin-2-yl)-2-methylamino-acetamide;
1-[1-(2-Amino-pyridin-4-ylmethyl)-5-fluoro-1H-indol-4-yl]-3-(5-bromo-2-methoxy-phenyl)-urea;
Cyclopropanecarboxylic acid (4-{4-[3-(5-bromo-2-methoxy-phenyl)-ureido]-indol-1-ylmethyl}-pyridin-2-yl)-amide;
Cyclopropanecarboxylic acid (4-{4-[3-(2-methoxy-5-trifluoromethyl-phenyl)-ureido]-indol-1-ylmethyl}-pyridin-2-yl)-amide;
Cyclopropanecarboxylic acid (4-{4-[3-(2-fluoro-5-trifluoromethyl-phenyl)-ureido]-indol-1-ylmethyl}-pyridin-2-yl)-amide;
1-[1-(2-Amino-pyridin-4-ylmethyl)-5-fluoro-1H-indol-4-yl]-3-(2-fluoro-5-trifluoromethylphenyl)-urea;
1-[1-(2-Amino-pyridin-4-ylmethyl)-5-fluoro-1H-indol-4-yl]-3-(2-methoxy-5-trifluoromethylphenyl)-urea;
Cyclopropanecarboxylic acid (4-{4-[3-(3-chloro-4-methyl-phenyl)-ureido]-indol-1-ylmethyl}-pyridin-2-yl)-amide;
N-(4-{4-[3-(5-Bromo-2-methoxy-phenyl)-ureido]-indol-1-ylmethyl}-pyridin-2-yl)-methanesulfonamide;
N-(4-{4-[3-(5-Chloro-2-methoxy-phenyl)-ureido]-indol-1-ylmethyl}-pyridin-2-yl)-methanesulfonamide;
1-[1-(2-Amino-pyridin-4-ylmethyl)-5-fluoro-1H-indol-4-yl]-3-(2,5-dichloro-phenyl)-urea;
1-[1-(2-Amino-pyridin-4-ylmethyl)-5-fluoro-1H-indol-4-yl]-3-(5-chloro-2-methoxy-phenyl)-urea;
1-(5-Chloro-2-methoxy-phenyl)-3-{1-[2-(pyrazin-2-ylamino)-pyridin-4-ylmethyl]-1H-indol-4-yl}-urea;
1-(5-Chloro-2-methoxy-phenyl)-3-{1-[2-(thiazol-2-ylamino)-pyridin-4-ylmethyl]-1H-indol-4-yl}-urea;
1-[1-(2-Amino-pyridin-4-ylmethyl)-7-fluoro-1H-indol-4-yl]-3-(5-bromo-2-methoxy-phenyl)-urea;
N-(4-{4-[3-(5-Chloro-2-methoxy-phenyl)-ureido]-indol-1-ylmethyl}-pyridin-2-yl)-2-methylamino-acetamide;
N-(4-{4-[3-(5-Bromo-2-methoxy-phenyl)-ureido]-indol-1-ylmethyl}-pyridin-2-yl)-3-diethylamino-propionamide;
1-[1-(2-Amino-pyridin-4-ylmethyl)-7-fluoro-1H-indol-4-yl]-3-(2-fluoro-5-trifluoromethylphenyl)-urea;
1-(2-Fluoro-5-trifluoromethyl-phenyl)-3-{1-[2-(pyridin-2-ylamino)-pyridin-4-ylmethyl]-1H-indol-4-yl}-urea;
1-(5-Bromo-2-methoxy-phenyl)-3-{1-[2-(3-isopropyl-ureido)-pyridin-4-ylmethyl]-1H-indol-4-yl}-urea;
3-(Acetyl-methyl-amino)-N-(4-{4-[3-(5-chloro-2-methoxy-phenyl)-ureido]-indol-1-ylmethyl}-pyridin-2-yl)-propionamide;
N-(4- {4-[3-(2-Fluoro-5-trifluoromethyl-phenyl)-ureido]-indol-1-ylmethyl}-pyridin-2-yl)-2-methylamino-acetamide;
1-[1-(2-Allylamino-pyridin-4-ylmethyl)-1H-indol-4-yl]-3-(5-bromo-2-methoxy-phenyl)-urea;
3-Amino-N-(4-{4-[3-(5-chloro-2-methoxy-phenyl)-ureido]-indol-1-ylmethyl}-pyridin-2-yl)-propionamide;
1-(2-Fluoro-5-trifluoromethyl-phenyl)-3-{1-[2-(pyridin-3-ylamino)-pyridin-4-ylmethyl]-1H-indol-4-yl}-urea;
1-[1-(2-Amino-5-fluoro-pyridin-4-ylmethyl)-1H-indol-4-yl]-3-(5-bromo-2-methoxy-phenyl)-urea;
1-[1-(2-Amino-pyridin4-ylmethyl)-5-chloro-1H-indol-4-yl]-3-(5-bromo-2-methoxy-phenyl)-urea;
1-[1-(2-Amino-3-methyl-pyridin-4-ylmethyl)-1H-indol-4-yl]-3-(5-bromo-2-methoxy-phenyl)-urea;
1-(2-Fluoro-5-trifluoromethyl-phenyl)-3-{1-[2-(1H-tetraol-5-yl)-pyridin-4-ylmethyl]-1H-indol-4-yl}-urea;
1-(2-Fluoro-5-trifluoromethyl-phenyl)-3-{1-[2-(pyrimidin-2-ylamino)-pyridin-4-ylmethyl]-1H-indol-4-yl}-urea;
1-[1-(2-Bromo-pyridin-4-ylmethyl)-1H-indol-4-yl]-3-(5-chloro-2-methoxy-phenyl)-urea;
1-[1-(2-Bromo-pyridin-4-ylmethyl)-1H-indol-4-yl]-3-(2-fluoro-5-trifluoromethyl-phenyl)-urea;
1-[1-(2-Cyano-pyridin-4-ylmethyl)-1H-indol-4-yl]-3-(2-fluoro-5-trifluoromethyl-phenyl)-urea;
1-(5-Chloro-2-methoxy-phenyl)-3-[1-(2-cyano-pyridin-4-ylmethyl)-1H-indol-4-yl]-urea;
1-(2-Fluoro-5-trifluoromethyl-phenyl)-3-{1-[2-(1H-imidazol-2-yl)-pyridin-4-ylmethyl]-1H-indol-4-yl}-urea;
1-(5-Chloro-2-methoxy-phenyl)-3-{1-[2-(4-cyano-phenylamino)-pyridin-4-ylmethyl]-1H-indol-4-yl}-urea; and
1-(5-Chloro-2-methoxy-phenyl)-3-{1-[2-(6-cyano-pyridin-3-ylamino)-pyridin-4-ylmethyl]-1H-indol-4-yl}-urea.

39. At least one chemical entity of claim 1 wherein the chemical entitiy is 1-[1-(2-Amino-pyridin-4-ylmethyl)-1 H-indol-4-yl]-3-(5-bromo-2-methoxy-phenyl)-urea or a pharmaceutically acceptable salt, solvate or crystal form thereof.

40. A pharmaceutical composition, comprising at least one chemical entity of any one of claims 1 to 39, together with at least one pharmaceutically acceptable vehicle chosen from carriers, adjuvants, and excipients.

41. A pharmaceutical composition of claim 40, wherein the composition is formulated in a form chosen from injectable fluids, aerosols, creams, gels, tablets, pills, capsules, syrups, ophthalmic solutions, and transdermal patches.

42. A packaged pharmaceutical composition, comprising the pharmaceutical composition of claim 40 or 41 in a container; and instructions for using the composition to treat a patient suffering from a disease or disorder responsive to kinase activity modulation of one or more tyrosine kinase.

43. The use of at least one chemical entity for the manufacture of a medicament for the treatment of a patient having a disease responsive to inhibition of at least one kinase chosen from VEGFR2, EphB₄, PDGFRβ, and c-Kit, wherein the at least one chemical entity is a chemical entity of any one of claims 1 to 39.

44. The use of claim 43, wherein the disease responsive to inhibition of at least one kinase is responsive to inhibition of VEGFR2 kinase activity.

45. The use of claim 44, wherein the disease responsive to inhibition of VEGFR2 kinase activity is chosen from cancer and diseases **characterized by** a change in angiogenesis.

46. The use of claim 45, wherein the diseases **characterized by** a change in angiogenesis are chosen from cancerous tumor, macular degeneration, and diabetic retinopathy.

47. A method for the manufacture of a medicament for the treatment of a patient having a disease responsive to inhibition of at least one kinase chosen from VEGFR2, EphB₄, PDGFRβ, and c-Kit, comprising including in said medicament at least one chemical entity of any one of claims 1 to 39.

48. The method of claim 47, wherein the disease responsive to inhibition of at least one kinase is responsive to inhibition of VEGFR2 kinase activity.

49. The method of claim 48, wherein the disease responsive to inhibition of VEGFR2 kinase activity is chosen from cancer and diseases **characterized by** a change in angiogenesis.

50. The method of claim 49, wherein the diseases **characterized by** a change in angiogenesis are chosen from cancerous tumor, macular degeneration, and diabetic retinopathy.

51. The use of at least one chemical entity for the manufacture of a medicament for the treatment of a patient having a female reproductive disorder or condition wherein the at least one chemical entity is a chemical entity of any one of claims 1 to 39.

52. The use of claim 51 wherein the female reproductive disorder or condition is chosen from endometriosis, endometrial carcinoma, gynecologic bleeding disorders, irregular menstrual cycles, ovulation, premenstrual syndrome (PMS), and menopausal dysfunction.

53. A chemical entity of any one of claims 1 to 39 for use in treatment of a patient having (i) a disease responsive to inhibition of at least one kinase chosen from VEGFR2, EphB₄, PDGFRβ, and c-Kit, or (ii) a female reproductive disorder or condition.

## Patentansprüche

1. Mindestens eine chemische Einheit, ausgewählt aus Verbindungen der Formel 1 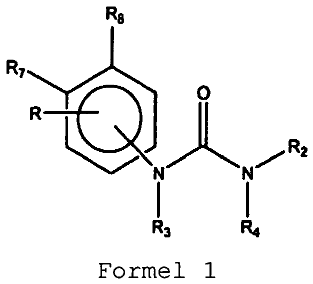 und pharmazeutisch annehmbare Salze, Solvate, Kristallformen und Gemische davon, wobei
R 0 bis 2 Substituenten darstellt, die unabhängig ausgewählt sind aus Hydroxy, Nitro, Cyano, optional substituiertem Amino, Aminocarbonyl, Halogen, Carboxy, optional substituiertem Acyl, optional substituiertem Alkoxycarbonyl, optional substituiertem C₁-C₆-Alkyl, optional substituiertem C₁-C₆-Alkoxy, Sulfanyl, Sulfinyl, Sulfonyl, optional substituiertem Aryl, optional substituiertem Heteroaryl und optional substituiertem Heterocycloalkyl;
R₇ und R₈ gemeinsam mit den Kohlenstoffen, an die sie gebunden sind, einen fusionierten 5- bis 7-gliedrigen Heteroarylring, substituiert mit einer -(Z₁)ₘR₁-Gruppe, bilden, wobei der fusionierte 5-bis 7-gliedrige Heteroarylring optional weiter substituiert ist und wobei
R₁ optional substituiertes Heteroaryl ist;
Z₁ -CR₅R₆- ist, wobei jedes R₅ und R₆ unabhängig ausgewählt ist aus Wasserstoff, optional substituiertem C₁-C₆-Alkyl und Halogen; und
m ausgewählt ist aus 0, 1 und 2;
R₂ optional substituiertes Aryl ist; und
R₃ und R₄ jeweils unabhängig ausgewählt sind aus Wasserstoff, optional substituiertem C₁-C₆-Alkyl, optional substituiertem Aryl und optional substituiertem Heteroaryl, wobei
die Begriffe "substituiertes" Alkyl, Cycloalkyl, Aryl, Heterocycloalkyl und Heteroaryl sich auf Alkyl, Cycloalkyl, Aryl, Heterocycloalkyl bzw. Heteroaryl beziehen, bei welchen ein oder mehrere Wasserstoffatome durch einen Substituenten ersetzt sind, der unabhängig ausgewählt ist aus:
-R^{a}, -OR^{b} -SR^{b}, Guanidin, Guanidin, bei dem ein oder mehrere der Guanidinwasserstoffe durch eine Niederalkylgruppe ersetzt sind, -NR^{b}R^{c}, Halogen, Cyano, Nitro, Oxo (als Substituent für Cycloalkyl, Heterocycloalkyl und Heteroaryl), -COR^{b}, -CO₂R^{b}, -CONR^{b}R^{c}, -OCOR^{b}, -OCO₂R^{a}, -OCONR^{b}R^{c}, -NR^{c}COR^{b}, - NR^{c}CO₂R^{a}, -NR^{c}CONR^{b}R^{c}, -CO₂R^{b}, -CONR^{b}R^{c}, -NR^{c}COR^{b}, - SOR^{a}, -SO₂R^{a}, -SO₂NR^{b}R^{c} und -NR^{c}SO₂R^{a},
wobei R^{a} ausgewählt ist aus optional substituiertem C₁-C₆-Alkyl, optional substituiertem Alkenyl, optional substituiertem Alkinyl, optional substituiertem Aryl und optional substituiertem Heteroaryl;
R^{b} ausgewählt ist aus H, optional substituiertem C₁-C₆-Alkyl, optional substituiertem Cycloalkyl, optional substituiertem Heterocycloalkyl, optional substituiertem Aryl und optional substituiertem Heteroaryl; und
R^{c} unabhängig ausgewählt ist aus Wasserstoff und optional substituiertem C₁-C₄-Alkyl; oder
R^{b} und R^{c} und der Stickstoff, an dem sie hängen, eine optional substituierte Heterocycloalkylgruppe bilden; und
wobei jede optional substituierte Gruppe unsubstituiert oder mit einem oder mehreren, wie einem, zwei oder drei, Substituenten unabhängig substituiert ist, die unabhängig ausgewählt sind aus C₁-C₄-Alkyl, Aryl, Heteroaryl, Aryl-C₁-C₄-alkyl-, Heteroaryl-C₁-C₄-alkyl-, C₁-C₄-Halogenalkyl-, -OC₁-C₄-Alkyl, -OC₁-C₄-Alkylphenyl, -C₁-C₄-Alkyl-OH, -OC₁-C₄-Halogenalkyl, Halogen, -OH, -NH₂, -C₁-C₄-Alkyl-NH₂, -N (C₁-C₄-Alkyl) (C₁-C₄-alkyl), -NH (C₁-C₄-Alkyl), -N (C₁-C₄-Alkyl) (C₁-C₄-alkylphenyl), -NH(C₁-C₄-Alkylphenyl), Cyano, Nitro, Oxo (als Substituent für Cycloalkyl, Heterocycloalkyl oder Heteroaryl), -CO₂H, -C(O)OC₁-C₄-Alkyl, -CON(C₁-C₄-Alkyl) (C₁-C₄-Alkyl), -CONH (C₁-C₄-Alkyl), -CONH₂, -NHC (O) (C₁-C₄-Alkyl), -NHC(O) (Phenyl), -N(C₁-C₄-Alkyl)C(O) (C₁-C₄-Alkyl), -N (C₁-C₄-Alkyl) C(O)(phenyl), -C(O) C₁-C₄-Alkyl, -C (O) C₁-C₄-Alkylphenyl, -C (O) C₁-C₄-Halogenalkyl, -OC(O)C₁-C₄-Alkyl, -SO₂(C₁-C₄-Alkyl), -SO₂(Phenyl), -SO₂(C₁-C₄-Halogenalkyl), -SO₂NH₂, - SO₂NH(C₁-C₄-Alkyl), -SO₂NH (Phenyl), -NHSO₂(C₁-C₄-Alkyl), -NHSO₂(Phenyl) und -NHSO₂(C₁-C₄-Halogenalkyl);
der Begriff "substituiertes Acyl" sich auf die Gruppen (substituiertes Alkyl)-C(O)-; (substituiertes Cycloalkyl)-C(O)-; (substituiertes Aryl)-C(O)-; (substituiertes Heteroaryl)-C(O)-; und (substituiertes Heterocycloalkyl)-C(O)- bezieht, wobei die Gruppe an der Stammstruktur durch die Carbonylfunktionalität hängt und wobei substituiertes Alkyl, Cycloalkyl, Aryl, Heteroaryl und Heterocycloalkyl sich auf Alkyl, Cycloalkyl, Aryl, Heteroaryl bzw. Heterocycloalkyl beziehen, bei dem ein oder mehrere Wasserstoffatome durch einen Substituenten ersetzt sind, der unabhängig ausgewählt ist aus:
-R^{a}, -OR^{b}, -SR^{b}, Guanidin, Guanidin, bei dem ein oder mehrere der Guanidinwasserstoffe mit einer Niederalkylgruppe, -NR^{b}R^{c}, Halogen, Cyano, Nitro, - COR^{b}, -CO²R^{b}, -CONR^{b}R^{c}, -OCOR^{b}, -OCO₂R^{a}, -OCONR^{b}R^{c}, - NR^{c}COR^{b}, -NR^{c}CO₂R^{a}, -NR^{c}CONR^{b}R^{c}, -CO₂R^{b}, -CONR^{b}R^{c}, - NR^{c}COR^{b}, -SOR^{a}, -SO₂R^{a}, -SO₂NR^{b}R^{c} und -NR^{c}SO₂R^{a} ersetzt sind,
der Begriff "substituiertes Alkoxy" sich auf Alkoxy bezieht, wobei der Alkylbestandteil substituiert ist (d.h. -O-(substituiertes Alkyl)), wobei sich "substituiertes Alkyl" auf Alkyl bezieht, bei dem ein oder mehrere Wasserstoffatome durch einen Substituenten ersetzt sind, der unabhängig ausgewählt ist aus:
-R^{a}, -OR^{b}, -SR^{b}, Guanidin, Guanidin, bei dem ein oder mehrere der Guanidinwasserstoffe mit einer Niederalkylgruppe, -NR^{b}R^{c}, Halogen, Cyano, Nitro, - COR^{b}, -CO₂R^{b}, -CONR^{b}R^{c}, -OCOR^{b}, -OCO₂R^{a}, -OCONR^{b}R^{c}, - NR^{c}COR^{b}, -NR^{c}CO₂R^{a}, -NR^{c}CONR^{b}R^{c}, -CO₂R^{b}, -CONR^{b}R^{c}, - NR^{c}COR^{b}, -SOR^{a}, -SO₂R^{a}, -SO₂NR^{b}R^{c} und -NR^{c}SO₂R^{a} ersetzt sind,
eine substituierte Alkoxygruppe "Polyalkoxy" oder -O-(optional substituiertes Alkylen)-(optional substituiertes Alkoxy) oder Hydroxyalkoxy oder -OCH₂(CH₂)yOH sein kann, wobei y eine ganze Zahl von 1-10, wie 1-4 ist,
der Begriff "substituiertes Alkoxycarbonyl" sich auf die Gruppe (substituiertes Alkyl)-O-C(O)- bezieht, wobei die Gruppe an der Stammstruktur durch die Carbonylfunktionalität hängt und wobei sich substituiert auf Alkyl bezieht, bei dem ein oder mehrere Wasserstoffatome durch einen Substituenten ersetzt sind, der unabhängig ausgewählt ist aus:
-R^{a}, -OR^{b}, -SR^{b}, Guanidin, Guanidin, bei dem ein oder mehrere der Guanidinwasserstoffe mit einer Niederalkylgruppe, -NR^{b}R^{c}, Halogen, Cyano, Nitro, - COR^{b}, -CO₂R^{b}, -CONR^{b}R^{c}, -OCOR^{b}, -OCO₂R^{a}, -OCONR^{b}R^{c}, - NR^{c}COR^{b}, -NR^{c}CO₂R^{a}, -NR^{c}CONR^{b}R^{c}, -CO₂R^{b}, -CONR^{b}R^{c}, - NR^{c}COR^{b}, -SOR^{a}, -SO₂R^{a}, -SO₂NR^{b}R^{c} und -NR^{c}SO₂R^{a} ersetzt sind,
der Begriff "substituiertes Amino" sich auf die Gruppe -NHR^{d} oder -NR^{d}R^{e} bezieht, wobei
R^{d} ausgewählt ist aus: Hydroxy, optional substituiertem Alkoxy, optional substituiertem Alkyl, optional substituiertem Cycloalkyl, optional substituiertem Acyl, Aminocarbonyl, optional substituiertem Aryl, optional substituiertem Heteroaryl, optional substituiertem Heterocycloalkyl, optional substituiertem Alkoxycarbonyl, Sulfinyl und Sulfonyl, und wobei R^{e} ausgewählt ist aus: optional substituiertem Alkyl, optional substituiertem Cycloalkyl, optional substituiertem Aryl, optional substituiertem Heteroaryl und optional substituiertem Heterocycloalkyl und wobei sich substituiertes Alkyl, Cycloalkyl, Aryl, Heterocycloalkyl und Heteroaryl auf Alkyl, Cycloalkyl, Aryl, Heterocycloalkyl bzw. Heteroaryl beziehen, bei dem ein oder mehrere Wasserstoffatome durch einen Substituenten ersetzt sind, der unabhängig ausgewählt ist aus:
-R^{a}, -OR^{b}, -SR^{b}, Guanidin, Guanidin, bei dem ein oder mehrere der Guanidinwasserstoffe mit einer Niederalkylgruppe, -NR^{b}R^{c}, Halogen, Cyano, Nitro, - COR^{b}, -CO₂R^{b}, -CONR^{b}R^{c}, -OCOR^{b}, -OCO₂R^{a}, -OCONR^{b}R^{c}, - NR^{c}COR^{b}, -NR^{c}CO₂R^{a}, -NR^{c}CONR^{b}R^{c}, -CO₂R^{b}, -CONR^{b}R^{c}, - NR^{c}COR^{b}, -SOR^{a}, -SO₂R^{a}, -SO₂NR^{b}R^{c} und -NR^{c}SO₂R^{a} ersetzt sind,
der Begriff "substituiertes Amino" sich auch auf N-Oxide der Gruppen -NHR^{d} und NR^{d}R^{d} bezieht,
vorausgesetzt, dass
die Verbindung von Formel 1 nicht ausgewählt ist aus 5-(Phenylcarbamoylamino)-3-(2-(4-pyridyl)ethyl)indol; 1-(4-Chlor-3-(trifluormethyl)phenyl)-3-(1-(pyridin-4-yl)-1H-indol-5-yl)harnstoff; und 1-(2-Methoxy-5-(trifluormethyl)phenyl)-3-(1-(pyridin-4-yl)-1H-indol-5-yl)harnstoff.

2. Mindestens eine chemische Einheit nach Anspruch 1, wobei R₇ und R₈ gemeinsam mit den Kohlenstoffen, an die sie gebunden sind, einen fusionierten 5-gliedrigen Heteroarylring, substituiert mit einer -(Z₁)ₘR₁-Gruppe bilden, wobei der Heteroarylring mindestens einen Stickstoff enthält und optional ein oder mehrere zusätzliche Heteroatome, ausgewählt aus N, O und S im Ring enthält.

3. Mindestens eine chemische Einheit nach Anspruch 2, wobei R₇ und R₈ gemeinsam mit den Kohlenstoffen, an die sie gebunden sind, einen fusionierten Ring bilden, ausgewählt aus Pyrazolyl, Imidazolyl, Isoxazolyl, Oxazolyl, Thiazolyl, Triazolyl und Pyrrolyl, von welchen jedes mit einer -(Z₁)ₘR₁-Gruppe substituiert ist.

4. Mindestens eine chemische Einheit nach Anspruch 3, wobei R₇ und R₈ gemeinsam mit den Kohlenstoffen, an die sie gebunden sind, einen fusionierten Heteroarylring bilden, ausgewählt aus 1H-Pyrrolyl und 1H-Pyrazolyl, von welchen jedes mit einer - (Z₁)ₘR₁-Gruppe substituiert ist.

5. Mindestens eine chemische Einheit nach Anspruch 1, wobei die Verbindung von Formel 1 ausgewählt ist aus Verbindungen von Formel 2 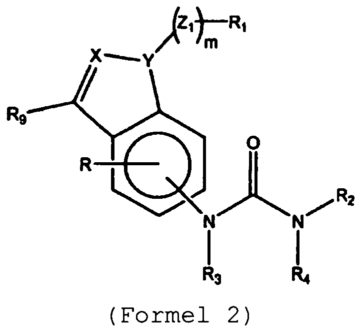 wobei
X und Y unabhängig ausgewählt sind aus CH und N; und
R₉ ausgewählt ist aus Wasserstoff und optional substituiertem Alkyl.

6. Mindestens eine chemische Einheit nach einem der Ansprüche 1 bis 5, wobei R₁ ausgewählt ist aus Pyridinyl und substituiertem Pyridinyl, wobei substituiertes Pyridinyl ausgewählt ist aus mono-, di-, und tri-substituierten Pyridinylen und wobei Substituenten an dem substituierten Pyridinyl unabhängig ausgewählt sind aus Hydroxy, Nitro, Cyano, optional substituiertem Amino, Aminocarbonyl, Halogen, Carboxy, optional substituiertem Acyl, optional substituiertem Alkoxycarbonyl, optional substituiertem C₁-C₆-Alkyl, optional substituiertem C₁-C₆-Alkoxy, Sulfanyl, Sulfinyl, Sulfonyl, optional substituiertem Aryl, optional substituiertem Heteroaryl und optional substituiertem Heterocycloalkyl.

7. Mindestens eine chemische Einheit nach Anspruch 6, wobei die Substituenten auf dem substituiertem Pyridinyl unabhängig ausgewählt sind aus Hydroxy, Nitro, Cyano, optional substituiertem Amino, Halogen, Carboxy, optional substituiertem C₁-C₆-Alkyl, optional substituiertem C₁-C₆-Alkoxy, C₁-C₆-Alkylsulfanyl, C₁-C₆-Acyl, C₁-C₆-Alkoxycarbonyl, optional substituiertem Heteroaryl und Heterocycloalkyl.

8. Mindestens eine chemische Einheit nach Anspruch 7, wobei die Substituenten auf dem substituierten Pyridinyl unabhängig ausgewählt sind aus Hydroxy, Cyano, Halogen, optional substituiertem C₁-C₂-Alkyl, optional substituiertem C₁-C₂-Alkoxy und - NHR₁₀ wobei R₁₀ ausgewählt ist aus Wasserstoff und optional substituiertem Acyl.

9. Mindestens eine chemische Einheit nach Anspruch 6, wobei R₁ Pyridin-4-yl ist.

10. Mindestens eine chemische Einheit nach einem der Ansprüche 1 bis 9, wobei mindestens eines von R₅ und R₆ Wasserstoff ist.

11. Mindestens eine chemische Einheit nach Anspruch 10, wobei beide von R₅ und R₆ Wasserstoff sind.

12. Mindestens eine chemische Einheit nach einem der Ansprüche 1 bis 9, wobei m 1 ist und mindestens eines von R₅ und R₆ Wasserstoff ist.

13. Mindestens eine chemische Einheit nach Anspruch 10, wobei beide von R₅ und R₆ Wasserstoff sind.

14. Mindestens eine chemische Einheit nach einem der Ansprüche 1 bis 9, wobei m 0 ist.

15. Mindestens eine chemische Einheit nach Anspruch 5 wobei die Verbindung von Formel 2 ausgewählt ist aus Verbindungen von Formel 3 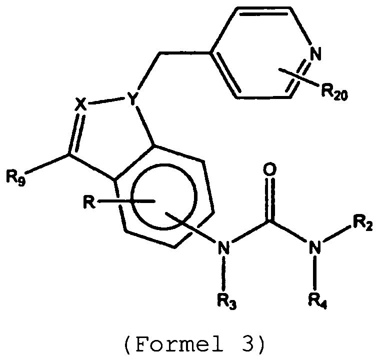 wobei
R₂₀ 0 bis 3 Substituenten darstellt, die unabhängig ausgewählt sind aus Hydroxy, Nitro, Cyano, optional substituiertem Amino, Aminocarbonyl, Halogen, Carboxy, optional substituiertem Acyl, optional substituiertem Alkoxycarbonyl, optional substituiertem C₁-C₆-Alkyl, optional substituiertem C₁-C₆-Alkoxy, Sulfanyl, Sulfinyl, Sulfonyl, optional substituiertem Aryl, optional substituiertem Heteroaryl und optional substituiertem Heterocycloalkyl.

16. Mindestens eine chemische Einheit nach Anspruch 15, wobei die Verbindung von Formel 3 ausgewählt ist aus Verbindungen von Formel 4

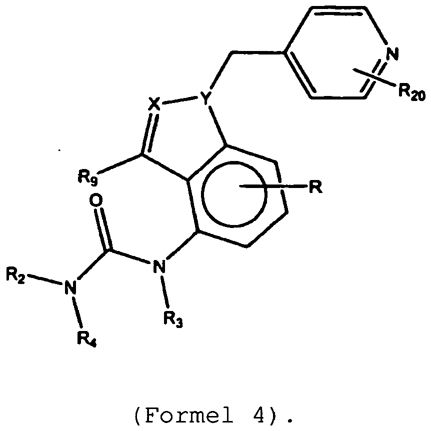

17. Mindestens eine chemische Einheit nach einem der Ansprüche 5 bis 16, wobei X und Y N sind.

18. Mindestens eine chemische Einheit nach einem der Ansprüche 5 bis 16, wobei Y N ist und X CH ist.

19. Mindestens eine chemische Einheit nach einem der Ansprüche 1 bis 18, wobei R₂ ausgewählt ist aus Phenyl und substituiertem Phenyl wobei substituiertes Phenyl ausgewählt ist aus mono-, di-, und tri-substituierten Phenylen und wobei Substituenten auf dem substituierten Phenyl unabhängig ausgewählt sind aus Hydroxy, Nitro, Cyano, optional substituiertem Amino, Aminocarbonyl, Halogen, Carboxy, optional substituiertem Acyl, optional substituiertem Alkoxycarbonyl, optional substituiertem C₁-C₆-Alkyl, optional substituiertem C₁-C₆-Alkoxy, optional substituiertem Aryloxy, Sulfanyl, Sulfinyl, Sulfonyl, optional substituiertem Aryl, optional substituiertem Heteroaryl und optional substituiertem Heterocycloalkyl.

20. Mindestens eine chemische Einheit nach Anspruch 19, wobei die Substituenten auf dem substituiertem Phenyl unabhängig ausgewählt sind aus Hydroxy, Nitro, Cyano, optional substituiertem Amino, Halogen, Carboxy, optional substituiertem C₁-C₆-Alkyl, optional substituiertem C₁-C₆-Alkoxy, optional substituiertem Phenyl, optional substituiertem Phenoxy, C₁-C₆-Alkylsulfanyl, C₁-C₆-Acyl, C₁-C₆-Alkoxycarbonyl, optional substituiertem Heteroaryl und Heterocycloalkyl.

21. Mindestens eine chemische Einheit nach Anspruch 20, wobei die Substituenten auf dem substituiertem Phenyl unabhängig ausgewählt sind aus Hydroxy, Cyano, Halogen, optional substituiertem C₁-C₂-Alkyl, Phenoxy und optional substituiertem C₁-C₂-Alkoxy.

22. Mindestens eine chemische Einheit nach Anspruch 21, wobei die Substituenten auf dem substituiertem Phenyl unabhängig ausgewählt sind aus Halogen, Methyl, Ethyl, Methoxy, Ethoxy, Difluormethyl, Trifluormethyl, Difluormethoxy und Trifluormethoxy.

23. Mindestens eine chemische Einheit nach Anspruch 16, wobei die Verbindungen von Formel 4 ausgewählt sind aus Verbindungen von Formel 5 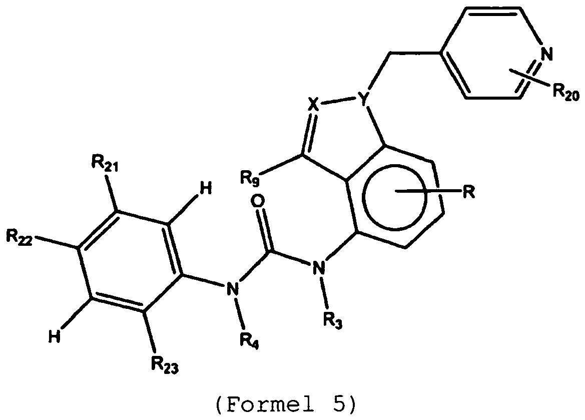 wobei
R₂₁ ausgewählt ist aus Wasserstoff, Halogen und
optional substituiertem Niederalkyl;
R₂₂ ausgewählt ist aus Wasserstoff, Halogen, Niederalkoxy und Niederalkyl; und
R₂₃ ausgewählt ist aus Wasserstoff, Niederalkyl, optional substituiertem Phenoxy, optional substituiertem Niederalkoxy und Halogen.

24. Mindestens eine chemische Einheit nach Anspruch 23, wobei R₂₁ ausgewählt ist aus Wasserstoff, Halogen, Methyl und Trifluormethyl.

25. Mindestens eine chemische Einheit nach Anspruch 23 oder 24, wobei R₂₂ ausgewählt ist aus Wasserstoff, Halogen, Methoxy und Methyl.

26. Mindestens eine chemische Einheit nach Anspruch 25, wobei R₂₂ Wasserstoff ist.

27. Mindestens eine chemische Einheit nach einem der Ansprüche 23 bis 25, wobei R₂₃ ausgewählt ist aus Wasserstoff, Methyl, Methoxy, Difluormethoxy, Trifluormethoxy, Ethoxy und Halogen.

28. Mindestens eine chemische Einheit nach einem der Ansprüche 15 bis 27, wobei R₂₀ optional substituiertes Amino ist.

29. Mindestens eine chemische Einheit nach einem der Ansprüche 5 bis 28, wobei R₉ ausgewählt ist aus Wasserstoff und optional substituiertem Niederalkyl.

30. Mindestens eine chemische Einheit nach Anspruch 29, wobei R₉ ausgewählt ist aus Wasserstoff und Niederalkyl.

31. Mindestens eine chemische Einheit nach Anspruch 30, wobei R₉ Wasserstoff ist.

32. Mindestens eine chemische Einheit nach einem der Ansprüche 1 bis 31, wobei R 1 oder 2 Substituenten darstellt, die unabhängig ausgewählt sind aus Halogen, C₁-C₂-Alkyl, und C₁-C₂-Alkoxy.

33. Mindestens eine chemische Einheit nach Anspruch 32, wobei R 1 oder 2 Substituenten darstellt, die unabhängig ausgewählt sind aus Halogen, Methyl und Methoxy.

34. Mindestens eine chemische Einheit nach Anspruch 33, wobei R einen Substituenten darstellt, der ausgewählt ist aus Halogen, Methyl und Methoxy.

35. Mindestens eine chemische Einheit nach einem der Ansprüche 1 bis 34, wobei R fehlt.

36. Mindestens eine chemische Einheit nach einem der Ansprüche 1 bis 35, wobei R₃ und R₄ jeweils unabhängig ausgewählt sind aus Wasserstoff und Methyl.

37. Mindestens eine chemische Einheit nach Anspruch 36, wobei R₃ und R₄ Wasserstoff sind.

38. Mindestens eine chemische Einheit nach Anspruch 1, wobei die Verbindung von Formel 1 ausgewählt ist aus
1-(5-Brom-2-methoxy-phenyl)-3-(1-pyridin-4-ylmethyl-1H-indol-4-yl)-harnstoff;
1-(2-Methoxy-5-trifluormethyl-phenyl)-3-(1-pyridin-4-ylmethyl-1H-indol-4-yl)-harnstoff;
1-(2-Methoxy-5-trifluormethyl-phenyl)-3-(1-pyridin-4-ylmethyl-1H-indol-6-yl)-harnstoff;
1-(4-Chlor-2-methoxy-5-trifluormethyl-phenyl)-3-(1-pyridin-4-ylmethyl-1H-indol-4-yl)-harnstoff;
1-(2-Fluor-5-trifluormethyl-phenyl)-3-(1-pyridin-4-ylmethyl-1H-indol-4-yl)-harnstoff;
1-(2,4-Dimethoxy-5-trifluormethyl-phenyl)-3-(1-pyridin-4-ylmethyl-1H-indol-4-yl)-harnstoff;
1-(2,4-Dimethyl-5-trifluormethyl-phenyl)-3-(1-pyridin-4-ylmethyl-1H-indol-4-yl)-harnstoff;
1-(2-Ethoxy-5-trifluormethyl-phenyl)-3-(1-pyridin-4-ylmethyl-1H-indol-4-yl)-harnstoff;
1-(5-Brom-2-methoxy-phenyl)-3-(1-pyridin-4-ylmethyl-1H-indazol-4-yl)-harnstoff;
1-(5-Brom-2-ethoxy-phenyl)-3-(1-pyridin-4-ylmethyl-1H-indol-4-yl)-harnstoff;
1-(5-Brom-2-methoxy-phenyl)-3-(1-pyridin-4-ylmethyl-1H-indol-5-yl)-harnstoff;
1-(4-Methyl-3-trifluormethyl-phenyl)-3-(1-pyridin-4-ylmethyl-1H-indol-4-yl)-harnstoff;
1-(4-Chlor-3-trifluormethyl-phenyl)-3-(1-pyridin-4-ylmethyl-1H-indol-4-yl)-harnstoff;
1-(5-Chlor-2-methoxy-phenyl)-3-(1-pyridin-4-ylmethyl-1H-indol-5-yl)-harnstoff;
1-(2-Methoxy-5-trifluormethyl-phenyl)-3-(1-pyridin-4-ylmethyl-1H-indol-5-yl)-harnstoff;
1-(4-Chlor-3-trifluormethyl-phenyl)-3-(1-pyridin-4-ylmethyl-1H-indol-5-yl)-harnstoff;
1-(2-Fluor-5-trifluormethyl-phenyl)-3-(1-pyridin-4-ylmethyl-1H-indol-5-yl)-harnstoff;
1-(2-Chlor-5-trifluormethyl-phenyl)-3-(1-pyridin-4-ylmethyl-1H-indol-5-yl)-harnstoff;
1-(2-Fluor-5-trifluormethyl-phenyl)-3-(3-methyl-1-pyridin-4-ylmethyl-1H-indol-4-yl)-harnstoff;
1-(2-Methoxy-5-trifluormethyl-phenyl)-3-(3-methyl-1-pyridin-4-ylmethyl-1H-indol-4-yl)-harnstoff;
1-(5-Brom-2-methoxy-phenyl)-3-(3-methyl-1-pyridin-4-ylmethyl-1H-indol-4-yl)-harnstoff;
1-(5-Brom-2-methoxy-phenyl)-3-(1-pyridin-3-ylmethyl-1H-indol-4-yl)-harnstoff;
1-(5-Brom-2-methoxy-phenyl)-3-(1-pyridin-3-ylmethyl-1H-indol-5-yl)-harnstoff;
1-(5-Brom-2-methoxy-phenyl)-3-(5-methyl-1-pyridin-4-ylmethyl-1H-indol-4-yl)-harnstoff;
1-(5-Brom-2-methoxy-phenyl)-3-(7-methyl-1-pyridin-4-ylmethyl-1H-indol-4-yl)-harnstoff;
1-(2-Methoxy-5-trifluormethyl-phenyl)-3-(5-methyl-1-pyridin-4-ylmethyl-1H-indol-4-yl)-harnstoff;
1-(2-Fluor-5-trifluormethyl-phenyl)-3-(5-methyl-1-pyridin-4-ylmethyl-1H-indol-4-yl)-harnstoff;
1-(5-Chlor-2-methoxy-phenyl)-3-(5-methyl-1-pyridin-4-ylmethyl-1H-indol-4-yl)-harnstoff;
1-(4-Methoxy-biphenyl-3-yl)-3-(1-pyridin-4-ylmethyl-1H-indol-4-yl)-harnstoff;
1-(2-Methoxy-5-trifluormethyl-phenyl)-3-(7-methyl-1-pyridin-4-ylmethyl-1H-indol-4-yl)-harnstoff;
1-(2-Fluor-5-trifluormethyl-phenyl)-3-(7-methyl-1-pyridin-4-ylmethyl-1H-indol-4-yl)-harnstoff;
1-(5-Chlor-2-methoxy-phenyl)-3-(7-methyl-1-pyridin-4-ylmethyl-1H-indol-4-yl)-harnstoff;
4-{4-[3-(2-Methoxy-5-trifluormethyl-phenyl)-ureido]-5-methyl-indol-1-ylmethyl}-pyridin-2-carbonsäuremethylamid;
1-(7-Methyl-1-pyridin-4-ylmethyl-1H-indol-4-yl)-3-(4-methyl-3-trifluormethyl-phenyl)-harnstoff;
1-(4-Chlor-3-trifluormethyl-phenyl)-3-(7-methyl-1-pyridin-4-ylmethyl-1H-indol-4-yl)-harnstoff;
1-[5-Chlor-2-([1,3]dioxolan-2-ylmethoxy)-phenyl]-3-(1-pyridin-4-ylmethyl-1H-indol-4-yl)-harnstoff;
1-[5-Chlor-2-(3-hydroxy-propoxy)-phenyl]-3-(5-methyl-1-pyridin-4-ylmethyl-1H-indol-4-yl)-harnstoff;
1-(3-Chlor-4-methoxy-phenyl)-3-(1-pyridin-4-ylmethyl-1H-indol-4-yl)-harnstoff;
1-(4-Methoxy-3-trifluormethyl-phenyl)-3-(1-pyridin-4-ylmethyl-1H-indol-4-yl)-harnstoff;
1-(2-Methoxy-4-methyl-5-trifluormethyl-phenyl)-3-(1-pyridin-4-ylmethyl-1H-indol-4-yl)-harnstoff;
1-(3-Brom-4-methyl-phenyl)-3-(1-pyridin-4-ylmethyl-1H-indol-4-yl)-harnstoff;
1-(4-Fluor-3-trifluormethyl-phenyl)-3-(1-pyridin-4-ylmethyl-1H-indol-4-yl)-harnstoff;
1-(5-Brom-2-methoxy-4-methyl-phenyl)-3-(1-pyridin-4-ylmethyl-1H-indol-4-yl)-harnstoff;
1-(5-Brom-2-methoxy-phenyl)-3-[1-(2-fluor-pyridin-4-ylmethyl)-1H-indol-4-yl]-harnstoff;
1-(5-Brom-2-methoxy-phenyl)-3-[1-(2-methyl-pyridin-4-ylmethyl)-1H-indol-4-yl]-harnstoff;
1-(3-Brom-4-methoxy-phenyl)-3-(1-pyridin-4-ylmethyl-1H-indol-4-yl)-harnstoff;
1-(5-Brom-2-methoxy-phenyl)-3-[1-(3-fluor-pyridin-4-ylmethyl)-1H-indol-4-yl]-harnstoff;
1-[1-(3-Fluor-pyridin-4-ylmethyl)-1H-indol-4-yl]-3-(4-methyl-3-trifluormethyl-phenyl)-harnstoff;
1-(3-Chlor-4-fluor-phenyl)-3-(1-pyridin-4-ylmethyl-1H-indol-4-yl)-harnstoff;
1-[1-(2-Amino-pyridin-4-ylmethyl)-1H-indol-4-yl]-3-(5-brom-2-methoxy-phenyl)-harnstoff;
1-(3,4-Dimethoxy-phenyl)-3-(1-pyridin-4-ylmethyl-1H-indol-4-yl)-harnstoff;
1-(3-Chlor-4-methyl-phenyl)-3-(1-pyridin-4-ylmethyl-1H-indol-4-yl)-harnstoff;
1-(5-Chlor-2-methoxy-4-methyl-phenyl)-3-(1-pyridin-4-ylmethyl-1H-indol-4-yl)-harnstoff;
1-[1-(2-Amino-pyridin-4-ylmethyl)-1H-indol-4-yl]-3-(5-brom-2-methoxy-4-methyl-phenyl)-harnstoff;
1-[1-(2-Amino-pyridin-4-ylmethyl)-1H-indol-4-yl]-3-(3-chlor-4-methyl-phenyl)-harnstoff;
1-[1-(2-Amino-pyridin-4-ylmethyl)-1H-indol-4-yl]-3-(5-chlor-2-methoxy-phenyl)-harnstoff;
1-[1-(2-Amino-pyridin-4-ylmethyl)-1H-indol-4-yl]-3-(5-chlor-2,4-dimethoxy-phenyl)-harnstoff;
1-[1-(2-Amino-pyridin-4-ylmethyl)-1H-indol-4-yl]-3-(5-chlor-2-methoxy-4-methyl-phenyl)-harnstoff;
1-[1-(2-Amino-pyridin-4-ylmethyl)-1H-indol-4-yl]-3-(2,4-dimethyl-5-trifluormethyl-phenyl)-harnstoff;
1-(5-Brom-2-methoxy-phenyl)-3-(5-methoxy-1-pyridin-4-ylmethyl-1H-indol-4-yl)-harnstoff;
1-[1-(2-Amino-pyridin-4-ylmethyl)-1H-indol-4-yl]-3-(2-methoxy-5-trifluormethyl-phenyl)-harnstoff;
1-[1-(2-Amino-pyridin-4-ylmethyl)-1H-indol-4-yl]-3-(2,4-dimethoxy-5-trifluormethyl-phenyl)-harnstoff;
1-[1-(2-Amino-pyridin-4-ylmethyl)-1H-indol-4-yl]-3-(2-methoxy-4-methyl-5-trifluormethyl-phenyl)-harnstoff;
1-Benzo[1,3]dioxol-5-yl-3-(1-pyridin-4-ylmethyl-1H-indol-4-yl)-harnstoff;
1-(5-Brom-2-methoxy-phenyl)-3-(6-methyl-1-pyridin-4-ylmethyl-1H-indol-4-yl)-harnstoff;
(4-{4-[3-(5-Brom-2-methoxy-phenyl)-ureido]-indol-1-ylmethyl}-pyridin-2-yl)-carbaminsäuremethylester;
1-(5-Brom-2-methoxy-phenyl)-3-[1-(3-chlor-pyridin-4-ylmethyl)-1H-indol-4-yl]-harnstoff;
1-(5-Fluor-2,4-dimethoxy-phenyl)-3-(1-pyridin-4-ylmethyl-1H-indol-4-yl)-harnstoff;
1-(3-Brom-4-fluor-phenyl)-3-(1-pyridin-4-ylmethyl-1H-indol-4-yl)-harnstoff;
N-(4-{4-[3-(5-Brom-2-methoxy-phenyl)-ureido]-indol-1-ylmethyl}-pyridin-2-yl)-acetamid;
1-(5-Ethansulfonyl-2-methoxy-phenyl)-3-(1-pyridin-4-ylmethyl-1H-indol-4-yl)-harnstoff;
1-(5-Brom-2,4-difluor-phenyl)-3-(1-pyridin-4-ylmethyl-1H-indol-4-yl)-harnstoff;
1-[1-(2-Amino-pyridin-4-ylmethyl)-1H-indol-4-yl]-3-(2-fluor-5-trifluormethyl-phenyl)-harnstoff;
N-(4-{4-[3-(5-Chlor-2-methoxy-4-methyl-phenyl)-ureido]-indol-1-ylmethyl}-pyridin-2-yl)-acetamid;
1-(5-Brom-2-methoxy-phenyl)-3-(7-fluor-1-pyridin-4-ylmethyl-1H-indol-4-yl)-harnstoff;
1-(5-Chlor-2-methoxy-4-methyl-phenyl)-3-(7-fluor-1-pyridin-4-ylmethyl-1H-indol-4-yl)-harnstoff;
N-(4-{4-[3-(5-Brom-2-methoxy-phenyl)-ureido]-indol-1-ylmethyl}-pyridin-2-yl)-2-hydroxy-acetamid;
2-Amino-N-(4-{4-[3-(5-brom-2-methoxy-phenyl)-ureido]-indol-1-ylmethyl}-pyridin-2-yl)-acetamid;
N-(4-{4-[3-(5-Brom-2-methoxy-phenyl)-ureido]-indol-1-ylmethyl}-pyridin-2-yl)-formamid;
1-[1-(2-Amino-pyridin-4-ylmethyl)-1H-indol-4-yl]-3-(3-brom-4-methoxy-phenyl)-harnstoff;
N-(4-{4-[3-(3-Chlor-4-methyl-phenyl)-ureido]-indol-1-ylmethyl}-pyridin-2-yl)-acetamid;
1-(5-Brom-2-methoxy-phenyl)-3-(7-methoxy-1-pyridin-4-ylmethyl-1H-indol-4-yl)-harnstoff;
1-(2,4-Dimethoxy-5-trifluormethyl-phenyl)-3-(7-methoxy-1-pyridin-4-ylmethyl-1H-indol-4-yl)-harnstoff;
1-(5-Brom-2-methoxy-phenyl)-3-(5-fluor-1-pyridin-4-ylmethyl-1H-indol-4-yl)-harnstoff;
N-(4-{4-[3-(5-Chlor-2-methoxy-phenyl)-ureido]-indol-1-ylmethyl}-pyridin-2-yl)-acetamid;
1-Methyl-1H-imidazol-2-carbonsäure(4-{4-[3-(5-brom-2-methoxy-phenyl)-ureido]-indol-1-ylmethyl}-pyridin-2-yl)-amid;
N-(4-{4-[3-(5-Brom-2-methoxy-phenyl)-ureido]-indol-1-ylmethyl}-pyridin-2-yl)-propionamid;
N-(4-{4-[3-(5-Brom-2-methoxy-phenyl)-ureido]-indol-1-ylmethyl}-pyridin-2-yl)-isobutyramid;
N-(4-{4-[3-(5-Brom-2-methoxy-phenyl)-ureido]-indol-1-ylmethyl}-pyridin-2-yl)-2-morpholin-4-yl-acetamid;
1-(5-Brom-2-methoxy-phenyl)-3-[1-(2-methylaminopyridin-4-ylmethyl)-1H-indol-4-yl]-harnstoff;
1-(5-Brom-2-methoxy-phenyl)-3-{1-[2-(2-methoxy-ethylamino)-pyridin-4-ylmethyl]-1H-indol-4-yl}-harnstoff;
1-(5-Brom-2-methoxy-phenyl)-3-[1-(2-morpholin-4-yl-pyridin-4-ylmethyl)-1H-indol-4-yl]-harnstoff;
1-(5-Brom-2-methoxy-phenyl)-3-[1-(2-methoxy-pyridin-4-ylmethyl)-1H-indol-4-yl]-harnstoff;
1-(5-Brom-2-methoxy-phenyl)-3-[1-(2-hydroxy-pyridin-4-ylmethyl)-1H-indol-4-yl]-harnstoff;
N-(4-{4-[3-(2-Fluor-5-trifluormethyl-phenyl)-ureido]-indol-1-ylmethyl}-pyridin-2-yl)-acetamid;
N-(4-{4-[3-(5-Chlor-2-methoxy-phenyl)-ureido]-indol-1-ylmethyl}-pyridin-2-yl)-2-morpholin-4-yl-acetamid;
N-(4-{4-[3-(5-Brom-2-methoxy-phenyl)-ureido]-indol-1-ylmethyl}-pyridin-2-yl)-2-methoxy-acetamid;
1-(5-Chlor-2-methoxy-phenyl)-3-[1-(3-methyl-pyridin-4-ylmethyl)-1H-indol-4-yl]-harnstoff;
Cyclopropancarbonsäure(4-{4-[3-(5-chlor-2-methoxyphenyl)-ureido]-indol-1-ylmethyl}-pyridin-2-yl)-amid;
1-[1-(2-Amino-pyridin-4-ylmethyl)-1H-indol-4-yl]-3-(5-brom-2-difluormethoxy-phenyl)-harnstoff;
N-(4-{4-[3-(5-Brom-2-difluormethoxy-phenyl)-ureido]-indol-1-ylmethyl}-pyridin-2-yl)-acetamid;
N-(4-{4-[3-(5-Chlor-2-methoxy-phenyl)-ureido]-indol-1-ylmethyl}-pyridin-2-yl)-3-diethylamino-propionamid;
1-(5-Brom-2-methoxy-phenyl)-3-(7-chlor-1-pyridin-4-ylmethyl-1H-indol-4-yl)-harnstoff;
1-(5-Brom-2-methoxy-phenyl)-3-{1-[2-(2-hydroxy-ethylamino)-pyridin-4-ylmethyl]-1H-indol-4-yl}-harnstoff;
1-(5-Brom-2-methoxy-phenyl)-3-(5-chlor-1-pyridin-4-ylmethyl-1H-indol-4-yl)-harnstoff;
N-(4-{4-[3-(5-Brom-2-methoxy-phenyl)-ureido]-indol-1-ylmethyl}-pyridin-2-yl)-2-pyrrolidin-1-yl-acetamid;
N-(4-{4-[3-(5-Brom-2-methoxy-phenyl)-ureido]-indol-1-ylmethyl}-pyridin-2-yl)-2-dimethylamino-acetamid;
N-(4-{4-[3-(5-Brom-2-methoxy-phenyl)-ureido]-indol-1-ylmethyl}-pyridin-2-yl)-2-methylamino-acetamid;
1-[1-(2-Amino-pyridin-4-ylmethyl)-5-fluor-1H-indol-4-yl]-3-(5-brom-2-methoxy-phenyl)-harnstoff; Cyclopropancarbonsäure(4-{4-[3-(5-brom-2-methoxyphenyl)-ureido]-indol-1-ylmethyl}-pyridin-2-yl)-amid;
Cyclopropancarbonsäure(4-{4-[3-(2-methoxy-5-trifluormethyl-phenyl)-ureido]-indol-1-ylmethyl}-pyridin-2-yl)-amid;
Cyclopropancarbonsäure(4-{4-[3-(2-fluor-5-trifluormethyl-phenyl)-ureido]-indol-1-ylmethyl}-pyridin-2-yl)-amid;
1-[1-(2-Amino-pyridin-4-ylmethyl)-5-fluor-1H-indol-4-yl]-3-(2-fluor-5-trifluoromethyl-phenyl)-harnstoff;
1-[1-(2-Amino-pyridin-4-ylmethyl)-5-fluor-1H-indol-4-yl]-3-(2-methoxy-5-trifluormethyl-phenyl)-harnstoff;
Cyclopropancarbonsäure(4-{4-[3-(3-chlor-4-methylphenyl)-ureido]-indol-1-ylmethyl}-pyridin-2-yl)-amid;
N-(4-{4-[3-(5-Brom-2-methoxy-phenyl)-ureido]-indol-1-ylmethyl}-pyridin-2-yl)-methansulfonamid;
N-(4-{4-[3-(5-Chlor-2-methoxy-phenyl)-ureido]-indol-1-ylmethyl}-pyridin-2-yl)-methansulfonamid;
1-[1-(2-Amino-pyridin-4-ylmethyl)-5-fluor-1H-indol-4-yl]-3-(2,5-dichlor-phenyl)-harnstoff;
1-[1-(2-Amino-pyridin-4-ylmethyl)-5-fluor-1H-indol-4-yl]-3-(5-chlor-2-methoxy-phenyl)-harnstoff;
1-(5-Chlor-2-methoxy-phenyl)-3-{1-[2-(pyrazin-2-ylamino)-pyridin-4-ylmethyl]-1H-indol-4-yl}-harnstoff;
1-(5-Chlor-2-methoxy-phenyl)-3-{1-[2-(thiazol-2-ylamino)-pyridin-4-ylmethyl]-1H-indol-4-yl}-harnstoff;
1-[1-(2-Amino-pyridin-4-ylmethyl)-7-fluor-1H-indol-4-yl]-3-(5-brom-2-methoxy-phenyl)-harnstoff; N-(4-{4-[3-(5-Chlor-2-methoxy-phenyl)-ureido]-indol-1-ylmethyl}-pyridin-2-yl)-2-methylamino-acetamid;
N-(4-{4-[3-(5-Brom-2-methoxy-phenyl)-ureido]-indol-1-ylmethyl}-pyridin-2-yl)-3-diethylamino-propionamid;
1-[1-(2-Amino-pyridin-4-ylmethyl)-7-fluor-1H-indol-4-yl]-3-(2-fluor-5-trifluormethyl-phenyl)-harnstoff;
1-(2-Fluor-5-trifluormethyl-phenyl)-3-{1-[2-(pyridin-2-ylamino)-pyridin-4-ylmethyl]-1H-indol-4-yl}-harnstoff;
1-(5-Brom-2-methoxy-phenyl)-3-{1-[2-(3-isopropyl-ureido)-pyridin-4-ylmethyl]-1H-indol-4-yl}-harnstoff;
3-(Acetyl-methyl-amino)-N-(4-{4-[3-(5-chlor-2-methoxy-phenyl)-ureido]-indol-1-ylmethyl}-pyridin-2-yl)-propionamid;
N-(4-{4-[3-(2-Fluor-5-trifluormethyl-phenyl)-ureido]-indol-1-ylmethyl}-pyridin-2-yl)-2-methylamino-acetamid;
1-[1-(2-Allylamino-pyridin-4-ylmethyl)-1H-indol-4-yl]-3-(5-brom-2-methoxy-phenyl)-harnstoff;
3-Amino-N-(4-{4-[3-(5-chlor-2-methoxy-phenyl)-ureido]-indol-1-ylmethyl}-pyridin-2-yl)-propionamid;
1-(2-Fluor-5-trifluormethyl-phenyl)-3-{1-[2-(pyridin-3-ylamino)-pyridin-4-ylmethyl]-1H-indol-4-yl}-harnstoff;
1-[1-(2-Amino-5-fluor-pyridin-4-ylmethyl)-1H-indol-4-yl]-3-(5-brom-2-methoxy-phenyl)-harnstoff;
1-[1-(2-Amino-pyridin-4-ylmethyl)-5-chlor-1H-indol-4-yl]-3-(5-brom-2-methoxy-phenyl)-harnstoff;
1-[1-(2-Amino-3-methyl-pyridin-4-ylmethyl)-1H-indol-4-yl]-3-(5-brom-2-methoxy-phenyl)-harnstoff;
1-(2-Fluor-5-trifluormethyl-phenyl)-3-{1-[2-(1H-tetrazol-5-yl)-pyridin-4-ylmethyl]-1H-indol-4-yl}-harnstoff;
1-(2-Fluor-5-trifluormethyl-phenyl)-3-{1-[2-(pyrimidin-2-ylamino)-pyridin-4-ylmethyl]-1H-indol-4-yl}-harnstoff;
1-[1-(2-Brom-pyridin-4-ylmethyl)-1H-indol-4-yl]-3-(5-chlor-2-methoxy-phenyl)-harnstoff;
1-[1-(2-Brom-pyridin-4-ylmethyl)-1H-indol-4-yl]-3-(2-fluor-5-trifluormethyl-phenyl)-harnstoff;
1-[1-(2-Cyano-pyridin-4-ylmethyl)-1H-indol-4-yl]-3-(2-fluor-5-trifluormethyl-phenyl)-harnstoff;
1-(5-Chlor-2-methoxy-phenyl)-3-[1-(2-cyano-pyridin-4-ylmethyl)-1H-indol-4-yl]-harnstoff;
1-(2-Fluor-5-trifluormethyl-phenyl)-3-{1-[2-(1H-imidazol-2-yl)-pyridin-4-ylmethyl]-1H-indol-4-yl}-harnstoff;
1-(5-Chlor-2-methoxy-phenyl)-3-{1-[2-(4-cyano-phenylamino)-pyridin-4-ylmethyl]-1H-indol-4-yl}-harnstoff; und
1-(5-Chlor-2-methoxy-phenyl)-3-{1-[2-(6-cyano-pyridin-3-ylamino)-pyridin-4-ylmethyl]-1H-indol-4-yl}-harnstoff.

39. Mindestens eine chemische Einheit nach Anspruch 1, wobei die chemische Einheit 1-[1-(2-Amino-pyridin-4-ylmethyl)-1H-indol-4-yl]-3-(5-brom-2-methoxyphenyl)-harnstoff oder ein pharmazeutisch annehmbares Salz, Solvat oder eine Kristallform davon ist.

40. Pharmazeutische Zusammensetzung, umfassend mindestens eine chemische Einheit nach einem der Ansprüche 1 bis 39 gemeinsam mit mindestens einem pharmazeutisch annehmbaren Vehikel, ausgewählt aus Trägern, Adjuvantien und Hilfsstoffen.

41. Pharmazeutische Zusammensetzung nach Anspruch 40, wobei die Zusammensetzung in einer Form formuliert ist, die ausgewählt ist aus injizierbaren Fluida, Aerosolen, Cremes, Gels, Tabletten, Pillen, Kapseln, Sirups, ophthalmischen Lösungen und transdermalen Patches.

42. Verpackte pharmazeutische Zusammensetzung, umfassend die pharmazeutische Zusammensetzung nach Anspruch 40 oder 41 in einem Behälter; und Gebrauchsanweisungen für die Zusammensetzung zur Behandlung eines Patienten, der an einer Krankheit oder Störung leidet, die auf eine Modulierung der Kinaseaktivität einer oder mehrerer Tyrosinkinase(n) anspricht.

43. Verwendung mindestens einer chemischen Einheit für die Herstellung eines Medikaments für die Behandlung eines Patienten mit einer Krankheit, die auf eine Hemmung mindestens einer Kinase anspricht, die ausgewählt ist aus VEGFR2, EphB₄, PDGFRβ und c-Kit, wobei die mindestens eine chemische Einheit eine chemische Einheit nach einem der Ansprüche 1 bis 39 ist.

44. Verwendung nach Anspruch 43, wobei die Krankheit, die auf eine Hemmung mindestens einer Kinase anspricht, auf eine Hemmung der VEGFR2 Kinaseaktivität anspricht.

45. Verwendung nach Anspruch 44, wobei die Krankheit, die auf eine Hemmung der VEGFR2 Kinaseaktivität anspricht, ausgewählt ist aus Krebs und Krankheiten, die durch eine Änderung in der Angiogenese charakterisiert sind.

46. Verwendung nach Anspruch 45, wobei die Krankheiten, die durch eine Änderung in der Angiogenese charakterisiert sind, ausgewählt sind aus kanzerösem Tumor, Makuladegeneration und diabetischer Retinopathie.

47. Verfahren zur Herstellung eines Medikaments für die Behandlung eines Patienten mit einer Krankheit, die auf eine Hemmung mindestens einer Kinase anspricht, die ausgewählt ist aus VEGFR2, EphB₄, PDGFRβ und c-Kit, umfassend das Einschließen mindestens einer chemischen Einheit nach einem der Ansprüche 1 bis 39 in dem Medikament.

48. Verfahren nach Anspruch 47, wobei die Krankheit, die auf eine Hemmung mindestens einer Kinase anspricht, auf eine Hemmung der VEGFR2 Kinaseaktivität anspricht.

49. Verfahren nach Anspruch 48, wobei die Krankheit, die auf eine Hemmung der VEGFR2 Kinaseaktivität anspricht, ausgewählt ist aus Krebs und Krankheiten, die durch eine Änderung in der Angiogenese charakterisiert sind.

50. Verfahren nach Anspruch 49, wobei die Krankheiten, die durch eine Änderung in der Angiogenese charakterisiert sind, ausgewählt sind aus kanzerösem Tumor, Makuladegeneration und diabetischer Retinopathie.

51. Verwendung mindestens einer chemischen Einheit für die Herstellung eines Medikaments für die Behandlung einer Patientin mit einer weiblichen Fortpflanzungsstörung oder -erkrankung, wobei die mindestens eine chemische Einheit eine chemische Einheit nach einem der Ansprüche 1 bis 39 ist.

52. Verwendung nach Anspruch 51, wobei die weibliche Fortpflanzungsstörung oder -erkrankung ausgewählt ist aus Endometriose, endometrialem Karzinoma, gynäkologischen Blutungsstörungen, unregelmäßigen Menstruationszyklen, Ovulation, prämenstruellem Syndrom (PMS) und menopausaler Dysfunktion.

53. Chemische Einheit nach einem der Ansprüche 1 bis 39 zur Verwendung in der Behandlung eines Patienten mit (i) einer Krankheit, die auf eine Hemmung mindestens einer Kinase anspricht, die ausgewählt ist aus VEGFR2, EphB₄, PDGFRβ und c-Kit, oder (ii) einer weiblichen Fortpflanzungsstörung oder -erkrankung.

## Revendications

1. Au moins une entité chimique choisie parmi les composés de Formule 1 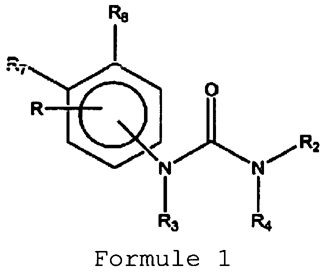 et les sels pharmaceutiquement acceptables, les solvats, les formes cristallines, et les mélanges de ceux-ci, où
R représente de 0 à 2 substituants choisis indépendamment parmi hydroxy, nitro, cyano, amino éventuellement substitué, aminocarbonyle, halogéno, carboxy, acyle éventuellement substitué, alcoxycarbonyle éventuellement substitué, C₁-C₆ alkyle éventuellement substitué, C₁-C₆ alcoxy éventuellement substitué, sulfanyle, sulfinyle, sulfonyle, aryle éventuellement substitué, hétéroaryle éventuellement substitué, et hétérocycloalkyle éventuellement substitué ;
R₇ et R₈, pris ensemble avec les carbones auxquels ils sont liés, forment un cycle hétéroaryle de 5 à 7 chaînons condensé substitué par un groupement -(Z₁)ₘR₁, où le cycle hétéroaryle de 5 à 7 chaînons condensé est éventuellement substitué davantage et où
R₁ est hétéroaryle éventuellement substitué ;
Z₁ est -CR₅R₆- où chaque R₅ et R₆ est choisi indépendamment parmi hydrogène, C₁-C₆ alkyle éventuellement substitué, et halogéno ; et
m est choisi parmi 0, 1, et 2 ;
R₂ est aryle éventuellement substitué ; et
R₃ et R₄ sont choisis chacun indépendamment parmi hydrogène, C₁-C₆ alkyle éventuellement substitué, aryle éventuellement substitué, et hétéroaryle éventuellement substitué ;
où
les termes alkyle, cycloalkyle, aryle, hétérocycloalkyle, et hétéroaryle « substitué » désignent respectivement alkyle, cycloalkyle, aryle, hétérocycloalkyle, et hétéroaryle où un ou plusieurs atomes d'hydrogène sont remplacés par un substituant choisi indépendamment parmi :
-R^{a}, -OR^{b}, -SR^{b}, guanidine, guanidine où un ou plusieurs parmi les hydrogènes de guanidine sont remplacés par un groupement alkyle inférieur, -NR^{b}R^{c}, halogéno, cyano, nitro, oxo (comme substituant pour cycloalkyle, hétérocycloalkyle, et hétéroaryle), -COR^{b}, -CO₂R^{b}, -CONR^{b}R^{c}, -OCOR^{b}, -OCO₂R^{a}, -OCONR^{b}R^{c}, -NR^{c}COR^{b}, -NR^{c}CO₂R^{a}, -NR^{c}CONR^{b}R^{c}, -CO₂R^{b}, -CONR^{b}R^{c}, -NR^{c}COR^{b}, -SOR^{a}, -SO₂R^{a}, -SO₂NR^{b}R^{c}, et -NR^{c}SO₂R^{a},
où R^{a} est choisi parmi C₁-C₆ alkyle éventuellement substitué, alcényle éventuellement substitué, alcynyle éventuellement substitué, aryle éventuellement substitué, et hétéroaryle éventuellement substitué ;
R^{b} est choisi parmi H, C₁-C₆ alkyle éventuellement substitué, cycloalkyle éventuellement substitué, hétérocycloalkyle éventuellement substitué, aryle éventuellement substitué, et hétéroaryle éventuellement substitué ; et
R^{c} est choisi indépendamment parmi hydrogène et C₁-C₄ alkyle éventuellement substitué ; ou
R^{b} et R^{c}, et l'azote auquel ils sont fixés, forment un groupement hétérocycloalkyle éventuellement substitué ; et où chaque groupement éventuellement substitué est non substitué ou substitué indépendamment par un ou plusieurs, tel qu'un, deux ou trois, substituants choisis indépendamment parmi C₁-C₄ alkyle, aryle, hétéroaryle, aryl-C₁-C₄ alkyl-, hétéroaryl-C₁-C₄ alkyl-, C₁-C₄ halogénoalkyl-, -OC₁-C₄ alkyle, -OC₁-C₄ alkylphényle, -C₁-C₄ alkyl-OH, -OC₁-C₄ halogénoalkyle, halogéno, -OH, -NH₂, -C₁-C₄ alkyl-NH₂, -N(C₁-C₄ alkyl) (C₁-C₄ alkyl), -NH(C₁-C₄ alkyl), -N(C₁-C₄ alkyl) (C₁-C₄ alkylphényl), -NH(C₁-C₄ alkylphényl), cyano, nitro, oxo (comme substituant pour cycloalkyle, hétérocycloalkyle, ou hétéroaryle), -CO₂H, -C(O)OC₁-C₄ alkyle, -CON(C₁-C₄ alkyl) (C₁-C₄ alkyl), -CONH (C₁-C₄ alkyl), -CONH₂, -NHC(O)(C₁-C₄ alkyl), -NHC(O)(phényl), -N(C₁-C₄ alkyl) C(O)(C₁-C₄ alkyl), -N(C₁-C₄ alkyl) C(O)(phényl), -C(O)C₁-C₄ alkyle, -C(O)C₁-C₄ alkylphényle, -C(O)C₁-C₄ halogénoalkyle, -OC(O)C₁-C₄ alkyle, -SO₂(C₁-C₄ alkyl), -SO₂(phényl), -SO₂(C₁-C₄ halogénoalkyl), -SO₂NH₂, -SO₂NH(C₁-C₄ alkyl), -SO₂NH(phényl), -NHSO₂(C₁-C₄ alkyl), -NHSO₂(phényl), et -NHSO₂(C₁-C₄ halogénoalkyl) ;
par le terme « acyle substitué » on entend les groupements (alkyle substitué)-C(O)- ; (cycloalkyle substitué)-C(O)- ; (aryle substitué)-C(O)- ; (hétéroaryle substitué)-C(O)- ; et (hétérocycloalkyle substitué)-C(O)-, où le groupement est fixé à la structure parente par l'intermédiaire de la fonctionnalité carbonyle et où alkyle, cycloalkyle, aryle, hétéroaryle, et hétérocycloalkyle substitué, désignent respectivement alkyle, cycloalkyle, aryle, hétéroaryle, et hétérocycloalkyle où un ou plusieurs atomes d'hydrogène sont remplacés par un substituant choisi indépendamment parmi :
-R^{a}, -OR^{b}, -SR^{b}, guanidine, guanidine où un ou plusieurs parmi les hydrogènes de guanidine sont remplacés par un groupement alkyle inférieur, -NR^{b}R^{c}, halogéno, cyano, nitro, -COR^{b}, -CO₂R^{b}, -CONR^{b}R^{c}, -OCOR^{b}, -OCO₂R^{a}, -OCONR^{b}R^{c}, -NR^{c}COR^{b}, -NR^{c}CO₂R^{a}, -NR^{c}CONR^{b}R^{c}, -CO₂R^{b}, -CONR^{b}R^{c}, -NR^{c}COR^{b}, -SOR^{a}, -SO₂R^{a}, -SO₂NR^{b}R^{c}, et -NR^{c}SO₂R^{a} ;
par le terme « alcoxy substitué » on entend alcoxy où le constituant alkyle est substitué (c'est-à-dire -0-(alkyle substitué)) où par « alkyle substitué » on entend alkyle où un ou plusieurs atomes d'hydrogène sont remplacés par un substituant choisi indépendamment parmi :
-R^{a}, -OR^{b}, -SR^{b}, guanidine, guanidine où un ou plusieurs parmi les hydrogènes de guanidine sont remplacés par un groupement alkyle inférieur, -NR^{b}R^{c}, halogéno, cyano, nitro, -COR^{b}, -CO₂R^{b}, -CONR^{b}R^{c}, -OCOR^{b}, -OCO₂R^{a}, -OCONR^{b}R^{c}, -NR^{c}COR^{b}, -NR^{c}CO₂R^{a}, -NR^{c}CONR^{b}R^{c}, -CO₂R^{b}, -CONR^{b}R^{c}, -NR^{c}COR^{b}, -SOR^{a}, -SO₂R^{a}, -SO₂NR^{b}R^{c}, et -NR^{c}SO₂R^{a} ;
un groupement alcoxy substitué peut être « polyalcoxy » ou -O-(alkylène éventuellement substitué)-(alcoxy éventuellement substitué), ou hydroxyalcoxy ou -OCH₂(CH₂)yOH, où y est un nombre entier allant de 1-10, tel que 1-4 ;
par le terme « alcoxycarbonyle substitué » on entend le groupement (alkyle substitué)-O-C(O)- où le groupement est fixé à la structure parente par l'intermédiaire de la fonctionnalité carbonyle et où « substitué » désigne alkyle où un ou plusieurs atomes d'hydrogène sont remplacés par un substituant choisi indépendamment parmi :
-R^{a}, -OR^{b}, -SR^{b}, guanidine, guanidine où un ou plusieurs parmi les hydrogènes de guanidine sont remplacés par un groupement alkyle inférieur, -NR^{b}R^{c}, halogéno, cyano, nitro, -COR^{b}, -CO₂R^{b}, -CONR^{b}R^{c}, -OCOR^{b}, -OCO₂R^{a}, -OCONR^{b}R^{c}, -NR^{c}COR^{b}, -NR^{c}CO₂R^{a}, -NR^{c}CONR^{b}R^{c}, -CO₂R^{b}, -CONR^{b}R^{c}, -NR^{c}COR^{b}, -SOR^{a}, -SO₂R^{a}, -SO₂NR^{b}R^{c}, et -NR^{c}SO₂R^{a} ;
par le terme « amino substitué » on entend le groupement -NHR^{d} ou -NR^{d}R^{e} où R^{d} est choisi parmi : hydroxy, alcoxy éventuellement substitué, alkyle éventuellement substitué, cycloalkyle éventuellement substitué, acyle éventuellement substitué, aminocarbonyle, aryle éventuellement substitué, hétéroaryle éventuellement substitué, hétérocycloalkyle éventuellement substitué, alcoxycarbonyle éventuellement substitué, sulfinyle et sulfonyle, et où R^{e} est choisi parmi : alkyle éventuellement substitué, cycloalkyle éventuellement substitué, aryle éventuellement substitué, hétéroaryle éventuellement substitué, et hétérocycloalkyle éventuellement substitué, et où alkyle, cycloalkyle, aryle, hétérocycloalkyle, et hétéroaryle substitué désignent respectivement alkyle, cycloalkyle, aryle, hétérocycloalkyle, et hétéroaryle où un ou plusieurs atomes d'hydrogène sont remplacés par un substituant choisi indépendamment parmi :
-R^{a}, -OR^{b}, -SR^{b}, guanidine, guanidine où un ou plusieurs parmi les hydrogènes de guanidine sont remplacés par un groupement alkyle inférieur, -NR^{b}R^{c}, halogéno, cyano, nitro, -COR^{b}, -CO₂R^{b}, -CONR^{b}R^{c}, -OCOR^{b}, -OCO₂R^{a}, -OCONR^{b}R^{c}, -NR^{c}COR^{b}, -NR^{c}CO₂R^{a}, -NR^{c}CONR^{b}R^{c}, -CO₂R^{b}, -CONR^{b}R^{c}, -NR^{c}COR^{b}, -SOR^{a}, -SO₂R^{a}, -SO₂NR^{b}R^{c}, et -NR^{c}SO₂R^{a} ;
par le terme « amino substitué » on entend également les N-oxydes des groupements -NHR^{d}, et NR^{d}R^{d} ;
à condition que le composé de Formule 1 ne soit pas choisi parmi le 5-(phénylcarbamoylamino)-3-(2-(4-pyridyl)éthyl)indole ; la 1-(4-chloro-3-(trifluoro-méthyl)phényl)-3-(1-(pyridin-4-yl)-1H-indol-5-yl)urée ; et la 1-(2-méthoxy-5-(trifluorométhyl)phényl)-3-(1-(pyridin-4-yl)-1H-indol-5-yl)urée.

2. Au moins une entité chimique selon la revendication 1, dans laquelle R₇ et R₈, pris ensemble avec les carbones auxquels ils sont liés, forment un cycle hétéroaryle de 5 chaînons condensé substitué par un groupement -(Z₁)ₘR₁ où le cycle hétéroaryle contient au moins un azote et comporte éventuellement un ou plusieurs hétéroatomes supplémentaires choisis parmi N, O, et S dans le cycle.

3. Au moins une entité chimique selon la revendication 2, dans laquelle R₇ et R₈, pris ensemble avec les carbones auxquels ils sont liés, forment un cycle condensé choisi parmi pyrazolyle, imidazolyle, isoxazolyle, oxazolyle, thiazolyle, triazolyle, et pyrrolyle, chacun d'entre eux étant substitué par un groupement - (Z₁)ₘR₁.

4. Au moins une entité chimique selon la revendication 3, dans laquelle R₇ et R₈, pris ensemble avec les carbones auxquels ils sont liés, forment un cycle hétéroaryle condensé choisi parmi 1H-pyrrolyle et 1H-pyrazolyle, chacun d'entre eux étant substitué par un groupement - (Z₁)ₘR₁.

5. Au moins une entité chimique selon la revendication 1, dans laquelle le composé de Formule 1 est choisi parmi les composés de Formule 2 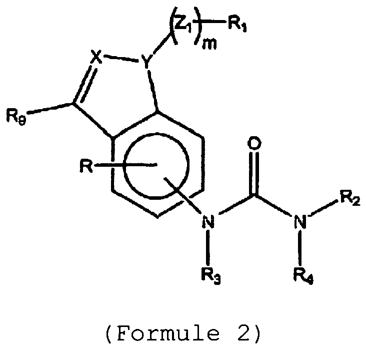 dans laquelle
X et Y sont choisis indépendamment parmi CH et N ; et
R₉ est choisi parmi hydrogène et alkyle éventuellement substitué.

6. Au moins une entité chimique selon l'une quelconque des revendications 1 à 5, dans laquelle R₁ est choisi parmi pyridinyle et pyridinyle substitué où pyridinyle substitué est choisi parmi pyridinyle mono-, di-, et trisubstitué et où les substituants sur pyridinyle substitué sont choisis indépendamment parmi hydroxy, nitro, cyano, amino éventuellement substitué, aminocarbonyle, halogéno, carboxy, acyle éventuellement substitué, alcoxycarbonyle éventuellement substitué, C₁-C₆ alkyle éventuellement substitué, C₁-C₆ alcoxy éventuellement substitué, sulfanyle, sulfinyle, sulfonyle, aryle éventuellement substitué, hétéroaryle éventuellement substitué, et hétérocycloalkyle éventuellement substitué.

7. Au moins une entité chimique selon la revendication 6, dans laquelle les substituants sur pyridinyle substitué sont choisis indépendamment parmi hydroxy, nitro, cyano, amino éventuellement substitué, halogéno, carboxy, C₁-C₆ alkyle éventuellement substitué, C₁-C₆ alcoxy éventuellement substitué, C₁-C₆ alkylsulfanyle, C₁-C₆ acyle, C₁-C₆ alcoxycarbonyle, hétéroaryle éventuellement substitué, et hétérocycloalkyle.

8. Au moins une entité chimique selon la revendication 7, dans laquelle les substituants sur pyridinyle substitué sont choisis indépendamment parmi hydroxy, cyano, halogéno, C₁-C₂ alkyle éventuellement substitué, C₁-C₂ alcoxy éventuellement substitué, et -NHR₁₀ où R₁₀ est choisi parmi hydrogène et acyle éventuellement substitué.

9. Au moins une entité chimique selon la revendication 6, dans laquelle R₁ est pyridin-4-yle.

10. Au moins une entité chimique selon l'une quelconque des revendications 1 à 9, dans laquelle au moins l'un parmi R₅ et R₆ est hydrogène.

11. Au moins une entité chimique selon la revendication 10, dans laquelle les deux parmi R₅ et R₆ sont hydrogène.

12. Au moins une entité chimique selon l'une quelconque des revendications 1 à 9, dans laquelle m vaut 1 et au moins l'un parmi R₅ et R₆ est hydrogène.

13. Au moins une entité chimique selon la revendication 10, dans laquelle les deux parmi R₅ et R₆ sont hydrogène.

14. Au moins une entité chimique selon l'une quelconque des revendications 1 à 9, dans laquelle m est 0.

15. Au moins une entité chimique selon la revendication 5, dans laquelle le composé de Formule 2 est choisi parmi les composés de Formule 3 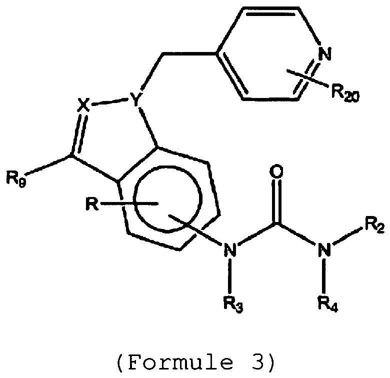 dans laquelle
R₂₀ représente 0 à 3 substituants choisis indépendamment parmi hydroxy, nitro, cyano, amino éventuellement substitué, aminocarbonyle, halogéno, carboxy, acyle éventuellement substitué, alcoxycarbonyle éventuellement substitué, C₁-C₆ alkyle éventuellement substitué, C₁-C₆ alcoxy éventuellement substitué, sulfanyle, sulfinyle, sulfonyle, aryle éventuellement substitué, hétéroaryle éventuellement substitué, et hétérocycloalkyle éventuellement substitué.

16. Au moins une entité chimique selon la revendication 15, dans laquelle le composé de Formule 3 est choisi parmi les composés de Formule 4

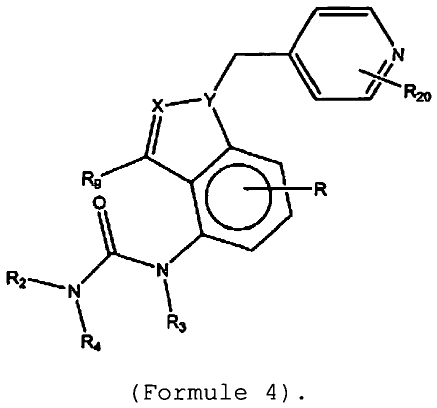

17. Au moins une entité chimique selon l'une quelconque des revendications 5 à 16, dans laquelle X et Y sont N.

18. Au moins une entité chimique selon l'une quelconque des revendications 5 à 16, dans laquelle Y est N et X est CH.

19. Au moins une entité chimique selon l'une quelconque des revendications 1 à 18, dans laquelle R₂ est choisi parmi phényle et phényle substitué où phényle substitué est choisi parmi phényle mono-, di-, et trisubstitué et où les substituants sur phényle substitué sont choisis indépendamment parmi hydroxy, nitro, cyano, amino éventuellement substitué, aminocarbonyle, halogéno, carboxy, acyle éventuellement substitué, alcoxycarbonyle éventuellement substitué, C₁-C₆ alkyle éventuellement substitué, C₁-C₆ alcoxy éventuellement substitué, aryloxy éventuellement substitué, sulfanyle, sulfinyle, sulfonyle, aryle éventuellement substitué, hétéroaryle éventuellement substitué, et hétérocycloalkyle éventuellement substitué.

20. Au moins une entité chimique selon la revendication 19, dans laquelle les substituants sur phényle substitué sont choisis indépendamment parmi hydroxy, nitro, cyano, amino éventuellement substitué, halogéno, carboxy, C₁-C₆ alkyle éventuellement substitué, C₁-C₆ alcoxy éventuellement substitué, phényle éventuellement substitué, phénoxy éventuellement substitué, C₁-C₆ alkylsulfanyle, C₁-C₆ acyle, C₁-C₆alcoxycarbonyle, hétéroaryle éventuellement substitué, et hétérocycloalkyle.

21. Au moins une entité chimique selon la revendication 20, dans laquelle les substituants sur phényle substitué sont choisis indépendamment parmi hydroxy, cyano, halogéno, C₁-C₂ alkyle éventuellement substitué, phénoxy, et C₁-C₂ alcoxy éventuellement substitué.

22. Au moins une entité chimique selon la revendication 21, dans laquelle les substituants sur phényle substitué sont choisis indépendamment parmi halogéno, méthyle, éthyle, méthoxy, éthoxy, difluorométhyle, trifluorométhyle, difluorométhoxy, et trifluorométhoxy.

23. Au moins une entité chimique selon la revendication 16, dans laquelle les composés de Formule 4 sont choisis parmi les composés de Formule 5 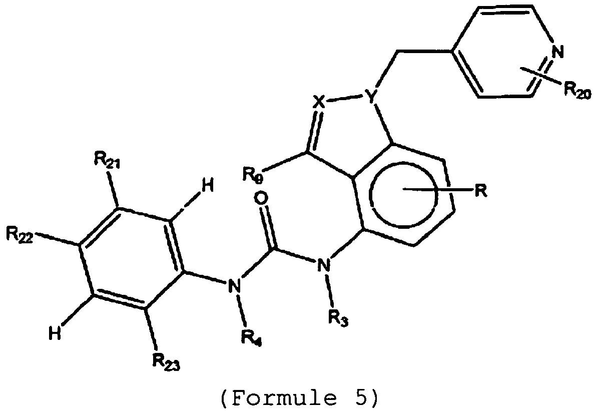 dans laquelle
R₂₁ est choisi parmi hydrogène, halogéno et alkyle inférieur éventuellement substitué ;
R₂₂ est choisi parmi hydrogène, halogéno, alcoxy inférieur, et alkyle inférieur ; et
R₂₃ est choisi parmi hydrogène, alkyle inférieur, phénoxy éventuellement substitué, alcoxy inférieur éventuellement substitué et halogéno.

24. Au moins une entité chimique selon la revendication 23, dans laquelle R₂₁ est choisi parmi hydrogène, halogéno, méthyle et trifluorométhyle.

25. Au moins une entité chimique selon la revendication 23 ou 24, dans laquelle R₂₂ est choisi parmi hydrogène, halogéno, méthoxy et méthyle.

26. Au moins une entité chimique selon la revendication 25, dans laquelle R₂₂ est hydrogène.

27. Au moins une entité chimique selon l'une quelconque des revendications 23 à 25, dans laquelle R₂₃ est choisi parmi hydrogène, méthyle, méthoxy, difluorométhoxy, trifluorométhoxy, éthoxy et halogéno.

28. Au moins une entité chimique selon l'une quelconque des revendications 15 à 27, dans laquelle R₂₀ est amino éventuellement substitué.

29. Au moins une entité chimique selon l'une quelconque des revendications 5 à 28, dans laquelle R₉ est choisi parmi hydrogène et alkyle inférieur éventuellement substitué.

30. Au moins une entité chimique selon la revendication 29, dans laquelle R₉ est choisi parmi hydrogène et alkyle inférieur.

31. Au moins une entité chimique selon la revendication 30, dans laquelle R₉ est hydrogène.

32. Au moins une entité chimique selon l'une quelconque des revendications 1 à 31, dans laquelle R représente 1 ou 2 substituants choisis indépendamment parmi halogéno, C₁-C₂ alkyle, et C₁-C₂ alcoxy.

33. Au moins une entité chimique selon la revendication 32, dans laquelle R représente 1 ou 2 substituants choisis indépendamment parmi halogéno, méthyle et méthoxy.

34. Au moins une entité chimique selon la revendication 33, dans laquelle R représente un substituant choisi parmi halogéno, méthyle et méthoxy.

35. Au moins une entité chimique selon l'une quelconque des revendications 1 à 34, dans laquelle R est absent.

36. Au moins une entité chimique selon l'une quelconque des revendications 1 à 35, dans laquelle R₃ et R₄ sont choisis chacun indépendamment parmi hydrogène et méthyle.

37. Au moins une entité chimique selon la revendication 36, dans laquelle R₃ et R₄ sont hydrogène.

38. Au moins une entité chimique selon la revendication 1, dans laquelle le composé de Formule 1 est choisi parmi
la 1-(5-bromo-2-méthoxyphényl)-3-(1-pyridin-4-ylméthyl-1H-indol-4-yl)urée ;
la 1-(2-méthoxy-5-trifluorométhylphényl)-3-(1-pyridin-4-ylméthyl-1H-indol-4-yl)urée ;
la 1-(2-méthoxy-5-trifluorométhylphényl)-3-(1-pyridin-4-ylméthyl-1H-indol-6-yl)urée ;
la 1-(4-chloro-2-méthoxy-5-trifluorométhylphényl)-3-(1-pyridin-4-ylméthyl-1H-indol-4-yl)urée ;
la 1-(2-fluoro-5-trifluorométhylphényl)-3-(1-pyridin-4-ylméthyl-1H-indol-4-yl)urée ;
la 1-(2,4-diméthoxy-5-trifluorométhylphényl)-3-(1-pyridin-4-ylméthyl-1H-indol-4-yl)urée ;
la 1-(2,4-diméthyl-5-trifluorométhylphényl)-3-(1-pyridin-4-ylméthyl-1H-indol-4-yl)urée ;
la 1-(2-éthoxy-5-trifluorométhylphényl)-3-(1-pyridin-4-ylméthyl-1H-indol-4-yl)urée ;
la 1-(5-bromo-2-méthoxyphényl)-3-(1-pyridin-4-ylméthyl-1H-indazol-4-yl)urée ;
la 1-(5-bromo-2-éthoxyphényl)-3-(1-pyridin-4-ylméthyl-1H-indol-4-yl)urée ;
la 1-(5-bromo-2-méthoxyphényl)-3-(1-pyridin-4-ylméthyl-1H-indol-5-yl)urée ;
la 1-(4-méthyl-3-trifluorométhylphényl)-3-(1-pyridin-4-ylméthyl-1H-indol-4-yl)urée ;
la 1-(4-chloro-3-trifluorométhylphényl)-3-(1-pyridin-4-ylméthyl-1H-indol-4-yl)urée ;
la 1-(5-chloro-2-méthoxyphényl)-3-(1-pyridin-4-ylméthyl-1H-indol-5-yl)urée ;
la 1-(2-méthoxy-5-trifluorométhylphényl)-3-(1-pyridin-4-ylméthyl-1H-indol-5-yl)urée ;
la 1-(4-chloro-3-trifluorométhylphényl)-3-(1-pyridin-4-ylméthyl-1H-indol-5-yl)urée ;
la 1-(2-fluoro-5-trifluorométhylphényl)-3-(1-pyridin-4-ylméthyl-1H-indol-5-yl)urée ;
la 1-(2-chloro-5-trifluorométhylphényl)-3-(1-pyridin-4-ylméthyl-1H-indol-5-yl)urée ;
la 1-(2-fluoro-5-trifluorométhylphényl)-3-(3-méthyl-1-pyridin-4-ylméthyl-1H-indol-4-yl)urée ;
la 1-(2-méthoxy-5-trifluorométhylphényl)-3-(3-méthyl-1-pyridin-4-ylméthyl-1H-indol-4-yl)urée ;
la 1-(5-bromo-2-méthoxyphényl)-3-(3-méthyl-1-pyridin-4-ylméthyl-1H-indol-4-yl)urée ;
la 1-(5-bromo-2-méthoxyphényl)-3-(1-pyridin-3-ylméthyl-1H-indol-4-yl)urée ;
la 1-(5-bromo-2-méthoxyphényl)-3-(1-pyridin-3-ylméthyl-1H-indol-5-yl)urée ;
la 1-(5-bromo-2-méthoxyphényl)-3-(5-méthyl-1-pyridin-4-ylméthyl-1H-indol-4-yl)urée ;
la 1-(5-bromo-2-méthoxyphényl)-3-(7-méthyl-1-pyridin-4-ylméthyl-1H-indol-4-yl)urée ;
la 1-(2-méthoxy-5-trifluorométhylphényl)-3-(5-méthyl-1-pyridin-4-ylméthyl-1H-indol-4-yl)urée ;
la 1-(2-fluoro-5-trifluorométhylphényl)-3-(5-méthyl-1-pyridin-4-ylméthyl-1H-indol-4-yl)urée ;
la 1-(5-chloro-2-méthoxyphényl)-3-(5-méthyl-1-pyridin-4-ylméthyl-1H-indol-4-yl)urée ;
la 1-(4-méthoxybiphényl-3-yl)-3-(1-pyridin-4-ylméthyl-1H-indol-4-yl)urée ;
la 1-(2-méthoxy-5-trifluorométhylphényl)-3-(7-méthyl-1-pyridin-4-ylméthyl-1H-indol-4-yl)urée ;
la 1-(2-fluoro-5-trifluorométhylphényl)-3-(7-méthyl-1-pyridin-4-ylméthyl-1H-indol-4-yl)urée ;
la 1-(5-chloro-2-méthoxyphényl)-3-(7-méthyl-1-pyridin-4-ylméthyl-1H-indol-4-yl)urée ;
l'acide 4-{4-[3-(2-méthoxy-5-trifluorométhylphényl)-uréido]-5-méthyl-indol-1-ylméthyl}pyridine-2-carboxylique méthylamide ;
la 1-(7-méthyl-1-pyridin-4-ylméthyl-1H-indol-4-yl)-3-(4-méthyl-3-trifluorométhylphényl)urée ;
la 1-(4-chloro-3-trifluorométhylphényl)-3-(7-méthyl-1-pyridin-4-ylméthyl-1H-indol-4-yl)urée ;
la 1-[5-chloro-2-([1,3]dioxolan-2-ylméthoxy)phényl]-3-(1-pyridin-4-ylméthyl-1H-indol-4-yl)urée ;
la 1-[5-chloro-2-(3-hydroxypropoxy)phényl]-3-(5-méthyl-1-pyridin-4-ylméthyl-1H-indol-4-yl)urée ;
la 1-(3-chloro-4-méthoxyphényl)-3-(1-pyridin-4-ylméthyl-1H-indol-4-yl)urée ;
la 1-(4-méthoxy-3-trifluorométhylphényl)-3-(1-pyridin-4-ylméthyl-1H-indol-4-yl)urée ;
la 1-(2-méthoxy-4-méthyl-5-trifluorométhylphényl)-3-(1-pyridin-4-ylméthyl-1H-indol-4-yl)urée ;
la 1-(3-bromo-4-méthylphényl)-3-(1-pyridin-4-ylméthyl-1H-indol-4-yl)urée ;
la 1-(4-fluoro-3-trifluorométhylphényl)-3-(1-pyridin-4-ylméthyl-1H-indol-4-yl)urée ;
la 1-(5-bromo-2-méthoxy-4-méthylphényl)-3-(1-pyridin-4-ylméthyl-1H-indol-4-yl)urée ;
la 1-(5-bromo-2-méthoxyphényl)-3-[1-(2-fluoropyridin-4-ylméthyl)-1H-indol-4-yl]urée ;
la 1-(5-bromo-2-méthoxyphényl)-3-[1-(2-méthylpyridin-4-ylméthyl)-1H-indol-4-yl]urée ;
la 1-(3-bromo-4-méthoxyphényl)-3-(1-pyridin-4-ylméthyl-1H-indol-4-yl)urée ;
la 1-(5-bromo-2-méthoxyphényl)-3-[1-(3-fluoropyridin-4-ylméthyl)-1H-indol-4-yl]urée ;
la 1-[1-(3-fluoropyridin-4-ylméthyl)-1H-indol-4-yl]-3-(4-méthyl-3-trifluorométhylphényl)urée ;
la 1-(3-chloro-4-fluorophényl)-3-(1-pyridin-4-ylméthyl-1H-indol-4-yl)urée ;
la 1-[1-(2-aminopyridin-4-ylméthyl)-1H-indol-4-yl]-3-(5-bromo-2-méthoxyphényl)urée ;
la 1-(3,4-diméthoxyphényl)-3-(1-pyridin-4-ylméthyl-1H-indol-4-yl)urée ;
la 1-(3-chloro-4-méthylphényl)-3-(1-pyridin-4-ylméthyl-1H-indol-4-yl)urée ;
la 1-(5-chloro-2-méthoxy-4-méthylphényl)-3-(1-pyridin-4-ylméthyl-1H-indol-4-yl)urée ;
la 1-[1-(2-aminopyridin-4-ylméthyl)-1H-indol-4-yl]-3-(5-bromo-2-méthoxy-4-méthylphényl)urée ;
la 1-[1-(2-aminopyridin-4-ylméthyl)-1H-indol-4-yl]-3-(3-chloro-4-méthylphényl)urée ;
la 1-[1-(2-aminopyridin-4-ylméthyl)-1H-indol-4-yl]-3-(5-chloro-2-méthoxyphényl)urée ;
la 1-[1-(2-aminopyridin-4-ylméthyl)-1H-indol-4-yl]-3-(5-chloro-2,4-diméthoxyphényl)urée ;
la 1-[1-(2-aminopyridin-4-ylméthyl)-1H-indol-4-yl]-3-(5-chloro-2-méthoxy-4-méthylphényl)urée ;
la 1-[1-(2-aminopyridin-4-ylméthyl)-1H-indol-4-yl]-3 (2,4-diméthyl-5-trifluorométhylphényl)urée ;
la 1-(5-bromo-2-méthoxyphényl)-3-(5-méthoxy-1-pyridin-4-ylméthyl-1H-indol-4-yl)urée ;
la 1-[1-(2-aminopyridin-4-ylméthyl)-1H-indol-4-yl]-3-(2-méthoxy-5-trifluorométhylphényl)urée ;
la 1-[1-(2-aminopyridin-4-ylméthyl)-1H-indol-4-yl]-3-(2,4-diméthoxy-5-trifluorométhylphényl)urée ;
la 1-[1-(2-aminopyridin-4-ylméthyl)-1H-indol-4-yl]-3-(2-méthoxy-4-méthyl-5-trifluorométhylphényl)urée ;
la 1-benzo[1,3]dioxol-5-yl-3-(1-pyridin-4-ylméthyl-1H-indol-4-yl)urée ;
la 1-(5-bromo-2-méthoxyphényl)-3-(6-méthyl-1-pyridin-4-ylméthyl-1H-indol-4-yl)urée ;
l'acide (4-{4-[3-(5-bromo-2-méthoxyphényl)uréido]indol-1-ylméthyl}pyridin-2-yl)carbamique méthylester ;
la 1-(5-bromo-2-méthoxyphényl)-3-[1-(3-chloro-pyridin-4-ylméthyl)-1H-indol-4-yl]urée ;
la 1-(5-fluoro-2,4-diméthoxyphényl)-3-(1-pyridin-4-ylméthyl-lH-indol-4-yl)urée ;
la 1-(3-bromo-4-fluorophényl)-3-(1-pyridin-4-ylméthyl-1H-indol-4-yl)urée ;
le N-(4-{4-[3-(5-bromo-2-méthoxyphényl)uréido]indol-1-ylméthyl}pyridin-2-yl)acétamide ;
la 1-(5-éthanesulfonyl-2-méthoxyphényl)-3-(1-pyridin-4-ylméthyl-1H-indol-4-yl)urée ;
la 1-(5-bromo-2,4-difluorophényl)-3-(1-pyridin-4-ylméthyl-1H-indol-4-yl)urée ;
la 1-[1-(2-aminopyridin-4-ylméthyl)-1H-indol-4-yl]-3-(2-fluoro-5-trifluorométhylphényl)urée ;
le N-(4-{4-[3-(5-chloro-2-méthoxy-4-méthylphényl)-uréido]indol-1-ylméthyl}pyridin-2-yl)acétamide ;
la 1-(5-bromo-2-méthoxyphényl)-3-(7-fluoro-1-pyridin-4-ylméthyl-1H-indol-4-yl)urée ;
la 1-(5-chloro-2-méthoxy-4-méthylphényl)-3-(7-fluoro-1-pyridin-4-ylméthyl-1H-indol-4-yl)urée ;
le N-(4-{4-[3-(5-bromo-2-méthoxyphényl)uréido]indol-1-ylméthyl}pyridin-2-yl)-2-hydroxyacétamide ;
le 2-amino-N-(4-{4-[3-(5-bromo-2-méthoxyphényl)uréido]-indol-1-ylméthyl}pyridin-2-yl)acétamide ;
le N-(4-{4-[3-(5-bromo-2-méthoxyphényl)uréido]indol-1-ylméthyl}pyridin-2-yl)formamide ;
la 1-[1-(2-aminopyridin-4-ylméthyl)-1H-indol-4-yl]-3-(3-bromo-4-méthoxyphényl)urée ;
le N-(4-{4-[3-(3-chloro-4-méthylphényl)uréido]indol-1-ylméthyl}pyridin-2-yl)acétamide ;
la 1-(5-bromo-2-méthoxyphényl)-3-(7-méthoxy-1-pyridin-4-ylméthyl-1H-indol-4-yl)urée ;
la 1-(2,4-diméthoxy-5-trifluorométhylphényl)-3-(7-méthoxy-1-pyridin-4-ylméthyl-1H-indol-4-yl)urée ;
la 1-(5-bromo-2-méthoxyphényl)-3-(5-fluoro-1-pyridin-4-ylméthyl-1H-indol-4-yl)urée ;
le N-(4-{4-[3-(5-chloro-2-méthoxyphényl)uréido]indol-1-ylméthyl}pyridin-2-yl)acétamide ;
l'acide 1-méthyl-1H-imidazole-2-carboxylique (4-{4-[3-(5-bromo-2-méthoxyphényl)uréido]indol-1-ylméthyl}-pyridin-2-yl)amide ;
le N-(4-{4-[3-(5-bromo-2-méthoxyphényl)uréido]indol-1-ylméthyl}pyridin-2-yl)propionamide ;
le N-(4-{4-[3-(5-bromo-2-méthoxyphényl)uréido]indol-1-ylméthyl}pyridin-2-yl)isobutyramide ;
le N-(4-{4-[3-(5-bromo-2-méthoxyphényl)uréido]indol-1-ylméthyl}pyridin-2-yl)-2-morpholin-4-ylacétamide ;
la 1-(5-bromo-2-méthoxyphényl)-3-[1-(2-méthylamino-pyridin-4-ylméthyl)-1H-indol-4-yl]urée ;
la 1-(5-bromo-2-méthoxyphényl)-3-{1-[2-(2-méthoxy-éthylamino)pyridin-4-ylméthyl]-1H-indol-4-yl}urée ;
la 1-(5-bromo-2-méthoxyphényl)-3-[1-(2-morpholin-4-yl-pyridin-4-ylméthyl)-1H-indol-4-yl]urée ;
la 1-(5-bromo-2-méthoxyphényl)-3-[1-(2-méthoxypyridin-4-ylméthyl)-1H-indol-4-yl]urée ;
la 1-(5-bromo-2-méthoxyphényl)-3-[1-(2-hydroxypyridin-4-ylméthyl)-1H-indol-4-yl]urée ;
le N-(4-{4-[3-(2-fluoro-5-trifluorométhylphényl)-uréido]indol-1-ylméthyl}pyridin-2-yl)acétamide ;
le N-(4-{4-[3-(5-chloro-2-méthoxyphényl)uréido]indol-1-ylméthyl}pyridin-2-yl)-2-morpholin-4-ylacétamide ;
le N-(4-{4-[3-(5-bromo-2-méthoxyphényl)uréido]indol-1-ylméthyl}pyridin-2-yl)-2-méthoxyacétamide ;
la 1-(5-chloro-2-méthoxyphényl)-3-[1-(3-méthylpyridin-4-ylméthyl)-1H-indol-4-yl]urée ;
l'acide cyclopropanecarboxylique (4-{4-[3-(5-chloro-2-méthoxyphényl)uréido]indol-1-ylméthyl}pyridin-2-yl)amide ;
la 1-[1-(2-aminopyridin-4-ylméthyl)-1H-indol-4-yl]-3-(5-bromo-2-difluorométhoxyphényl)urée ;
le N-(4-{4-[3-(5-bromo-2-difluorométhoxyphényl)-uréido]indol-1-ylméthyl}pyridin-2-yl)acétamide ;
le N-(4-{4-[3-(5-chloro-2-méthoxyphényl)uréido]indol-1-ylméthyl}pyridin-2-yl)-3-diéthylamino-propionamide ;
la 1-(5-bromo-2-méthoxyphényl)-3-(7-chloro-1-pyridin-4-ylméthyl-1H-indol-4-yl)urée ;
la 1-(5-bromo-2-méthoxyphényl)-3-{1-[2-(2-hydroxy-éthylamino)pyridin-4-ylméthyl]-1H-indol-4-yl}urée ;
la 1-(5-bromo-2-méthoxyphényl)-3-(5-chloro-1-pyridin-4-ylméthyl-1H-indol-4-yl)urée ;
le N-(4-{4-[3-(5-bromo-2-méthoxyphényl)uréido]indol-1-ylméthyl}pyridin-2-yl)-2-pyrrolidin-1-ylacétamide ;
le N-(4-{4-[3-(5-bromo-2-méthoxyphényl)uréido]indol-1-ylméthyl}pyridin-2-yl)-2-diméthylaminoacétamide ;
le N-(4-{4-[3-(5-bromo-2-méthoxyphényl)uréido]indol-1-ylméthyl}pyridin-2-yl)-2-méthylaminoacétamide ;
la 1-[1-(2-aminopyridin-4-ylméthyl)-5-fluoro-1H-indol-4-yl]-3-(5-bromo-2-méthoxyphényl)urée ;
l'acide cyclopropanecarboxylique (4-{4-[3-(5-bromo-2-méthoxyphényl)uréido]indol-1-ylméthyl}pyridin-2-yl)amide ;
l'acide cyclopropanecarboxylique (4-{4-[3-(2-méthoxy-5-trifluorométhylphényl)uréido]indol-1-ylméthyl}pyridin-2-yl)amide ;
l'acide cyclopropanecarboxylique (4-{4-[3-(2-fluoro-5-trifluorométhylphényl)uréido]indol-1-ylméthyl}pyridin-2-yl)amide ;
la 1-[1-(2-aminopyridin-4-ylméthyl)-5-fluoro-1H-indol-4-yl]-3-(2-fluoro-5-trifluorométhylphényl)urée ;
la 1-[1-(2-aminopyridin-4-ylméthyl)-5-fluoro-1H-indol-4-yl]-3-(2-méthoxy-5-trifluorométhylphényl)urée ;
l'acide cyclopropanecarboxylique (4-{4-[3-(3-chloro-4-méthylphényl)uréido]indol-1-ylméthyl}pyridin-2-yl)amide ;
le N-(4-{4-[3-(5-bromo-2-méthoxyphényl)uréido]indol-1-ylméthyl}pyridin-2-yl)méthanesulfonamide ;
le N-(4-{4-[3-(5-chloro-2-méthoxyphényl)uréido]indol-1-ylméthyl}pyridin-2-yl)méthanesulfonamide ;
la 1-[1-(2-aminopyridin-4-ylméthyl)-5-fluoro-1H-indol-4-yl]-3-(2,5-dichlorophényl)urée ;
la 1-[1-(2-aminopyridin-4-ylméthyl)-5-fluoro-1H-indol-4-yl]-3-(5-chloro-2-méthoxyphényl)urée ;
la 1-(5-chloro-2-méthoxyphényl)-3-{1-[2-(pyrazin-2-ylamino)pyridin-4-ylméthyl]-1H-indol-4-yl}urée ;
la 1-(5-chloro-2-méthoxyphényl)-3-{1-[2-(thiazol-2-ylamino)pyridin-4-ylméthyl]-1H-indol-4-yl}urée ;
la 1-[1-(2-aminopyridin-4-ylméthyl)-7-fluoro-1H-indol-4-yl]-3-(5-bromo-2-méthoxyphényl)urée ;
le N-(4-{4-[3-(5-chloro-2-méthoxyphényl)uréido]indol-1-ylméthyl}pyridin-2-yl)-2-méthylaminoacétamide ;
le N-(4-{4-[3-(5-bromo-2-méthoxyphényl)uréido]indol-1-ylméthyl}pyridin-2-yl)-3-diéthylaminopropionamide ;
la 1-[1-(2-aminopyridin-4-ylméthyl)-7-fluoro-1H-indol-4-yl]-3-(2-fluoro-5-trifluorométhylphényl)urée ;
la 1-(2-fluoro-5-trifluorométhylphényl)-3-{1-[2-(pyridin-2-ylamino)pyridin-4-ylméthyl]-1H-indol-4-yl}urée ;
la 1-(5-bromo-2-méthoxyphényl)-3-{1-[2-(3-isopropyl-uréido)pyridin-4-ylméthyl]-1H-indol-4-yl}urée ;
le 3-(acétylméthylamino)-N-(4-{4-[3-(5-chloro-2-méthoxyphényl)uréido]indol-1-ylméthyl}pyridin-2-yl)-propionamide ;
le N-(4-{4-[3-(2-fluoro-5-trifluorométhylphényl)-uréido]indol-1-ylméthyl}pyridin-2-yl)-2-méthylamino-acétamide ;
la 1-[1-(2-allylaminopyridin-4-ylméthyl)-1H-indol-4-yl]-3-(5-bromo-2-méthoxyphényl)urée ;
le 3-amino-N-(4-{4-[3-(5-chloro-2-méthoxyphényl)-uréido]indol-1-ylméthyl}pyridin-2-yl)propionamide ;
la 1-(2-fluoro-5-trifluorométhylphényl)-3-{1-[2-(pyridin-3-ylamino)pyridin-4-ylméthyl]-1H-indol-4-yl}urée ;
la 1-[1-(2-amino-5-fluoropyridin-4-ylméthyl)-1H-indol-4-yl]-3-(5-bromo-2-méthoxyphényl)urée ;
la 1-[1-(2-aminopyridin-4-ylméthyl)-5-chloro-1H-indol-4-yl]-3-(5-bromo-2-méthoxyphényl)urée ;
la 1-[1-(2-amino-3-méthylpyridin-4-ylméthyl)-1H-indol-4-yl]-3-(5-bromo-2-méthoxyphényl)urée ;
la 1-(2-fluoro-5-trifluorométhylphényl)-3-{1-[2-(1H-tétrazol-5-yl)pyridin-4-ylméthyl]-1H-indol-4-yl}urée ; la 1-(2-fluoro-5-trifluorométhylphényl)-3-{1-[2-(pyrimidin-2-ylamino)pyridin-4-ylméthyl]-1H-indol-4-yl}urée ;
la 1-[1-(2-bromopyridin-4-ylméthyl)-1H-indol-4-yl]-3-(5-chloro-2-méthoxyphényl)urée ;
la 1-[1-(2-bromopyridin-4-ylméthyl)-1H-indol-4-yl]-3-(2-fluoro-5-trifluorométhylphényl)urée ;
la 1-[1-(2-cyanopyridin-4-ylméthyl)-1H-indol-4-yl]-3-(2-fluoro-5-trifluorométhylphényl)urée ;
la 1-(5-chloro-2-méthoxyphényl)-3-[1-(2-cyanopyridin-4-ylméthyl)-1H-indol-4-yl]urée ;
la 1-(2-fluoro-5-trifluorométhylphényl)-3-{1-[2-(1H-imidazol-2-yl)pyridin-4-ylméthyl]-1H-indol-4-yl}urée ; la 1-(5-chloro-2-méthoxyphényl)-3-{1-[2-(4-cyano-phénylamino)pyridin-4-ylméthyl]-1H-indol-4-yl}urée ;
et la 1-(5-chloro-2-méthoxyphényl)-3-{1-[2-(6-cyano-pyridin-3-ylamino)pyridin-4-ylméthyl]-1H-indol-4-yl}urée.

39. Au moins une entité chimique selon la revendication 1, **caractérisée en ce que** l'entité chimique est la 1-[1-(2-aminopyridin-4-ylméthyl)-1H-indol-4-yl]-3-(5-bromo-2-méthoxyphényl)urée, ou un sel pharmaceutiquement acceptable, un solvat ou une forme cristalline de celle-ci.

40. Composition pharmaceutique, comprenant au moins une entité chimique selon l'une quelconque des revendications 1 à 39, conjointement avec au moins un véhicule pharmaceutiquement acceptable choisi parmi les supports, les adjuvants et les excipients.

41. Composition pharmaceutique selon la revendication 40, dans laquelle la composition est formulée sous une forme choisie parmi les fluides injectables, les aérosols, les crèmes, les gels, les comprimés, les pilules, les capsules, les sirops, les solutions ophtalmiques et les patchs transdermiques.

42. Composition pharmaceutique conditionnée, comprenant la composition pharmaceutique selon la revendication 40 ou 41 dans un conteneur ; et des instructions pour l'utilisation de la composition afin de traiter un patient souffrant d'une maladie ou d'un trouble répondant à la modulation de l'activité de kinases d'une ou plusieurs tyrosine kinases.

43. Utilisation d'au moins une entité chimique pour l'élaboration d'un médicament destiné au traitement d'un patient souffrant d'une maladie répondant à l'inhibition d'au moins une kinase choisie parmi VEGFR2, EphB₄, PDGFRβ, et c-Kit, dans laquelle la au moins une entité chimique est une entité chimique selon l'une quelconque des revendications 1 à 39.

44. Utilisation selon la revendication 43, dans laquelle la maladie répondant à l'inhibition d'au moins une kinase répond à l'inhibition de l'activité de la kinase VEGFR2.

45. Utilisation selon la revendication 44, dans laquelle la maladie répondant à l'inhibition de l'activité de la kinase VEGFR2 est choisie parmi le cancer et les maladies **caractérisées par** un changement de l'angiogenèse.

46. Utilisation selon la revendication 45, dans laquelle les maladies **caractérisées par** un changement de l'angiogenèse sont choisies parmi les tumeurs cancéreuses, la dégénérescence maculaire, et la rétinopathie diabétique.

47. Méthode d'élaboration d'un médicament destiné au traitement d'un patient souffrant d'une maladie répondant à l'inhibition d'au moins une kinase choisie parmi VEGFR2, EphB₄, PDGFRβ, et c-Kit, comprenant l'incorporation dans ledit médicament d'au moins une entité chimique selon l'une quelconque des revendications 1 à 39.

48. Méthode selon la revendication 47, dans laquelle la maladie répondant à l'inhibition d'au moins une kinase répond à l'inhibition de l'activité de la kinase VEGFR2.

49. Méthode selon la revendication 48, dans laquelle la maladie répondant à l'inhibition de l'activité de la kinase VEGFR2 est choisie parmi le cancer et les maladies **caractérisées par** un changement de l'angiogenèse.

50. Méthode selon la revendication 49, dans laquelle les maladies **caractérisées par** un changement de l'angiogenèse sont choisies parmi les tumeurs cancéreuses, la dégénérescence maculaire, et la rétinopathie diabétique.

51. Utilisation d'au moins une entité chimique pour l'élaboration d'un médicament destiné au traitement d'un patient ayant un trouble ou une condition de l'appareil reproducteur féminin, dans laquelle la au moins une entité chimique est une entité chimique selon l'une quelconque des revendications 1 à 39.

52. Utilisation selon la revendication 51, dans laquelle le trouble ou la condition de l'appareil reproducteur féminin est choisi(e) parmi l'endométriose, le carcinome de l'endomètre, les troubles de saignement gynécologique, les cycles menstruels irréguliers, l'ovulation, le syndrome prémenstruel (PMS), et les dysfonctionnements liés à la ménopause.

53. Entité chimique selon l'une quelconque des revendications 1 à 39, pour une utilisation dans le traitement d'un patient ayant (i) une maladie répondant à l'inhibition d'au moins une kinase choisie parmi VEGFR2, EphB₄, PDGFRβ, et c-Kit, ou (ii) un trouble ou une condition de l'appareil reproducteur féminin.
